# FASCICULE DE BREVET EUROPEEN

(11) **EP 1 765 893 B1**
(45) Date de publication et mention de la délivrance du brevet: **22.08.2012**
(21) Numéro de dépôt: 05786071.0
(22) Date de dépôt: 01.07.2005
(51) Int. Cl.: C08F 220/34

(54) **NOUVEAUX COPOLYMERES ETHYLENIQUES, COMPOSITIONS LES COMPRENANT ET PROCEDES DE PREPARATION ET DE TRAITEMENT**
NEUARTIGE ETHYLEN-COPOLYMERE, ZUSAMMENSETZUNGEN DAMIT UND HERSTELLUNG UND BEHANDLUNGSMETHODEN
NOVEL ETHYLENE COPOLYMERS, COMPOSITIONS COMPRISING SAME AND PREPARATION AND TREATMENT METHODS

(30) Priorité: 02.07.2004 FR 0451411; 14.07.2004 US 587553 P
(43) Date de publication de la demande: 28.03.2007
(73) Titulaire: L'Oréal, 75008 Paris (FR)
(72) Inventeur: MOUGIN, Nathalie, F-75011 Paris (FR); JEGOU, Gwenaëlle, F-93190 Livry Gardan (FR)
(74) Mandataire: Dodin, Catherine
(86) Numéro de dépôt international: PCT/FR2005/001700
(87) Numéro de publication internationale: WO 2006/013269

(56) Documents cités:
- EP-A- 0 372 546
- FR-A- 2 419 947
- US-A- 5 608 021
- US-A1- 2004 052 746
- PATENT ABSTRACTS OF JAPAN vol. 1999, no. 12, 29 octobre 1999 (1999-10-29) & JP 11 181029 A (KAO CORP), 6 juillet 1999 (1999-07-06)

## Description

La présente invention a trait à de nouveaux polymères, à leur utilisation notamment en cosmétique, ainsi qu'aux compositions les comprenant.

Il est connu d'employer des polymères dans le domaine cosmétique, et notamment en capillaire, par exemple pour apporter de la tenue ou du coiffant à la chevelure. Dans le domaine des compositions capillaires dites "rincées", telles que les shampooings ou après-shampooings, on utilise notamment des polymères cationiques synthétiques, solubles dans l'eau, qui sont connus pour apporter une bonne cosmétique aux cheveux; toutefois, ces polymères n'apportent aucun effet de mise en forme des cheveux. Il en est de même avec les polymères dérivés naturels cationiques tels les guars modifiées qui apportent également un caractère cosmétique sans permettre une mise en forme. Dans le domaine des compositions rincées, il n'est pas connu de polymères apportant du coiffant et une cosmétique acceptable. Dans le domaine des compositions capillaires dites "non rincées", telles que les produits de coiffage de type laques, gels ou sprays coiffants, on est constamment à la recherche de polymères apportant des effets coiffants, et de la tenue à la chevelure. On connaît dans les produits de coiffage, des polymères à motifs amines, tels que les polymères à base de vinylpyrrolidone et de méthacrylate de diméthylaminoéthyle (polymères Gaffix). Les compositions obtenues présentent toutefois une tenue insuffisante dans le temps. On connaît également des polymères de type Luviquat, à base de vinylimidazole quaternisée et de vinylpyrrolidone, qui apportent de la douceur mais épaississent les compositions.

La présente invention a pour but de proposer des polymères capables d'apporter un réel effet coiffant tout en conservant une cosmétique acceptable aux compositions, et notamment des polymères peu visqueux qui ne modifient que faiblement la viscosité des compositions les comprenant. Après de nombreuses recherches, la demanderesse a mis en évidence que l'utilisation de polymères comprenant entre autre des monomères du type (méth)acrylate de polyéthylèneglycol tels que définis ci-après, pouvait permettre la réalisation de compositions coiffantes ayant une cosmétique adéquate.

Des polymères contenant des motifs (méth)acrylate de polyéthylène glycol (MPEG) sont décrits dans l'art antérieur.
Ainsi, il est connu par EP372546, des copolymères à base de MPEG et de monomères de type (méth)acrylamides d'alkyles en C1-C8, pouvant comprendre des monomères cationiques. Toutefois, ces polymères ne comprennent qu'une faible proportion de monomères cationiques, ce qui ne leur permet pas de générer des effets cosmétiques adéquats, notamment un dépôt sur le cheveu qui soit suffisant pour apporter les propriétés recherchées.

Il revient à la demanderesse d'avoir constaté qu'il était préférable d'utiliser des polymères comprenant des taux de charge cationique élevés pour obtenir un dépôt satisfaisant dudit polymère sur le cheveu.
Le document JP2002-322219 décrit des polymères contenant des motifs MPEG en association avec des monomères hydrophobes à base de polypropylène glycol (PPO) ou de polyoxyde de tétraméthylène, et des monomères cationiques. Or, on a constaté que ces polymères qui comprennent des monomères hydrophobes, ne permettent pas d'obtenir des propriétés cosmétiques satisfaisantes.
Il est également connu par le brevet JP2002-284627 une composition comprenant des polymères cationiques dans lequel les monomères de type PEG sont associés à des monomères comprenant des motifs amines quaternaires. Or, la présence de motifs quaternaires peut induire, au fur et à mesure des applications, un surcroît de dépôt qui peut nuire, dans certains cas, à la qualité cosmétique de la composition. Par ailleurs, ces polymères contiennent un taux de charge cationique faible, de l'ordre de 0,5 à 6%, qui ne permet pas une affinité optimale pour le cheveu.
On connaît encore par JP2003-055164 des polymères contenant des motifs du type MPEG; toutefois ces polymères sont réticulés, ce qui rend difficile le contrôle de leur synthèse.
Le document JP2000-302649 décrit une composition capillaire comprenant un polymère qui comprend des monomères cationiques ou amphotères, des monomères à groupement polyéther, notamment de type PEG ou PPO, ainsi que des monomères optionnels qui peuvent être principalement hydrophobes (par exemple méthacrylate de stéaryle).
Il est également connu par le brevet JP07-285831, des compositions capillaires comprenant un polymère qui comprend des monomères de type MPEG en association avec des monomères ioniques, cationiques ou amphotères, et des monomères additionnels de type (méth)acrylates d'alkyle en C1-C24, principalement hydrophobes.
Toutefois, la présence de comonomères hydrophobes, par exemple de type acrylate de butyle ou de stéaryle, ne permet pas d'obtenir des propriétés cosmétiques adéquates, notamment ne permettent pas d'obtenir un bon démêlage des cheveux mouillés, juste après le shampoing.
On connaît encore la demande WO03/075867 qui décrit des copolymères blocs linéaires comportant une séquence poly(alkylène glycol) encadrée par deux séquences éthyléniques. Ces polymères présentent le défaut d'avoir une séquence centrale de type poly(alkylène glycol) de masse élevée qui confère au polymère une forte cristallinité, ce qui peut conduire à des produits opaques et/ou présentant un caractère gras.

La demanderesse a mis en évidence de nouveaux polymères permettant d'apporter un effet coiffant et conditionneur aux produits cosmétiques capillaires.

De manière surprenante, les polymères selon l'invention possèdent des propriétés cosmétiques intéressantes, par exemple lors de l'application dans une formulation de type shampooing; on a en effet constaté que les cheveux se démêlent facilement lors du shampooing, et/ou lorsque les cheveux sont encore mouillés; par ailleurs, ils présentent de la douceur; après séchage, les compositions selon l'invention permettent également, une fois la chevelure séchée, une mise en forme des cheveux particulièrement intéressante.
Sans être tenu par la présente explication, on peut penser que ceci peut notamment être dû à la présence de motifs (méth)acrylate de PEG (MPEG) au sein de la chaîne de polymère, motifs qui contribuent en grande part à l'effet obtenu. On a en effet constaté que cet effet n'était pas obtenu avec un simple mélange de polymère cationique et de polymère de type PEG.
Ces effets peuvent également être liés au fait que les motifs cationiques du polymère sont neutralisés, en encore plus particulièrement au fait qu'ils sont neutralisés avec un agent neutralisant de nature organique.

Un objet de la présente invention est donc un copolymère éthylénique comprenant, en % en poids par rapport au poids total du polymère :
- a) 10-80% en poids d'un ou plusieurs monomères de formule (I) telle que définie ci-après;
- b) 20-90% en poids d'au moins un monomère cationique, ou l'un de ses sels, choisi parmi les monomères de formule (IIa) telle que définie ci-après;
   ledit copolymère étant neutralisé au moins partiellement par au moins un agent neutralisant choisi parmi les acides organiques au sens de Bronsted.

Un autre objet de l'invention est une composition comprenant, dans un milieu physiologiquement acceptable, au moins un tel copolymère.

Encore un autre objet de l'invention est un procédé de préparation dudit copolymère éthylénique, dans lequel on neutralise les motifs amines des monomères entrant dans la constitution du polymère, en particulier les motifs amines des monomères cationiques de formule (IIa), avant leur polymérisation, puis on copolymérise les monomères neutralisés afin d'obtenir le copolymère recherché.

Encore un autre objet de l'invention est un procédé de préparation dudit copolymère éthylénique, dans lequel on copolymérise les monomères non neutralisés entrant dans la constitution du copolymère, puis on neutralise ledit copolymère après sa formation.

Les polymères selon l'invention apportent donc de bonnes propriétés cosmétiques, notamment de démêlage et/ou de lissage, en particulier lorsque les cheveux sont humides, mais également lorsque les cheveux sont secs. Ces polymères apportent également de la douceur aux cheveux.

Par ailleurs, la présente invention a pour avantage de proposer des polymères généralement véhiculables dans l'eau, c'est-à-dire solubles ou dispersibles dans l'eau, ce qui permet de les employer de manière avantageuse dans des compostions cosmétiques, notamment de soin de la peau, ou capillaires, généralement à base aqueuse.
Par hydrosoluble ou soluble dans l'eau, on entend que le polymère forme une solution limpide dans l'eau, à raison d'au moins 5% en poids, à 25°C.
Par hydrodispersible ou dispersible dans l'eau, on entend que le polymère forme dans l'eau, à une concentration de 5 % en poids, à 25°C, une suspension ou dispersion stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.

Dans la suite de la présente description, on entendra par 'radical cyclique' un radical monocyclique ou polycyclique, qui peut se présenter lui-même sous forme d'un ou plusieurs cycles, saturés et/ou insaturés, éventuellement substitués (par exemple cyclohexyle, cyclodécyle, benzyle ou fluorényle), mais également un radical qui comprend un ou plusieurs desdits cycles (par exemple p-tertbutylcyclohexyle ou 4-hydroxybenzyle).
On entendra par'radical saturé et/ou insaturé', les radicaux totalement saturés, les radicaux totalement insaturés, y compris aromatiques, ainsi que les radicaux comportant une ou plusieurs doubles et/ou triples liaisons, le reste des liaisons étant des liaisons simples.

Le copolymère éthylénique selon l'invention comprend donc au moins un monomère de formule (I), qui peut être présent seul ou en mélange, : 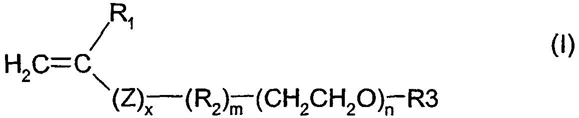 dans laquelle :
- R1 est un atome d'hydrogène ou un radical hydrocarboné, linéaire ou ramifié, de type CₚH₂ₚ₊₁, avec p étant un entier compris entre 1 et 12 inclus;
- Z est un groupement divalent choisi parmi -COO-, -CONH-, -CONCH₃-, -OCO-, - O-, -SO₂- -CO-O-CO- ou -CO-CH₂-CO-;
- x est 0 ou 1;
- R2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m est 0 ou 1
- n est un entier compris entre 3 et 300 inclus;
- R3 est un atome d'hydrogène ou un radical carboné, saturé ou insaturé, éventuellement aromatique, linéaire, ramifié ou cyclique, de 1 à 30 atomes de carbone, pouvant comprendre 1 à 20 hétéroatomes choisis parmi O, N, S, F, Si et P;
et leurs sels.

Notamment, R1 peut représenter un radical méthyle, éthyle, propyle, butyle. De préférence, R1 représente l'hydrogène ou un radical méthyle.

De préférence, Z représente COO ou CONH.

De préférence, x est égal à 1.

Dans le radical R2, le ou les hétéroatomes, quand ils sont présents, peuvent être intercalés dans la chaîne dudit radical R2, ou bien ledit radical R2 peut être substitué par un ou plusieurs groupes les comprenant tels que hydroxy ou amino (NH2, NHR' ou NR'R" avec R' et R" identiques ou différents représentant un alkyle linéaire ou ramifié en C1-C22, notamment méthyle ou éthyle).
Notamment R2 peut être :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 25 hétéroatomes choisis parmi O, N, S, F, Si et P; ou bien un radical benzylène -C₆H₄-CH₂- éventuellement substitué, par un radical alkyle en C1-C12 comprenant éventuellement 1 à 8 hétéroatomes choisis parmi O, N, S, F, Si et P;
- un radical pyridinium de formule : 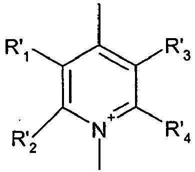 avec R'1 à R'4, identiques ou différents, choisis parmi H et un radical alkyle en C1-C12 comprenant éventuellement 1 à 8 hétéroatomes choisis parmi O, N, S, F, Si et P; notamment R'1 à R'4 peuvent être méthyle et/ou éthyle;
- un radical de formule -CH₂-O-CO-O-, CH₂-CH₂-O-CO-O-, -CH₂-CO-O-, -CH₂-CH₂-CO-O-, -CH₂-O-CO-NH-, -CH₂-CH₂-O-CO-NH-; -CH₂-NH-CO-NH-, -CH₂-CH₂-NH-CO-NH- ;-CH₂-CHOH-, -CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R")-, -CH₂-CH(NR'R")-, -CH₂-CH₂-CH₂-NR'-, -CH₂-CH₂-CH₂-O-; -CH₂-CH₂-CHR'-O- avec R' et R" représentant un alkyle linéaire ou ramifié en C1-C22 comprenant éventuelle-ment 1 à 12 hétéroatomes choisis parmi O, N, S, F, Si et P; - ou un mélange de ces radicaux.

De préférence, n est compris entre 5 et 200 inclus, et encore mieux entre 7 et 100 inclus, voire entre 9 et 50 inclus.

De préférence, R3 est un atome d'hydrogène; un radical benzyle ou phényle éven-tuellement substitué par un radical alkyle en C1-C12 comprenant éventuellement 1 à 8 hétéroatomes choisis parmi O, N, S, F, Si et P; un radical alkyle en C1-C30, notamment C1-C22, voire C2-C16, comprenant éventuellement 1 à 18 hétéroato-mes choisis parmi O, N, S, F, Si et P.
Ces radicaux benzyle, phényle ou alkyle peuvent comprendre notamment une fonction choisie parmi les fonctions suivantes:

| | | | | | |
|---|---|---|---|---|---|
| Succinimido | Glutarate-succinimido | | | | Glutarate |
| 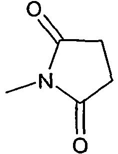 | 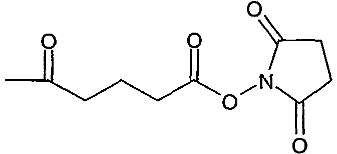 | | | | 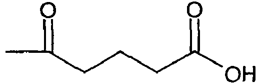 |
| maléimido | | Mésityle | | benzoate | |
| 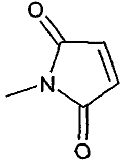 | | 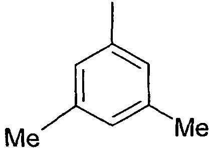 | | 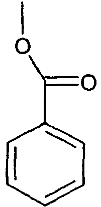 | |
| Tosyle | | Triéthoxysilane | | Phtalimide | |
| 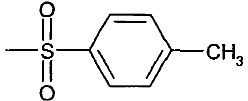 | | 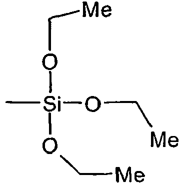 | | 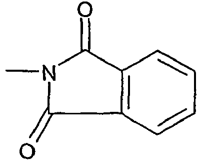 | |
| Thioester | | Benzotriazole carbonate | | Butyraldéhyde | |
| 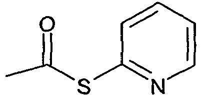 | | 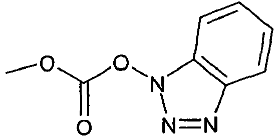 | | 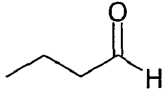 | |
| Acétaldéhyde diéthylacétal | | | Biotine | | |
| 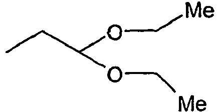 | 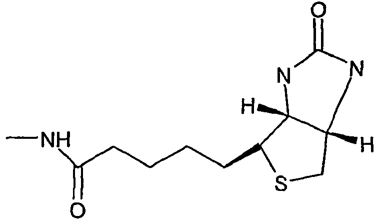 | | | | |
| Phospholipide | | Succinate N-hydroxysuccinimide | | | |
| 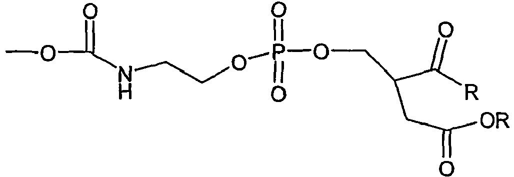 | | 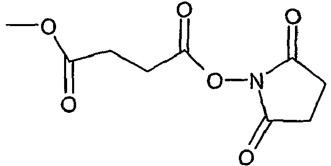 | | | |
| avec R = alkyle en C12-C18, et notamment lauryle, myristyle, palmityle, stéaryle, oléyle ou linoléyle | | | | | |

ou encore choisie parmi -SO₃H, -COOH, -PO₄, -NR5R6 ou -N⁺R5R6R7, avec R5, R6 et R7, indépendamment l'un de l'autre, choisis parmi H ou alkyles en C1-C18, linéaire, ramifié ou cyclique, notamment méthyle, comprenant éventuellement 1 ou plusieurs hétéroatomes ou encore portant des groupements protecteurs tels que le t-butyloxycarbonyle (aussi appelé BOC) ou le 9-fluorenylmethoxycarbonyle (aussi appelé FmoC).

Parmi les radicaux R3, on peut citer les chaînes méthyle, éthyle, propyle, benzyle, éthylhexyle, lauryle, stéaryle, béhényle (-(CH₂)₂₁-CH₃), et également les chaînes alkyles fluorées telles que par exemple heptadecafluorooctyl sulfonyl amino éthyle CF₃-(CF₂)₇-SO₂-N(C₂H₅)-CH₂-CH₂; ou encore les chaînes -CH₂-CH₂-CN, succinimido, maléimido, mésityle, tosyle, triéthoxysilane ou phtalimide.

Les éventuels motifs amines du monomère de formule (I) peuvent éventuellement être neutralisés, de la manière décrite plus loin.

Parmi les monomères de formule (I) particulièrement préférés, on peut citer :
- le (méth)acrylate de poly(éthylène glycol) dans lequel R1 est H ou méthyle; Z est COO, x = 1, m=0 et R3 = H;
- le (méth)acrylate de méthyl-poly(éthylène glycol), aussi appelé (méth)acrylate de méthoxy-poly(éthylèneglycol), dans lequel R1 est H ou méthyle, Z est COO, x = 1, m=0 et R3 = méthyle;
- les (méth)acrylate d'alkyl-poly(éthylène glycol) dans lequel R1 est H ou méthyle, Z est COO, x = 1, m=0 et R3 = alkyl.
- les (méth)acrylates de phényl-poly(éthylène glycol), aussi appelé (méth)acrylate de poly(éthylène glycol) phényl éther, dans lequel R1 est H ou méthyl, Z est COO, x = 1, m=0 et R3 = phényle;
- le monomère suivant : 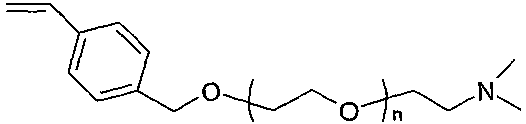
dans lequel n est de préférence compris entre 3 et 100 inclus, notamment 5 à 50 inclus, voire 7 à 30 inclus.

De préférence, le monomère de formule (I) a un poids moléculaire compris entre 350 et 13 000 g/mol., notamment entre 500 et 8000 g/mol.

Les monomères de formule (I) tout particulièrement préférés sont choisis parmi les (méth)acrylates de poly(éthylène glycol) et les (méth)acrylates de méthylpoly(éthylène glycol), de préférence ceux ayant un poids moléculaire compris entre 350 et 13 000 g/mol., notamment entre 500 et 8000 g/mol.
Tout particulièrement, on préfère les (méth)acrylates de poly(éthylène glycol) et en particulier ceux ayant un poids moléculaire entre 350 et 13 000 g/mol., notamment entre 500 et 8000 g/mol.

Des exemples de monomères commerciaux sont :
- le CD 350 (méthacrylate de méthoxy-poly(éthylène glycol 350) et le CD 550 (méthacrylate de méthoxy-poly(éthylène glycol 550), fourni par SARTOMER Chemicals;
- le M90G (méthacrylate de méthoxy-poly(éthylène glycol (9 unités de répétition)) et le M230G (méthacrylate de méthoxy-polyéthylène glycol (23 unités de répétitions)) disponibles chez Shin-Nakamura Chemicals;
- les méthacrylates de méthoxy-poly(éthylène glycol) de poids moléculaires moyens 300, 475 ou 1100, disponibles chez Sigma-Aldrich;
- l'acrylate de méthoxy-poly(éthylène glycol) de poids moléculaire moyen 426 disponible chez Sigma-Aldrich;
- les méthacrylates de méthoxy-poly(éthylène glycol) disponibles chez LAPORTE sous les dénominations commerciales : MPEG 350, MPEG 550, S10W, S20W.
- les poly(éthylène glycol) monomethyl éther, mono(succinimidyl succinate) ester de poids moléculaire moyen 1900 ou 5000, de chez Polysciences;
- le méthacrylate de behenyl poly(éthylène-glycol PEG-25), disponible chez Rhodia, sous la dénomination SIPOMER BEM;
- les acrylates de poly(éthylène glycol) phényl éther de poids moléculaires moyens 236, 280 ou 324 disponibles chez Aldrich;
- le méthoxy polyéthylène glycol 5000 2-(vinyl sulfonyl) éthyl éther disponible commercialement chez Fluka;
- le méthacrylate de polyéthylène glycol éthyl éther disponible chez Aldrich ;
- les méthacrylates de polyéthylène glycol 8000, 4000, 2000 de Monomer & Polymer Dajac laboratories ;
- le polyéthylène glycol N-hydroxy succinimide vinyl sulfone disponible commercialement chez Nektar Molecule enginnering ( Shearwater).

Le monomère de formule (I), seul ou en mélange, est présent à raison de 10 à 80% en poids, par rapport au poids du polymère final, notamment de 20 à 60% en poids, de préférence de 30 à 50% en poids.

Le copolymère éthylénique selon l'invention comprend également au moins un monomère cationique, ou l'un de ses sels, choisi parmi les monomères de formule (IIa).

Il peut en outre comprendre un ou plusieurs monomères ioniques additionnels, choisis parmi les monomères amphotères de formules (IIc) et (IId), et/ou les monomères anioniques choisis parmi l'anhydride maléique et/ou ceux de formule (IIb).

Par monomère cationique, on entend un monomère comprenant des motifs capables de posséder une charge cationique dans le domaine de pH compris entre 3 et 12. Ces motifs ne possèdent pas obligatoirement une charge permanente quelque soit le pH. L'unité cationique n'a pas besoin d'être protonée à chacun des ces pH.

Le monomère cationique, ou l'un de ses sels, est donc choisi parmi les monomères de formule (IIa) : 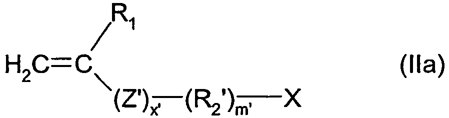 dans laquelle :
- R1 est un atome d'hydrogène ou un radical hydrocarboné, linéaire ou ramifié, de type CₚH₂ₚ₊₁, avec p étant un entier compris entre 1 et 12, inclus;
   Notamment, R1 peut représenter un radical méthyle, éthyle, propyle, butyle. De préférence, R1 représente l'hydrogène ou un radical méthyle.
- Z' est un groupement divalent choisi parmi -COO-, -CONH-, -CONCH₃-, -OCO-ou -O-, -SO₂- -CO-O-CO- ou -CO-CH₂-CO-;
   De préférence, Z' est choisi parmi COO et CONH.
- x' est 0 ou 1, de préférence 1.
- R'2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
Dans le radical R'2, le ou les hétéroatomes, quand ils sont présents, peuvent être intercalés dans la chaîne dudit radical R'2, ou bien ledit radical R'2 peut être substitué par un ou plusieurs groupes les comprenant tels que hydroxy ou amino (NH2, NHR' ou NR'R" avec R' et R" identiques ou différents représentant un alkyle linéaire ou ramifié en C1-C22, notamment méthyle ou éthyle).
Notamment R'2 peut être :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 25 hétéroatomes choisis parmi N, O, S, F, Si et/ou P; ou bien un radical benzylène -C₆H₄-CH₂-éventuellement substitué, par un radical alkyle en C1-C12 comprenant éventuellement 1 à 25 hétéroatomes choisis parmi O, N, S, F, Si et P;
- un radical de formule -CH₂-O-CO-O-, CH₂-CH₂-O-CO-O-, -CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, - CH₂-CH₂-O-CO-NH-; -CH₂-NH-CO-NH- ou -CH₂-CH₂-NH-CO-NH-, -CH₂-CHOH-, - CH₂-CH₂-CHOH-, -CH₂-CH₂-CH(NH₂)-, -CH₂-CH(NH₂)-, -CH₂-CH₂-CH(NHR')-, - CH₂-CH(NHR')-, -CH₂-CH₂-CH(NR'R")-, -CH₂-CH(NR'R")-, -CH₂-CH₂-CH₂-NR'-, - CH₂-CH₂-CH₂-O-; -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux;
- m'est 0 ou 1;
- X est un groupe guanidino, amidino, ou bien un groupe de formule -N(R₆)(R₇) ou -P(R₆)(R₇) ou -P⁺R₆R₇R₈, avec R6, R7 et R8 représentant, indépendamment l'un de l'autre, soit (i) un atome d'hydrogène, soit (ii) un groupement alkyle linéaire, ramifié ou cyclique, saturé ou insaturé, éventuellement aromatique, comprenant de 1 à 18 atomes de carbone, pouvant comprendre 1 à 10 hétéroatomes choisis parmi O, N, S, F, Si et P; soit (iii) R6 et R7 peuvent former avec l'atome d'azote ou de phosphore, un premier cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O, S et N.

Par exemple, R6 et R7 peuvent être choisis parmi l'hydrogène, un groupement méthyle, éthyle, propyle, isopropyle, n-butyle, t-butyle, isobutyle, octyle, lauryle, stéaryle.
De préférence R6 et R7 sont choisis, indépendamment l'un de l'autre, parmi H, CH3 et C2H5.

De manière alternative, X peut représenter un groupement -R'6-N-R'7- dans lequel R'6 et R'7 forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N.
Par exemple, X peut constituer un cycle aromatique ou non comportant un groupement amine tertiaire ou peut représenter un hétérocycle aromatique ou non, contenant un azote tertiaire.
Parmi ces radicaux X préférés, on peut citer les radicaux de type pyridine, indolyle, isoindolinyle, imidazolyle, imidazolinyle, piperidinyl, pyrazolynyl, pyrazolyl, quinoline, pyrazolinyl, pyridinyl, piperazinyl, pyrrolidinyl, quinidinyl, thiazolinyl, morpholine, guanidino, amidino, et leurs mélanges.

Les groupements guanidino et amidino sont respectivement de formule :

| | |
|---|---|
| 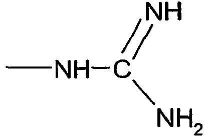 | 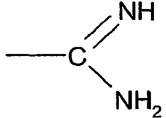 |
| Guanidino | Amidino |

Les motifs cationiques (amines) du monomère de formule (IIa) peuvent éventuellement être neutralisés à ce stade, de la manière décrite plus loin.

Parmi les monomères de formule (IIa) préférés, on peut citer :

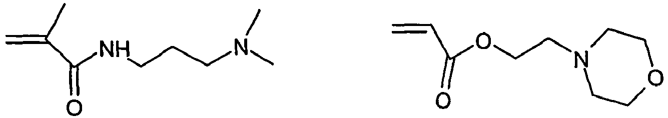

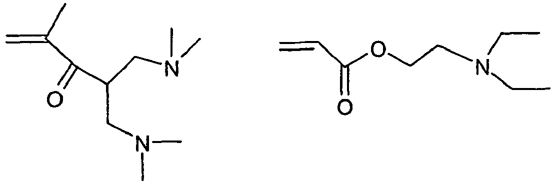

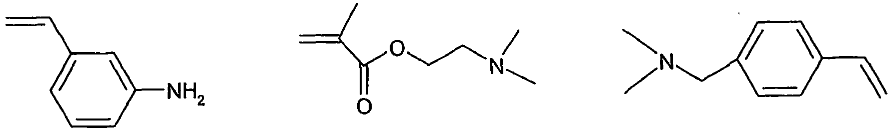

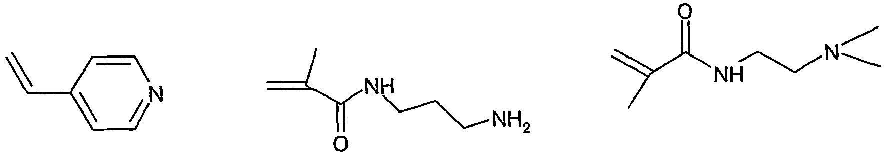

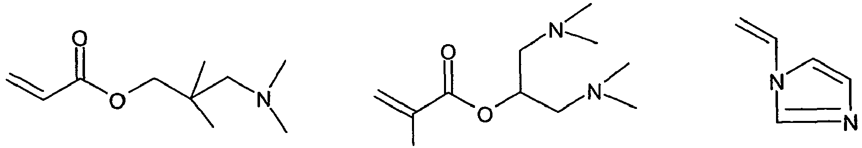

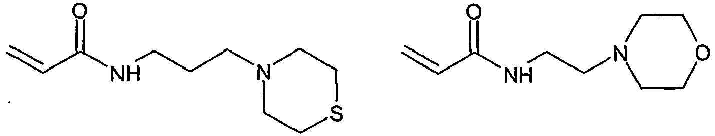

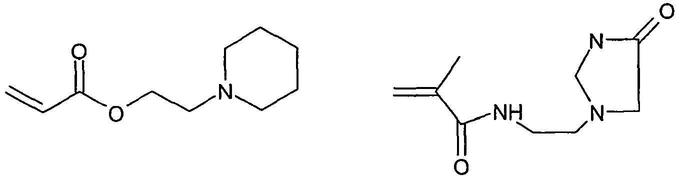

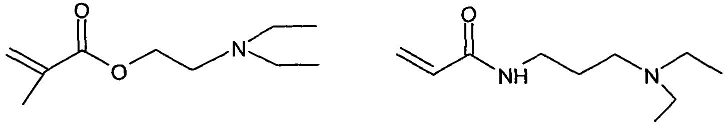

Parmi les monomères de formule (IIa) particulièrement préférés, on peut citer le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle, et leurs mélanges.

Le copolymère peut, bien évidemment, comprendre un mélange de différents monomères cationiques de formule (IIa).

Les monomères cationiques de formule (IIa) sont de préférence présents à raison de 20 à 90% en poids par rapport au poids du polymère final, notamment de 40 à 80% en poids, de préférence de 50 à 70% en poids.

Le copolymère éthylénique peut en outre comprendre au moins un monomère ionique additionnel, choisi parmi les monomères amphotères de formule (IIc) et (IId), et/ou les monomères anioniques choisis parmi l'anhydride maléique et/ou ceux de formule (IIb): 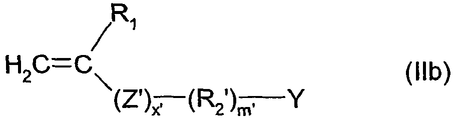 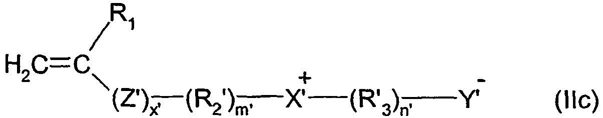 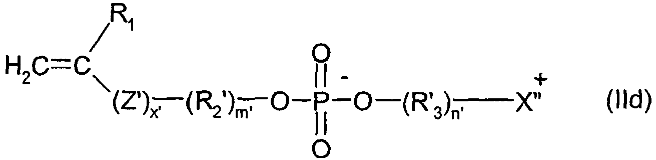 dans lesquelles :
- R1, Z', x', R'2 et m' ont la même signification que celle donnée ci-dessus pour la formule (IIa);
- Y est un groupement choisi parmi -COOH, -SO₃H, -OSO₃H, -PO₃H₂ et -OPO₃H₂. Il est entendu que selon l'état de l'art, les groupes SO₄H₂ et PO₄H₂ sont liés à R'2 par l'atome d'oxygène tandis que les groupes SO₃H et PO₃H sont liés à R'2 respectivement via les atomes de S et P.
- X'⁺ est un groupe divalent de formule -N⁺(R₆)(R₇)- avec R6 et R7 représentant, indépendamment l'un de l'autre, soit (i) un atome d'hydrogène, soit (ii) un groupement alkyle linéaire, ramifié ou cyclique, éventuellement aromatique, comprenant de 1 à 25 atomes de carbone, pouvant comprendre 1 à 20 hétéroatomes choisis parmi O, N, S et P; soit (iii) R6 et R7 peuvent former avec l'atome d'azote un premier cycle, saturé ou insaturé, éventuellement aromatique, comprenant au total 5, 6, 7 ou 8 atomes, et notamment 4, 5, 6 ou 7 atomes de carbone et/ou 2 à 3 hétéroatomes choisi parmi O, S et N; ledit premier cycle pouvant être fusionné avec un ou plusieurs autres cycles, saturés ou insaturés, éventuellement aromatiques, comprenant chacun 5, 6, 7 ou 8 atomes, et notamment 4, 5, 6 ou 7 atomes de carbone et/ou 2 à 3 hétéroatomes choisi parmi O, S et N.

Par exemple, R6 et R7 peuvent être choisis parmi l'hydrogène, un groupement méthyle, éthyle, propyle, isopropyle, n-butyle, t-butyle ou isobutyle.

Parmi les radicaux X'⁺ préférés, on peut citer les radicaux de type pyridine, indolyle, isoindolinyle, imidazolyle, imidazolinyle, piperidinyl, pyrazolynyl, pyrazolyl, quinoline, pyrazolinyl, pyridinyl, piperazinyl, pyrrolidinyl, quinidinyl, thiazolinyl, morpholine, guanidino, amidino, et leurs mélanges.
- Y'⁻ est un groupement choisi parmi -COO-, -SO₃^{-,} -OSO₃⁻, -PO₃²⁻ et -OPO₃²⁻.
- R'3 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P.
Dans le radical R'3, le ou les hétéroatomes, quand ils sont présents, peuvent être intercalés dans la chaîne dudit radical R'3, ou bien ledit radical R'3 peut être substitué par un ou plusieurs groupes les comprenant tels que hydroxy ou amino (NH₂, NHR' ou NR'R" avec R' et R" identiques ou différents représentant un alkyle linéaire ou ramifié en C1-C18, notamment méthyle ou éthyle).
Notamment R'3 peut être :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 5 hétéroatomes choisis parmi O, N, S, F, Si et P; ou bien un radical benzylène -C₆H₄-CH₂- éventuellement substitué, par un radical alkyle en C1-C12 comprenant éventuellement 1 à 5 hétéroatomes choisis parmi O, N, S, F, Si et P;
- un radical de formule -CH₂-O-CO-O-, CH₂-CH₂-O-CO-O-, -CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, - CH₂-CH₂-O-CO-NH-; -CH₂-NH-CO-NH- ou -CH₂-CH₂-NH-CO-NH-, -CH₂-CHOH-, - CH₂-CH₂-CHOH-, -CH₂-CH₂-CH(NH₂)-, -CH₂-CH(NH₂)-, -CH₂-CH₂-CH(NHR')-, - CH₂-CH(NHR')-, -CH₂-CH₂-CH(NR'R")-, -CH₂-CH(NR'R")-, -CH₂-CH₂-CH₂-NR'-, - CH₂-CH₂-CH₂-O-; -[CH₂-CH₂-O]ₙ- et -[CH₂-CH(CH₃)-O]ₙ-, -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux;
- n' est compris entre 1 et 100, de préférence 1 et 5 inclus.
- X"⁺ est un groupement de formule -N⁺R₆R₇R₈ avec R6, R7 et R8 représentant, indépendamment l'un de l'autre, soit (i) un atome d'hydrogène, soit (ii) un groupement alkyle linéaire, ramifié ou cyclique, éventuellement aromatique, comprenant de 1 à 18 atomes de carbone, pouvant comprendre 1 à 5 hétéroatomes choisis parmi O, N, S et P; soit (iii) R6 et R7 peuvent former avec l'atome d'azote un premier cycle, saturé ou insaturé, éventuellement aromatique, comprenant au total 5, 6 ou 7 atomes, et notamment 4, 5 ou 6 atomes de carbone et/ou 2 à 3 hétéroatomes choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 3 hétéroatomes choisi parmi O, S et N.
Par exemple, R6, R7 et R8 peuvent être choisis parmi l'hydrogène, un groupement méthyle, éthyle, propyle, isopropyle, n-butyle, t-butyle, isobutyle, octyle, lauryle ou stéaryle.

Parmi les radicaux X"⁺ préférés, on peut citer les radicaux triméthylammonium; triéthylammonium; N,N-diméthyl,N-octylammonium; N,N-diméthyl,N-laurylammonium.

Parmi les monomères anioniques préférés, on peut citer l'anhydride maléique, l'acide acrylique, l'acide méthacrylique, l'acide crotonique, l'acide itaconique, l'acide fumarique, l'acide maléique, l'acrylate de 2-carboxyéthyle (CH2=CH-C(O)-O-(CH₂)₂-COOH); l'acide styrènesulfonique, l'acide 2-acrylamido-2-methylpropanesulfonique, l'acide vinylbenzoïque, l'acide vinylphosphorique, le (méth)acrylate de sulfopropyle, et les sels de ceux-ci.

Parmi les monomères de formule (IIc) ou (IId) préférés, on peut citer la N,N-dimethyl-N-(2-methacryloyloxyethyl)-N-(3-sulfopropyl) ammonium bétaïne (notamment la SPE de la société Raschig); la N,N-dimethyl-N-(3-methacrylamidopropyl)-N-(3-sulfopropyl) ammonium bétaïne (SPP de Raschig), et la 1-(3-sulfopropyl)-2-vinylpyridinium bétaïne (SPV de Raschig), ainsi que la 2-méthacryloyloxyéthylphosphorylcholine.

Le copolymère peut, bien évidemment, comprendre un mélange de différents monomères ioniques additionnels.

Lorsque le copolymère comprend un monomère ionique additionnel, ou un mélange de monomères ioniques additionnels, lesdits monomères ioniques additionnels sont de préférence présents à raison de 5 à 40% en poids, par rapport au poids du mélange "cationique(s) + ionique(s) additionnel(s)", notamment de 10 à 30% en poids, de préférence de 15 à 25% en poids.

Le(s) monomère(s) ionique(s), à savoir le/les monomère(s) cationique(s) + le/les optionnel(s) monomère(s) ionique(s) additionnel(s), sont de préférence présents à raison de 20 à 90% en poids par rapport au poids du polymère final, notamment de 40 à 80% en poids, de préférence de 50 à 70% en poids.

De préférence, le polymère selon l'invention comprend les monomères de formule (I) et les monomères ioniques (les monomères cationiques + les éventuels monomères amphotères et anioniques) en un rapport pondéral pouvant aller de 60/40 à 40/60, avec une préférence pour un rapport 50/50.

Le copolymère éthylénique selon l'invention peut également comprendre, de manière optionnelle, des monomères additionnels non ioniques. Lorsqu'il comprend de tels monomères additionnels non ioniques, ceux-ci sont préférentiellement choisis parmi les monomères dits hydrophiles au sens de la présente invention. Par monomère hydrophile, on entend dans la présente description, les monomères ayant une valeur du logarithme du coefficient de partage apparent octanol-1/eau, aussi appelé log P, inférieure ou égale à 2, par exemple comprise entre -8 et 2, de préférence inférieure ou égale à 1,5, notamment inférieure ou égale à 1, et en particulier comprise entre -7 et 1, voire entre -6 et 0.

Les valeurs de log P sont connues et sont déterminées selon un test standard qui détermine la concentration du monomère dans l'octanol-1 et l'eau.
Les valeurs peuvent notamment être calculées à l'aide du logiciel ACD (Advanced Chemistry Development) Software solaris V4.67; elles peuvent également être obtenues à partir de Exploring QSAR : hydrophobic, electronic and steric constants (ACS professional reference book, 1995). Il existe encore un site Internet qui fournit des valeurs estimées (adresse : http://esc.syrres.com/interkow/kowdemo.htm).
Nous indiquons ci-après la valeur du log P de certains monomères usuels, déterminée à l'aide du logiciel ACD :

| | méthacrylate (* ou méthacrylamide) | acrylate (* ou acrylamide) |
|---|---|---|
| Méthyl (méth)acrylate | 1.346 +/- 0.250 | 0.793 +/- 0.223 |
| Ethyl (méth)acrylate | 1.877 +/- 0.250 | 1.325 +/- 0.223 |
| Propyl (méth)acrylate | 2.408 +/- 0.250 | 1.856 +/- 0.223 |
| Isopropyl (méth)acrylate | 2.224 +/- 0.254 | 1.672 +/- 0.228 |
| Butyl (méth)acrylate | 2.940 +/- 0.250 | 2.387 +/- 0.223 |
| Isobutyl (méth)acrylate | 2.756 +/- 0.254 | 2.208+/-0.228 |
| terbutyl(méth)acrylate | 2.574 +/- 0.261 | 2.022 +/- 0.238 |
| cyclohexyle (méth)acrylate | 3.405 +/-0.252 | 2.853+/- 0.226 |
| octyl(méth)acrylate | 5.065 +/- 0.521 | 4.513 +/- 0.224 |
| lauryl(méth)acrylate | 7.190 +/- 0.251 | 6.638 +/- 0.224 |
| tridecyl(méth)acrylate | 7.712+/-0.251 | 7.170+/- 0.224 |
| cétyl (méth)acrylate | 9.316+/-0.251 | 8.764+/- 0.224 |
| palmityl(méth)acrylate | >9 | >9 |
| stéaryl (méth)acrylate | 10.379+/- 0.251 | 9.826+/-0.224 |
| behenyl (méth)acrylate | 11.952 +/- 0.225 | 12.504 ± 0.251 |
| oléyl (méth)acrylate | >9 | 9.308 ± 0.232 |
| Tetrahydrofurfuryl (méth)acrylate | 1,352 ± 0,283 | 0,800 ± 0,263 |
| 2-ethyl hexyl (méth)acrylate | 4,881 ± 0,254 | 4,329 ± 0,229 |
| 2-hydroxyethyl (méth)acrylate | 0,718 ± 0,277 | 0,166 ± 0,258 |
| éthoxyéthyle (méth)acrylate | 1,887 ± 0,293 | 1,335 ± 0,268 |
| Hydroxypropyl (méth)acrylate | | 0,383 ± 0,241 |
| N-isopropyle (méth)acrylamide * | 0,748 ± 0,276 | 0,195 ± 0,256 |
| N-octyl (méth)acrylamide * | 3,558 ± 0,273 | 3,036 ± 0,253 |
| N,N-diméthyl (méth)acrylamide* | 0,906 ± 0,553 | -0,168 ± 0,556 |
| N,N-dibutyl (méth)acrylamide* | 3,573 ± 0,570 | 3,021 ± 0,557 |
| | | |
| Acétate de vinyle | 0,730 ± 0,286 | |
| méthyl vinyl éther | 0,509 ± 0,286 | |
| éthyl vinyl éther | 1,040 ± 0,286 | |
| vinylcaprolactame | 1,499 ± 0,207 | |
| Vinylpyrrolidone | 0,370 ± 0,206 | |
| N-vinyl acétamide | 0 ± 0,231 | |

Les monomères hydrophiles non ioniques additionnels peuvent notamment être choisis parmi ceux de formule (III), seuls ou en mélange, : 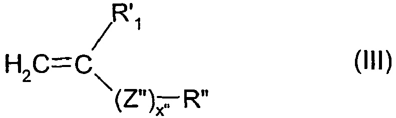 dans lequel :
- R'₁ est l'hydrogène ou -CH₃;
- Z" est un groupement divalent choisi parmi -COO-, -CONH-, -CONCH₃-, -OCO-, - SO₂ , -CO-O-CO-, -CO-CH₂-CO- ou -O- ; de préférence COO et CONH;
- x" est 0 ou 1;
- R" est un radical carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
Dans le radical R", le ou les hétéroatomes, quand ils sont présents, peuvent être intercalés dans la chaîne dudit radical ou bien ledit radical peut être substitué par un ou plusieurs groupes les comprenant tels que hydroxy, ester, amide, uréthane ou urée.
Notamment R" peut être un radical méthyle, éthyle, propyle, isopropyle, n-butyle, isobutyle, tertiobutyle, phényle, benzyle, ou un radical de formule -CH₂-CH₂-CH₂OH -CH₂-CH₂-OH, -CH₂-CH₂-CH₂OH ou furfuryle.

Les monomères non ioniques hydrophiles additionnels sont notamment choisis parmi les monomères ci-après : le méthacrylate de méthyle, l'acrylate de méthyle, le méthacrylate d'éthyle, l'acrylate d'éthyle, l'acrylate de propyle, l'acrylate d'isopropyle, le méthacrylate de tetrahydrofurfuryle, l'acrylate de tetrahydrofurfuryle, le méthacrylate de 2-hydroxyéthyle, l'acrylate de 2-hydroxyéthyle, le méthacrylate d'éthoxyéthyle, l'acrylate d'éthoxyéthyle, le N-isopropyl acrylamide, le N-isopropyl méthacrylamide, le N,N-diméthyl acrylamide, le N,N-diméthyl méthacrylamide, l'acétate de vinyle, le méthyl vinyl éther, l'éthyl vinyl éther, la vinylpyrrolidone, la vinylcaprolactame, la N-vinyl acétamide, l'acrylate d'hydroxypropyle, la N-vinyllactame, l'acrylamide, la N-méthyl acrylamide, la N,N-diméthyl acrylamide, la N-méthyl-N-vinylacétamide, le N-vinylformamide, le N-méthyl-N-vinylformamide, l'alcool vinylique (copolymérisé sous forme d'acétate de vinyle puis hydrolysé).

Le monomère hydrophile non ionique additionnel, seul ou en mélange, peut ne pas être présent dans le polymère selon l'invention (0%), ou bien peut être présent en une quantité pouvant aller jusqu'à 50% en poids, par rapport au poids du polymère final; notamment il peut être présent en une quantité de 0,1 à 35% en poids, de préférence de 1 à 25% en poids, par exemple de 3 à 15% en poids, voire de 5 à 9,5% en poids, par rapport au poids total du polymère.
Toutefois, on a constaté que lorsque ce monomère hydrophile non ionique additionnel était choisi parmi l'acrylate de méthyle, le méthacrylate de méthyle et de l'acrylate d'isopropyle, ces monomères, de préférence, ne pouvaient être présents en une quantité supérieure ou égale à 10% en poids. Ces monomères peuvent, de préférence, donc être présents à raison de 0-9,5% en poids dans le polymère final, notamment de 0,1 à 8% en poids, de préférence de 1 à 5% en poids.

Dans un mode de réalisation particulier de l'invention, le polymère est constitué essentiellement de monomère de formule (I), seul ou en mélange, et de monomères de formule (IIa), seul ou en mélange; ledit copolymère étant neutralisé comme défini plus loin.

Le copolymère selon l'invention est, lorsqu'il est prêt à être employé pour préparer des compositions selon l'invention par exemple, dans un état neutralisé (ses motifs amines sont neutralisés) par au moins un agent neutralisant particulier choisi parmi les acides organiques au sens de Bronsted.
On entend par là que les monomères, notamment ceux de formule (IIa) et/ou le polymère, ont été neutralisés par au moins un tel agent neutralisant.

Il est ainsi possible de neutraliser les motifs amines des monomères entrant dans la constitution du polymère, en particulier les motifs amines des monomères cationiques de formule (IIa), avant leur polymérisation, puis de copolymériser les monomères neutralisés afin d'obtenir le polymère selon l'invention; on parlera alors de pré-neutralisation.
On peut également tout d'abord polymériser les monomères non neutralisés, puis neutraliser le polymère après sa formation.
De préférence, on neutralise le polymère après sa formation.

Les agents neutralisants de type acides organiques peuvent être choisis parmi les acides aliphatiques linéaires, ramifiés ou cycliques et/ou les acides insaturés ou aromatiques, et peuvent notamment comprendre 1 à 1000 atomes de carbone, notamment 2 à 500 atomes de carbone. Ils possèdent au moins une fonction acide au sens de Bronsted, et notamment un ou plusieurs groupes acide carboxylique, sulfonique et/ou phosphonique. Ils peuvent comporter, en outre, un ou plusieurs hétéroatomes choisis parmi O, N, Si, F et P, par exemple sous la forme de groupes hydroxyle.

On peut en particulier employer comme neutralisant des acides gras ayant 6 à 32 atomes de carbone, notamment 8 à 28, linéaires, ramifiés ou cycliques, saturés ou insaturés, éventuellement aromatiques, et comportant au moins une fonction COOH ou acide sulfonique (-SO₃H)
On peut également employer des hydroxyacides, notamment des alphahydroxy-acides, ayant 6 à 32 atomes de carbone, notamment 8 à 28, linéaires, ramifiés ou cycliques, saturés ou insaturés, éventuellement aromatiques, et comportant au moins une fonction COOH ou acide sulfonique (-SO₃H).
On peut encore employer des acides alkylbenzène-sulfoniques, dans lesquels le groupement alkyle peut comprendre de 4 à 30, notamment 6 à 24, atomes de carbone.
On peut encore employer des agents neutralisants amphotères, notamment du type alkyl-bétaïnes ou alkylamidopropylbétaïnes, dans lesquels le groupement alkyle peut comprendre 1 à 30, notamment 4 à 24, voire 6 à 22 atomes de carbone; on peut en particulier citer la cocoamidopropylbétaïne.

On peut notamment citer l'acide alpha-hydroxyéthanoïque, l'acide alphahydroxyoctanoïque, l'acide alpha-hydroxycaprylique, l'acide ascorbique, l'acide acétique, l'acide benzoïque, l'acide béhénique, l'acide caprique, l'acide citrique, l'acide caproïque, l'acide caprylique, l'acide dodécylbenzène sulfonique, l'acide 2-éthylcaproïque, l'acide folique, l'acide fumarique, l'acide galactarique, l'acide gluconique, l'acide glycolique, l'acide 2-hexadécyl eicosanoïque, l'acide hydroxycaproique, l'acide 12-hydroxystéarique, l'acide isolaurique (ou 2-butyl octanoïque), l'acide isomyristique (ou 2-hexyl octanoïque), l'acide isoarachidique (ou 2-octyl dodécanoïque), l'acide isolignocérique (ou 2-décyl tétradécanoïque), l'acide lactique, l'acide laurique, l'acide malique, l'acide myristique, l'acide oléïque, l'acide palmitique, l'acide propionique, l'acide sébacique, l'acide stéarique, l'acide tartrique, l'acide téréphtalique, l'acide trimésique, l'acide undécylénique, la propylebétaïne, la cocoamidopropylbétaïne, et le chlorhydrate de bétaïne de formule [(CH₃)₃N+CH₂CO₂H.Cl-], ainsi que leurs mélanges.

De préférence, on peut utiliser comme agent neutralisant, l'acide caproïque, l'acide 2-éthylcaproïque, l'acide oléïque, l'acide béhénique, l'acide stéarique, l'acide acétique, l'acide citrique, l'acide tartrique, le chlorhydrate de bétaïne et/ou l'acide gluconique, et préférentiellement le chlorhydrate de bétaïne et/ou l'acide béhénique.

De manière avantageuse, l'agent neutralisant peut avoir un log P inférieur ou égal à 2, par exemple compris entre -8 et 2, de préférence compris entre -6 et 1, notamment compris entre -6 et 0.
Il peut toutefois avoir un log P supérieur à 2, de préférence supérieur ou égal à 2,5, notamment supérieur à 3, et en particulier compris entre 3 et 15, voire entre 3,5 et 10.
Les valeurs de log P sont connues et sont déterminées selon un test standard qui détermine la concentration du monomère dans l'octanol-1 et l'eau, ainsi que décrit ci-dessus.

A titre d'information, on donne ci-après la valeur de log P de certains acides usuels :

| | |
|---|---|
| Acide acétique | -0.285+/-0.184 |
| Acide propionique | 0.246+/-0.184 |
| Acide citrique | - 1.721 +/-0.396 |
| Acide gluconique | -3.175 +/- 0.852 |
| Acide benzoïque | 1.895+/-0.206 |
| Acide stéarique | 8.216 +/-0.186 |
| Acide béhénique | 10.342+-0.186 |
| Acide oléique | 7.698 +/- 0.199 |

Par neutralisation, on entend l'action d'un acide, organique selon l'invention, et comprenant au moins une fonction acide au sens de Bronsted, sur tout ou partie des monomères et/ou polymère ci-dessus mentionné, comprenant au moins une fonction basique au sens de Bronsted.

L'agent neutralisant, seul ou en mélange, peut être ajouté en une quantité de 0,01 à 3 équivalent molaire, notamment 0,05 à 2,5, voire 0,1 à 2 équivalent molaire, par rapport aux fonctions amines totales du polymère ou des monomères.

Il est ainsi possible de neutraliser partiellement le polymère, c'est-à-dire que l'agent neutralisant peut être présent en une quantité nécessaire pour neutraliser 1 à 99%, notamment 5 à 90%, voire 10 à 80%, des fonctions amines totales du polymère ou des monomères; ce qui signifie qu'il est présent en une quantité de 0,01 à 0,99 équivalent molaire, notamment 0,05 à 0,9, voire 0,1 à 0,8 équivalent molaire.
Il est également possible de "sur-neutraliser" le polymère, c'est-à-dire que l'agent neutralisant peut être présent en une quantité nécessaire pour neutraliser 101 à 300%, notamment de 120 à 250%, voire de 150 à 200%, des fonctions amines totales du polymère ou des monomères; ceci peut être le cas lorsque l'on souhaite assurer au polymère une gamme de pH et/ou une force ionique adéquate vis-à-vis des formulations envisagées. Il peut donc être présent en une quantité de 1,01 à 3 équivalent molaire, notamment 1,2 à 2,5, voire 1,5 à 2 équivalent molaire, par rapport aux fonctions amines totales du polymère ou des monomères.
De préférence, l'agent neutralisant, seul ou en mélange, est présent en une quantité stoechiométrique par rapport aux fonctions amines totales du polymère ou des monomères; il est donc présent en une quantité nécessaire pour neutraliser 100% des motifs amines du polymère ou des monomères, soit 1 équivalent molaire.

Préférentiellement, la nature et la quantité d'agent neutralisant peut être déterminée par l'homme du métier de manière à obtenir au final un polymère soluble ou dispersible dans l'eau.

Tout particulièrement, on préfère les polymères dans lesquels:
- le monomère de formule (I), seul ou en mélange, est présent à raison de 10 à 80% en poids, par rapport au poids du polymère final, notamment de 20 à 60% en poids, de préférence de 30 à 50% en poids, et est choisi parmi, seul ou en mélange, les (méth)acrylates de poly(éthylène glycol) de préférence ceux ayant un poids moléculaire compris entre 350 et 13 000 g/mol., notamment entre 500 et 8000 g/mol.; et
- le monomère 'essentiellement cationique' est présent à raison de 20 à 90% en poids par rapport au poids du polymère final, notamment de 40 à 80% en poids, de préférence de 50 à 70% en poids et est choisi parmi, seul ou en mélange, le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle; et
- le polymère est neutralisé par un agent neutralisant choisi parmi l'acide 2-éthylcaproïque, l'acide oléïque, l'acide béhénique, l'acide stéarique, l'acide acétique, l'acide citrique, l'acide tartrique, le chlorhydrate de bétaïne et/ou l'acide gluconique, et préférentiellement l'acide béhénique et/ou le chlorhydrate de bétaïne.

Encore plus particulièrement, on préfère les polymères dans lesquels:
- le monomère de formule (I), seul ou en mélange, est présent à raison de 10 à 80% en poids, par rapport au poids du polymère final, notamment de 20 à 60% en poids, de préférence de 30 à 50% en poids, et est choisi parmi, seul ou en mélange, les (méth)acrylates de poly(éthylène glycol) de préférence ceux ayant un poids moléculaire compris entre 350 et 13 000 g/mol., notamment entre 500 et 8000 g/mol.; et
- le monomère 'essentiellement cationique' est présent à raison de 20 à 90% en poids par rapport au poids du polymère final, notamment de 40 à 80% en poids, de préférence de 50 à 70% en poids et est choisi parmi, seul ou en mélange, le (méth)acrylamide de diméthylaminopropyle, et
- le polymère est neutralisé par un agent neutralisant choisi parmi l'acide béhénique et/ou le chlorhydrate de bétaïne.

Les polymères selon l'invention peuvent être préparés selon les méthodes usuelles de polymérisation radicalaire classique, bien connues de l'homme du métier, et telles que décrites par exemple dans l'ouvrage "Chimie et physicochimie des polymères" de Gnanou et al. (édition Dunod).

Notamment ces polymères peuvent être préparés par :
- polymérisation directe en solution dans l'eau avec pré-neutralisation ou non du motif cationique;
- polymérisation en émulsion dans l'eau avec pré-neutralisation ou non du motif cationique, avec utilisation d'un tensioactif;
- polymérisation dans un solvant organique, tel que l'éthanol ou la méthyléthylcétone, avec pré-neutralisation ou non du motif cationique, suivie d'une étape de mise en solution ou dispersion dans l'eau avec évaporation du solvant.
Ces polymérisations peuvent être effectuées en présence d'amorceur radicalaire notamment de type peroxyde (Trigonox 21 S : tert-Butyl peroxy-2-ethylhexanoate) ou azoïque (AIBN V50 : 2,2'-azo-bis(2-amidinopropane) dihydrochlorure) ou persulfate de potassium ou d'ammonium, qui peut être présent à raison de 0,3 à 5% en poids par rapport au poids total des monomères.

Les polymères selon l'invention sont non réticulés. Ils se présentent sous forme de copolymères éthyléniques statistiques, de préférence filmogènes, d'un ou plusieurs monomères éthyléniques contenant des groupes PEG (les groupes PEG sont pendants le long du squelette) et d'un ou plusieurs monomères éthyléniques comportant des fonctions cationiques neutralisées (amines neutralisées et non quaternaires) et, éventuellement d'un ou plusieurs autres comonomères éthyléniques hydrophiles non ioniques, et/ou amphotères, et/ou anioniques, de préférence monovalents.

Par polymère "éthylénique", on entend un polymère obtenu par polymérisation de monomères comprenant une insaturation éthylénique.
Par polymère "filmogène", on entend un polymère apte à former à lui seul ou en présence d'un agent auxiliaire de filmification, un film continu et adhérent sur un support, notamment sur les matières kératiniques.

Ils présentent une masse moléculaire moyenne en poids (Mw) qui est de préférence comprise entre 500 et 5 000 000, notamment comprise entre 1 000 et 3 000 000 et plus préférentiellement comprise entre 2 000 et 2 000 000, voire 4 000 et 500 000, entre autre 7 000 et 400 000, encore mieux 20 000 et 350 000, et encore 150 000 et 300 000. On détermine les masses molaires moyennes en poids (Mw) par chromatographie par perméation sur gel ou par diffusion de la lumière, selon l'accessibilité de la méthode (solubilité des polymères considérés).

Les polymères selon l'invention sont de préférence véhiculables en milieu aqueux, c'est-à-dire qu'ils sont de préférence hydrosolubles ou hydrodispersibles.
La mise en solution ou en dispersion dans l'eau peut être effectuée par solubilisation directe du polymère s'il est soluble, ou bien par neutralisation des motifs amines de façon à rendre le polymère soluble ou dispersible dans l'eau.
La mise en solution ou dispersion aqueuse peut également s'effectuer via une étape intermédiaire de solubilisation dans un solvant organique suivie de l'addition d'eau avant évaporation du solvant organique.

Par ailleurs, on a constaté que les polymères selon l'invention présentent avantageusement une viscosité dans l'eau adéquate avec les applications envisagées, qui peut être, par exemple comprise entre 1 et 1000 mPa.s, de préférence entre 1,5 et 750 mPa.s, et encore mieux entre 2 et 500 mPa.s.
La viscosité est mesurée à l'aide d'un viscosimètre BROOKFIELD, pour une solution à 15% en poids de polymère dans l'eau ou la méthyléthylcétone (solvant choisi en fonction de la solubilité du polymère et/ou de la méthode de polymérisation), à 25°C, avec un mobile de type aiguille (spindle) choisies parmi les modèles numéros 00 à 07 de Brookfield, de préférence le mobile no 1 ; pour un temps de mesure de 5 minutes, à une vitesse comprise entre 0,1 et 6 tours/minute. La viscosité est mesurée après complète dissolution du polymère dans l'eau ou la méthyléthylcétone.

En outre, les polymères selon l'invention peuvent présenter de préférence une température de transition vitreuse (Tg) comprise entre -150°C et 20°C, notamment -120°C et 10°C, mieux entre -100°C et 0°C; la Tg est mesurée selon la méthode donnée avant les exemples.

Les polymères selon l'invention peuvent présenter de préférence une température de fusion (Tf) comprise entre -100°C et 80°C, notamment entre -80°C et 50°C, mieux entre -70°C et 45°C, voire -10°C et 25°C.

En outre, les polymères selon l'invention présentent de préférence une reprise en eau comprise entre 3% et 150% en poids, de préférence entre 4% et 100% en poids, notamment entre 5% et 50% en poids, à 75% d'humidité relative (75%HR); la reprise en eau est mesurée selon la méthode donnée avant les exemples.
Ils peuvent également présenter une reprise en eau comprise entre 3% et 200% en poids, de préférence entre 55% et 150% en poids, notamment entre 60% et 120% en poids, voire entre 70 et 100% en poids, à 85% d'humidité relative (85%HR).

Les polymères selon l'invention trouvent une application toute particulière dans le domaine de la cosmétique. Ils peuvent être présents dans la composition sous forme solubilisée, par exemple dans l'eau ou un solvant organique, ou bien sous forme de dispersion aqueuse ou organique.

Ils peuvent être utilisés dans les compositions cosmétiques ou pharmaceutiques à raison de 0,01 à 50% en poids de matière sèche, notamment de 0,1 à 30% en poids, voire de 0,3 à 10% en poids, encore mieux de 1 à 3% en poids, par rapport au poids total de la composition.

Les compositions cosmétiques ou pharmaceutiques selon l'invention comprennent, outre lesdits polymères, un milieu physiologiquement acceptable, notamment cosmétiquement ou pharmaceutiquement acceptable, c'est-à-dire un milieu compatible avec les matières kératiniques telles que la peau du visage ou du corps, les cheveux, les cils, les sourcils et les ongles.

La composition peut ainsi comprendre, un milieu hydrophile comprenant de l'eau ou un mélange d'eau et de solvant(s) organique(s) hydrophile(s) comme les alcools et notamment les monoalcools, linéaires ou ramifiés en C1-C6, comme l'éthanol, le tertiobutanol, le n-butanol, l'isopropanol ou le n-propanol, et les polyols comme la glycérine, la diglycérine, le propylène glycol, le sorbitol, le pentylène glycol, et les polyéthylène glycols, ou bien encore les éthers de glycols notamment en C₂ et des aldéhydes en C₂-C₄ hydrophiles.

L'eau ou le mélange d'eau et de solvants organiques hydrophiles peut être présent dans la composition selon l'invention en une teneur allant de 0,1% à 99% en poids, par rapport au poids total de la composition, et de préférence de 10% à 80% en poids.

La composition peut également comprendre une phase grasse, notamment constituée de corps gras liquides à température ambiante (25°C en général) et/ou de corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.
Comme corps gras liquides à température ambiante, appelés souvent huiles, utilisables dans l'invention, on peut citer : les huiles hydrocarbonées d'origine animale telles que le perhydrosqualène; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le düsostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol; des alcools gras ayant de 12 à 26 atomes de carbone comme l'octyldodécanol, le 2-butyloctanol, le 2-hexyldécanol, le 2-undécylpentadécanol, l'alcool oléique; les huiles fluorées partiellement hydrocarbonées et/ou siliconées; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes; leurs mélanges.
Ces huiles peuvent être présentes en une teneur allant de 0,01 à 90%, et mieux de 0,1 à 85% en poids, par rapport au poids total de la composition.

La composition selon l'invention peut également comprendre un ou plusieurs solvants organiques, physiologiquement acceptables.

Ces solvants peuvent être généralement présents en une teneur allant de 0,1 à 90%, de préférence de 0,5 à 85%, de préférence encore de 10 à 80% en poids, par rapport au poids total de la composition, et mieux de 30 à 50 %.
On peut notamment citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone; les éthers de propylène glycol liquides à température ambiante tels que le monométhyléther de propylène glycol, l'acétate de monométhyléther de propylène glycol, le mono n-butyl éther de dipropylène glycol; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à 25°C tels que le diéthyléther, le diméthyléther ou le dichlorodiéthyléther; les alcanes liquides à 25°C tels que le décane, l'heptane, le dodécane, l'isododécane, le cyclohexane; les composés cycliques aromatiques liquides à 25°C tels que le toluène et le xylène ; les aldéhydes liquides à 25°C tels que le benzaldéhyde, l'acétaldéhyde et leurs mélanges.

Par cire au sens de la présente invention, on entend un composé lipophile, solide à température ambiante (25°C), à changement d'état solide/liquide réversible, ayant un point de fusion supérieur ou égal à 25°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METTLER.
Les cires peuvent être hydrocarbonées, fluorées et/ou siliconées et être d'origine végétale, minérale, animale et/ou synthétique. En particulier, les cires présentent un point de fusion supérieur à 30°C et mieux supérieur à 45°C. Comme cire utilisable dans la composition de l'invention, on peut citer la cire d'abeilles, la cire de Camauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméthicone ayant de 16 à 45 atomes de carbone.
Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.
La nature et la quantité des corps solides sont fonction des propriétés mécaniques et des textures recherchées. A titre indicatif, la composition peut contenir de 0,1 à 50% en poids de cires, par rapport au poids total de la composition et mieux de 1 à 30% en poids.

La composition selon l'invention peut en outre comprendre, dans une phase particulaire, des pigments et/ou des nacres et/ou des charges habituellement utilisés dans les compositions cosmétiques.
La composition peut également comprendre d'autres matières colorantes choisies parmi les colorants hydrosolubles ou les colorants liposolubles bien connus de l'homme du métier.
Par pigments, il faut comprendre des particules de toute forme, blanches ou colorées, minérales ou organiques, insolubles dans le milieu physiologique, destinées à colorer la composition.
Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.
Par nacres, il faut comprendre des particules de toute forme irisées, notamment produites par certains mollusques dans leur coquille ou bien synthétisées.
Les pigments peuvent être présents dans la composition à raison de 0,01 à 25% en poids de la composition finale, et de préférence à raison de 3 à 10% en poids. Ils peuvent être blancs ou colorés, minéraux ou organiques. On peut citer les oxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium. On peut encore citer les pigments D&C et les laques couramment employées pour conférer aux lèvres et à la peau un effet de maquillage, qui sont des sels de calcium, de baryum, d'aluminium, de strontium ou de zirconium.
Les nacres peuvent être présentes dans la composition à raison de 0,01 à 20% en poids, de préférence à un taux de l'ordre de 3 à 10% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Parmi les colorants, liposolubles ou hydrosolubles, qui peuvent être présents dans la composition, seul ou en mélange, à raison de 0,001 à 15% en poids, de préférence 0,01 à 5% en poids et notamment de 0,1 à 2% en poids, par rapport au poids total de la composition, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène, le carmin de cochenille, les colorants halogéno-acides, azoïques, anthraquinoniques, le sulfate de cuivre ou de fer, le brun Soudan, le rouge Soudan et le rocou, ainsi que le jus de betterave et le carotène.
La composition selon l'invention peut comprendre en outre en outre une ou plusieurs charges, notamment en une teneur allant de 0,01% à 50% en poids, par rapport au poids total de la composition, de préférence allant de 0,02% à 30% en poids. Les charges peuvent être minérales ou organiques de toute forme, plaquettaires, sphériques ou oblongues. On peut citer le talc, le mica, la silice, le kaolin, les poudres de polyamide (Nylon®), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Coming) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition peut également comprendre avantageusement au moins un agent tensioactif qui est généralement présent en une quantité comprise entre 0,01 % et 50% en poids, de préférence entre 0,1 % et 40% et encore plus préférentiellement entre 0,5% et 30%, par rapport au poids total de la composition.
Cet agent tensioactif peut être choisi parmi les agents tensioactifs anioniques, amphotères, non ioniques, cationiques ou leurs mélanges.
Les tensioactifs convenant à la mise en oeuvre de la présente invention sont notamment, seuls ou en mélange, :
- les tensioactifs anioniques parmi lesquels on peut citer, seuls ou mélanges, les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffinesulfonates; les alkylsulfosuccinates, les alkyléthersulfosuccinates, les alkylamide-sulfosuccinates; les alkylsulfosuccinamates; les alkylsulfoacétates ; les alkylétherphosphates; les acylsarcosinates; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle.
On peut également citer les sels d'acides gras tels que les sels des acides oléique, ricinoléique, palmitique, stéarique, les acides d'huile de coprah ou d'huile de coprah hydrogénée; les acyl-lactylates dont le radical acyle comporte 8 à 20 atomes de carbone; les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'éthylène, et leurs mélanges. - les tensioactifs non ioniques parmi lesquels on peut citer, seuls ou mélanges, les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.
On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.
- les tensioactifs amphotères parmi lesquels on peut citer, seuls ou mélanges, les dérivés d'amines secondaires ou'tertiaires aliphatiques, dans lesquels le radical aliphatique est une chaîne linéaire ou ramifiée comportant 8 à 22 atomes de carbone et comprenant au moins un groupe anionique hydrosolubilisant (par exemple carboxylate, sulfonate, sulfate, phosphate ou phosphonate); on peut citer encore les alkyl (C₈-C₂₀) bétaïnes, les sulfobétaïnes, les alkyl (C₈-C₂₀) amidoalkyl (C₁-C₆) bétaïnes telles que la cocoamidopropylbétaïne ou les alkyl (C₈-C₂₀) amidoalkyl (C₁-C₆) sulfobétaïnes.
- les tensioactifs cationiques parmi lesquels on peut citer, seuls ou mélanges,
   A) les sels d'ammonium quaternaires de la formule générale (XVI) suivante : 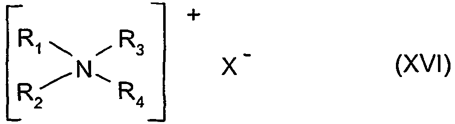 dans laquelle X est un anion choisi parmi les halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate, et
      a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
         R4 désigne un radical alkyle, linéaire ou ramifié, comportant de 16 à 30 atomes de carbone.
         De préférence le tensioactif cationique est un sel (par exemple chlorure) de béhényl triméthyl ammonium.
      b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
         R3 et R4, identiques ou différents, désignent un radical alkyle, linéaire ou ramifié, comportant de 12 à 30 atomes de carbone, ledit radical comprenant au moins une fonction ester ou amide.
         R3 et R4 sont notamment choisis parmi les radicaux alkyl(C₁₂-C₂₂)amido alkyle(C₂-C₆), alkyl(C₁₂-C₂₂)acétate ;
         De préférence le tensioactif cationique est un sel (par exemple chlorure) de stéaramidopropyl diméthyl (myristylacétate) ammonium.
   B) - les sels d'ammonium quaternaire de l'imidazolinium, comme par exemple celui de formule (XVII) suivante : 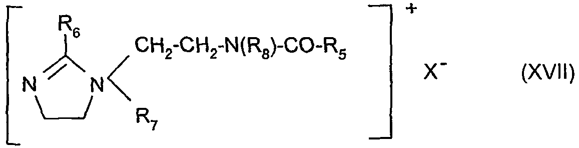 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
   C) - les sels de diammonium quaternaire de formule (XVIII) : 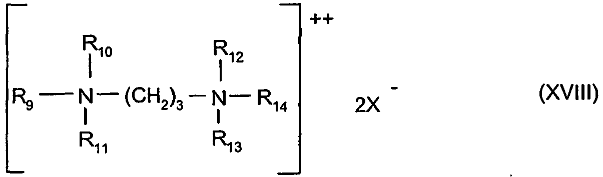 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
   D) - les sels d'ammonium quaternaire comprenant au moins une fonction ester de formule (XIX) suivante : 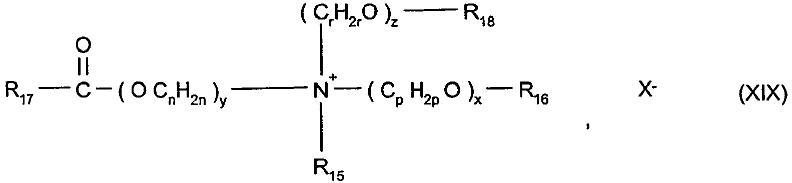 dans laquelle : radicaux
      - R15 est choisi parmi les radicaux alkyles en C₁-C₆ et les radicaux hydroxyalkyles ou dihydroxyalkyles en C1-C6 ;
      - R16 est choisi parmi :
         - le radical 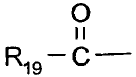
         - les radicaux R20 hydrocarbonés en C1-C22 linéaires ou ramifiés, saturés ou insaturés,
         - l'atome d'hydrogène,
      - R18 est choisi parmi :
         - le radical 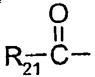
         - les radicaux R22 hydrocarbonés en C1-C6 linéaires ou ramifiés, saturés ou insaturés,
         - l'atome d'hydrogène,
      - R17, R19 et R21, identiques ou différents, sont choisis parmi les radicaux hydrocarbonés en C7-C22, linéaires ou ramifiés, saturés ou insaturés ;
      - n, p et r, identiques ou différents, sont des entiers valant de 2 à 6 ;
      - y est un entier valant de 1 à 10 ;
      - x et z, identiques ou différents, sont des entiers valant de 0 à 10 ;
      - X- est un anion simple ou complexe, organique ou inorganique ;
sous réserve que la somme x + y + z vaut de 1 à 15, que lorsque x vaut 0 alors R16 désigne R20 et que lorsque z vaut 0 alors R18 désigne R22.

La composition selon l'invention peut également comprendre des ingrédients couramment utilisés en cosmétique, tels que les vitamines, les parfums, les agents nacrants, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; Cette composition peut avoir l'aspect d'une lotion, d'une crème, d'une pommade, d'une pâte souple, d'un onguent, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres et des cheveux, d'un produit solaire ou autobronzant, voire d'un produit capillaire. Elle trouve notamment une application particulièrement intéressante dans le domaine capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

Dans un mode de réalisation préféré, les compositions conformes à l'invention peuvent être utilisées pour le lavage ou le traitement des matières kératiniques telles que les cheveux, la peau, les cils, les sourcils, les ongles, les lèvres, le cuir chevelu et plus particulièrement les cheveux.
Les compositions selon l'invention peuvent être des compositions détergentes telles que des shampooings, des gels-douche et des bains moussants. Dans ce mode de réalisation de l'invention, les compositions comprennent au moins une base lavante, généralement aqueuse, qui peut comprendre de 5 à 35% en poids de tensioactif.

L'invention a donc encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.
Ainsi, ce procédé selon l'invention permet le maintien de la coiffure, le traitement, le soin ou le lavage ou le démaquillage de la peau, des cheveux ou de toute autre matière kératinique.

Dans un autre mode de réalisation préféré, les compositions de l'invention peuvent se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.
Lorsque la composition se présente sous la forme d'un après-shampooing éventuellement à rincer, elle contient avantageusement au moins un tensioactif cationique, par exemple en une concentration généralement comprise entre 0,1 et 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.
Les compositions selon l'invention peuvent également se présenter sous forme de lotions aqueuses ou hydroalcooliques pour le soin de la peau et/ou des cheveux. L'invention a aussi pour objet un procédé cosmétique de maquillage ou de soin des matières kératiniques, notamment de la peau du corps ou du visage, des ongles, des cheveux et/ou des cils, comprenant l'application sur lesdites matières d'une composition cosmétique telle que définie précédemment.

L'invention est illustrée plus en détail dans les exemples suivants.

### Mesure de la Tg

Un film est réalisé à partir d'une solution aqueuse à 6% en poids de polymère et séché 48 heures dans une atmosphère contrôlée à 50% d'humidité relative et 25°C. Les films ainsi obtenus ont une épaisseur comprise entre 10 et 20 µm. L'appareil de mesure est une DSC (TA instruments).
L'échantillon issu du film est déposé dans un creuset hermétique et est chauffé selon le protocole suivant :
- équilibre à température initiale Ti;
- chauffe 1 : augmentation de la température, à une vitesse de +10°C/min jusqu'à température finale :Tf (°C);
- isotherme durant 1 minute;
- diminution de la température à une vitesse de -10°C/min jusqu'à Ti (°C);
- chauffe2 : augmentation de la température à une vitesse de +10°C/min jusqu'à Tf (°C);
- isotherme durant 1 minute.
   avec Ti : Température initiale - 120°C
   avec Tf : Température finale + 120°C
   Les Tg sont mesurées lors des étapes de chauffe 1 et 2.

### Mesure de la reprise en eau

On dépose environ 1 g de polymère sec dans une coupelle en aluminium de 4,5 cm de diamètre (0,01 m²). On laisse sécher 48 heures à l'étuve à 60°C, sous pression réduite. On retire les coupelles et on les pèse immédiatement (moins d'une minute après leur sortie de l'étuve). On obtient P1.
Les coupelles sont ensuite placées dans une boite à gant ayant l'humidité relative considérée (75% HR ou 85% HR) et y sont laissées 6 heures. Elles sont ensuite pesées à nouveau immédiatement après leur sortie de la boite à gant. On obtient P2.
La reprise en eau est calculée de la manière suivante : [(P2-P1) x 100] / P1

### Exemple 1

### Etape 1

Dans un réacteur (quadricol) surmonté de deux ampoules d'addition, d'un réfrigérant et muni d'une agitation mécanique, on introduit 75 ml de méthyléthylcétone (MEC), on porte à 80°C.

Parallèlement, on prépare une solution 1 comprenant les monomères : 50 g de méthacrylate de polyéthylèneglycol (MPEG 550), 50 g de diméthylaminopropylméthacrylamide (DMAPMA) et l'amorceur : 0,5 g de (Trigonox 21S).
On prépare également une solution 2 comprenant 75 ml de méthyléthylcétone et 0,5 g d'amorceur (Trigonox 21 S).
La solution 1 est coulée goutte à goutte en 1 heure et la solution 2 en deux heures, dans le réacteur quadricol. Le mélange résultant est maintenu à 80°C ensuite pendant 5 heures. La solution jaune-orangée obtenue est refroidie. On obtient 95 g de polymère.
Le polymère présente une viscosité Brookfield à 15% dans la MEC, à 25°C, mesurée avec un mobile du type aiguille (spindle) numéro 1, à une vitesse de 0,1 tour/minute, de : 7,5 mPa.s.

### Etape 2

On peut ensuite procéder à la neutralisation du polymère de la manière suivante : on ajoute 45 g de chlorhydrate de bétaïne, en solution dans l'eau, à 95 g de polymère; puis on procède à l'évaporation du solvant (MEC) au rotovapeur sous pression réduite (80 mbar et 56°C).
Le polymère neutralisé selon l'invention est soluble dans l'eau (au moins jusqu'à 50% en poids).
Sa Tg est de -66°C. Il présente une reprise en eau à 85% HR de 80%.

### Etape 2 comparative

A titre de comparaison, on procède à la neutralisation du polymère obtenu à l'étape 1 de la manière suivante : on ajoute 290 ml d'HCl 1 N sous agitation à 95 g de polymère, et 200 ml d'eau distillée; puis on procède à l'évaporation du solvant (MEC).
Le polymère neutralisé comparatif est soluble dans l'eau (au moins jusqu'à 50% en poids).
Sa Tg est de -60°C. Il présente une reprise en eau à 85% HR de 51 %.

### Exemple 2

Dans un réacteur (quadricol) surmonté de deux ampoules d'addition, d'un réfrigérant et muni d'une agitation mécanique, on introduit 100 ml d'eau que l'on porte à 80°C.
Parallèlement, on prépare une solution 1 comprenant 50 g de monomère MPEG 550, 1 g d'amorceur (persulfate de potassium KPS) et 50 ml d'eau.
On prépare également une solution 2 comprenant 50 g de monomère DMAPMA neutralisé à 100% par du chlorhydrate de bétaïne et 50 g d'eau.
Les solutions 1 et 2 sont coulées en 1 heure dans le quadricol. Après une heure à 80°C, on y coule goutte à goutte en 15 minutes, un mélange de 1 g de KPS dans 50 ml d'eau.
Le mélange résultant est ensuite maintenu à 80°C pendant 3 heures. On obtient 90 g de polymère neutralisé par le chlorhydrate de bétaïne.

Le polymère présente une viscosité Brookfield à 15% dans l'eau, à 25°C, mesurée avec un mobile du type aiguille (spindle) numéro 1, à une vitesse de 6 tours/minute, de : 164 mPa.s.
Le polymère est soluble dans l'eau (au moins jusqu'à 50% en poids).
Sa Tg est de -60°C. Le polymère neutralisé présente une reprise en eau à 85% HR de 90%.

### Exemples 3 à 25

On prépare, selon le procédé de l'exemple 1 (procédé solvant) ou de l'exemple 2 (procédé dans l'eau), les polymères suivants :
La neutralisation peut être effectuée sur le monomère cationique avant polymérisation (pré-neutralisation), ou sur le polymère (post-neutralisation):

| Exemple | Monomères (% en poids) | Procédé et neutralisation | Solubilité du polymère |
|---|---|---|---|
| Exemple 3 | MPEG 550 50% DMAPMA 50% | Procédé 1 Post-neutralisation chlorhydrate de bétaïne | Soluble dans l'eau |
| Exemple 4 | MPEG 5000 50% DMAPMA 50% | Procédé 2 Pré-neutralisation chlorhydrate de bétaïne | Soluble dans l'eau |
| Exemple 5 | MPEG 5000 75% DMAPMA 25% | Procédé 2 Pré-neutralisation chlorhydrate de bétaïne | Soluble dans l'eau |
| Exemple 6 | MPEG 5000 25% DMAPMA 75% | Procédé 2 Pré-neutralisation chlorhydrate de bétaïne | Soluble dans l'eau |
| Exemple 7 | MPEG 5000 50% DMAPMA 50% | Procédé 1 Post-neutralisation chlorhydrate de bétaïne | Soluble dans l'eau |
| Exemple 8 | MPEG 2000 50% DMAPMA 50% | Procédé 1 Post-neutralisation chlorhydrate de bétaïne | Soluble dans l'eau |
| Exemple 9 | DMAPMA 50% Acrylamide de Jeffamine M-1000 50% | Procédé 1 Post-neutralisation chlorhydrate de bétaïne | Soluble dans l'eau |
| Exemple 10 | MPEG 8000 50% DMAPMA 50% | Procédé 1 Post-neutralisation chlorhydrate de bétaïne | Soluble dans l'eau |
| Exemple 11 | MPEG 550 40% DMAPMA 50% Vinylpyrrolidone 10% | Procédé 1 Post-neutralisation chlorhydrate de bétaïne | Soluble dans l'eau |
| Exemple 12 | MPEG 550 40% DMAPMA 50% Acétate de vinyle 10% | Procédé 1 Post-neutralisation chlorhydrate de bétaïne | Soluble dans l'eau |
| Exemple 13 | MPEG 550 40% DMAPMA 50% Vinylcaprolactame 10% | Procédé 1 Post-neutralisation chlorhydrate de bétaïne | Soluble dans l'eau |
| Exemple 14 | MPEG 550 40% DMAPMA 50% THF MA 10% | Procédé 1 Post-neutralisation chlorhydrate de bétaïne | Soluble dans l'eau |
| Exemple 15 | MPEG 550 50% DMAPMA 50% | Procédé 1 Post-neutralisation acide gluconique (log p = -3.175) | Soluble dans l'eau |
| Exemple 16 | MPEG 550 40% DMAPMA 50% Vinylpyrrolidone 10% | Procédé 1 Post-neutralisation acide gluconique | Soluble dans l'eau |
| Exemple 17 | MPEG 550 40% DMAPMA 50% Acétate de vinyle 10% | Procédé 1 Post-neutralisation acide gluconique | Soluble dans l'eau |
| Exemple 18 | MPEG 550 40% DMAPMA 50% Vinylcaprolactame 10% | Procédé 1 Post-neutralisation acide gluconique | Soluble dans l'eau |
| Exemple 19 | MPEG 550 50% DMAPMA 50% | Procédé 1 Post-neutralisation acide éthyl-caproïque | Soluble dans l'eau |
| Exemple 20 | MPEG 550 40% DMAPMA 50% Vinylpyrrolidone 10% | Procédé 1 Post-neutralisation acide caproïque | Soluble dans l'eau |
| Exemple 21 | MPEG 550 40% DMAPMA 50% Acétate de vinyle 10% | Procédé 1 Post-neutralisation acide caproïque | Soluble dans l'eau |
| Exemple 22 | MPEG 550 40% DMAPMA 50% Vinylcaprolactame 10% | Procédé 1 Post-neutralisation acide caproïque | Soluble dans l'eau |
| Exemple 23 | MPEG 550 40% DMAPMA 50% THF MA 10% | Procédé 1 Post-neutralisation acide caproïque | Soluble dans l'eau |
| Exemple 24 | MPEG 550 50% DMAPMA 50% | Procédé 1 Post-neutralisation acide béhénique à 20% | Dispersible dans l'eau |
| Exemple 25 | MPEG 550 50% DMAPMA 50% | Procédé 1 Post-neutralisation acide oléïque | Dispersible dans l'eau |

| | | | |
|---|---|---|---|
| - MPEG : méthacrylate de polyéthylèneglycol (avec PM = 550, 2000 ou 5000) - DMAPMA : méthacrylamide de diméthylaminopropyle - Acrylamide de Jeffamine M-1000 de formule : 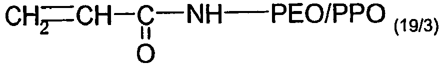 - THF MA : méthacrylate de tetrahydrofurfuryle | | | |

### Exemple 26

On prépare une composition comprenant les constituants suivants (% en poids):
- 7,5% de lauryl éther sulfate
- 2,5% de tensioactif amphotère coco-bétaïne (Dehyton AB30 de Cognis)
- 5% de tensioactif coco-polyglucoside (Plantacare 818 UP de Cognis)
- 1,5% de polymère de l'exemple 3
- qsp 100% eau

La composition de shampooing obtenue apporte un bon effet coiffant. La cosmétique sur cheveux humides est correcte. Les résultats coiffants obtenus après application sur têtes sont bons et les propriétés cosmétiques sur cheveux secs particulièrement bonnes. On constate que cette composition apporte des propriétés cosmétiques équivalentes à celles apportées par un shampooing conditionneur usuel : cosmétique supérieure à l'état de la technique pour un coiffant équivalent; certains critères cosmétiques (lissage, souplesse et brillance) sont meilleurs avec la composition selon l'invention.

### Exemple 27

On prépare une composition comprenant les constituants suivants (% en poids):
- 7,5% de lauryl éther sulfate
- 2,5% de tensioactif amphotère coco-bétaïne
- 5% de tensioactif coco-polyglucoside
- 1,5% de polymère neutralisé de l'exemple 24
- qsp 100% eau

On applique la composition résultante sur têtes en mono-application, et on évalue les critères suivants (panel de 6 personnes):
- sur cheveux humides : le lissage et le démêlage
- sur cheveux secs : ressort de la mèche enroulée et séchée (effet coiffant), démêlage, brillance.

On obtient les résultats suivants :
- sur cheveux humides, le démêlage et le lissage sont très bons.
- sur cheveux secs, l'effet coiffant est bon ; les propriétés cosmétiques sont bonnes, supérieures à l'état de la technique pour un effet coiffant équivalent ; certains critères cosmétiques (lissage, souplesse, brillance) sont meilleurs avec la composition selon l'invention.

Les résultats sont rassemblés dans le tableau ci-après (test effectué pour la composition de l'exemple 27, comprenant le polymère de l'exemple 24, et pour la composition de l'exemple 26, comprenant le polymère de l'exemple 3)

| | Démêlage cheveu humide | Lissage cheveu humide | Démêlage cheveu sec | brillance | ressort |
|---|---|---|---|---|---|
| Composition selon l'exemple 26 | +++ | +++ (souplesse supérieure au témoin) | +++ Bonne | +++ | ++ bon |
| Composition selon l'exemple 27 | ++++ | ++++ | | ++++ | |
| Témoin (shampooing DOP camomille) | ++ | ++ | ++ | ++ | 0 |

### Exemple 28

On prépare une composition comprenant les constituants suivants (% en poids):
- 6% de polymère de l'exemple 1
- 0,001 % de conservateur
- qsp 100% eau

La composition est introduite dans un flacon-pompe; elle est appliquée sur cheveux secs et coiffés et l'on évalue les critère suivants: ressort de la mèche enroulée et séchée (effet coiffant). On constate que le ressort de la boucle et l'effet coiffant sont améliorés par rapport à une mèche traitée avec une composition témoin comprenant 6% de polymère Resyn 28-2930 de National Starch (VA/crotonates/vinyl neodecanoate copolymer), 0,001 % de conservateur et qsp 100% d'eau.

### Exemple 29

On prépare un shampoing comprenant les constituants suivants (% en poids):
- 7,5% de lauryl éther sulfate
- 2,5% de tensioactif amphotère coco-bétaïne (Dehyton AB30 de Cognis)
- 5% de tensioactif coco-polyglucoside (Plantacare 818 UP de Cognis)
- qs conservateur
- 1,5% de matière active de polymère
- qsp 100% eau

On applique la composition résultante sur têtes en mono-application, et on évalue les critères suivants (panel de 6 personnes):
- sur cheveux humides : le lissage, la légèreté et la facilité de démêlage
- sur cheveux secs : ressort de la mèche enroulée et séchée (effet coiffant)

On compare les deux polymères suivants et l'on obtient les résultats suivants :

| Polymère | |
|---|---|
| Selon l'invention | MPEG550/DMAPMA 50/50 neutralisé chlorhydrate de bétaïne |
| Comparatif | MPEG550/DMAPMA 50/50 neutralisé HCl |

Les mèches traitées avec la composition selon l'invention sont plus douces et plus soyeuses; elles semblent plus traitées. La nervosité (ressort) des mèches est équivalente.

## Revendications

1. Copolymère éthylénique non réticulé, comprenant, en % en poids par rapport au poids total du polymère :
- a) 10-80% en poids d'un ou plusieurs monomères de formule (I) : 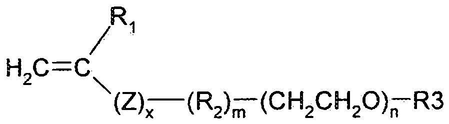 dans laquelle :
- R1 est un atome d'hydrogène ou un radical hydrocarboné, linéaire ou ramifié, de type CₚH₂ₚ₊₁, avec p étant un entier compris entre 1 et 12 inclus;
- Z est un groupement divalent choisi parmi -COO-, -CONH-, -CONCH₃-, -OCO-, - O-, -SO₂- -CO-O-CO- ou -CO-CH₂-CO-;
- x est 0 ou 1;
- R2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m est 0 ou 1
- n est un entier compris entre 3 et 300 inclus;
- R3 est un atome d'hydrogène ou un radical carboné, saturé ou insaturé, éventuellement aromatique, linéaire, ramifié ou cyclique, de 1 à 30 atomes de carbone, pouvant comprendre 1 à 20 hétéroatomes choisis parmi O, N, S, F, Si et P;
et leurs sels.
- b) 20-90% en poids d'au moins un monomère cationique, ou l'un de ses sels, choisi parmi les monomères de formule (IIa) : 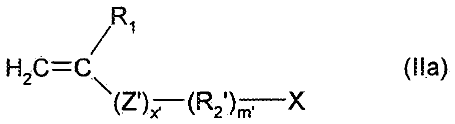 dans laquelle :
- R1 est un atome d'hydrogène ou un radical hydrocarboné, linéaire ou ramifié, de type CₚH₂ₚ₊₁, avec p étant un entier compris entre 1 et 12, inclus; de préférence l'hydrogène ou un radical méthyle, éthyle, propyle, butyle.
- Z' est un groupement divalent choisi parmi -COO-, -CONH,-, -CONCH₃-, -OCO-ou -O-, -SO₂- -CO-O-CO- ou -CO-CH₂-CO-; de préférence, COO et CONH.
- x' est 0 ou 1, de préférence 1.
- R'2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m' est 0 ou 1;
- X est
(a) un groupe guanidino, amidino, ou bien
(b) un groupe de formule -N(R₆)(R₇) ou -P(R₆)(R₇) ou -P+R₆R₇R₈, avec R6, R7 et R8 représentant, indépendamment l'un de l'autre, soit (i) un atome d'hydrogène, soit (ii) un groupement alkyle linéaire, ramifié ou cyclique, saturé ou insaturé, éventuellement aromatique, comprenant de 1 à 18 atomes de carbone, pouvant comprendre 1 à 10 hétéroatomes choisis parmi O, N, S, F, Si et P; soit (iii) R6 et R7 peuvent former avec l'atome d'azote ou de phosphore, un premier cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O, S et N; ou bien
(c) un groupement -R'6-N-R'7- dans lequel R'6 et R'7 forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N;
ledit copolymère étant neutralisé au moins partiellement par au moins un agent neutralisant choisi parmi les acides organiques au sens de Bronsted.

2. Polymère selon la revendication 1 dans lequel, dans la formule (I), R1 représente l'hydrogène ou un radical méthyle, éthyle, propyle, butyle.

3. Polymère selon l'une des revendications précédentes, dans lequel, dans la formule (I), Z représente COO ou CONH.

4. Polymère selon l'une des revendications précédentes, dans lequel, dans la formule (I), le radical R2 est choisi parmi :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 25 hétéroatomes choisis parmi O, N, S, F, Si et P; ou bien un radical benzylène -C₆H₄-CH₂- éventuellement substitué, par un radical alkyle en C1-C12 comprenant éventuellement 1 à 8 hétéroatomes choisis parmi O, N, S, F, Si et P;
- un radical pyridinium de formule: 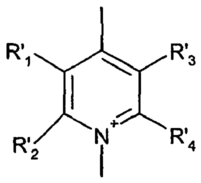 avec R'1 à R'4, identiques ou différents, choisis parmi H et un radical alkyle en C1-C12 comprenant éventuellement 1 à 8 hétéroatomes choisis parmi O, N, S, F, Si et P; notamment R'1 à R'4 peuvent être méthyle et/ou éthyle;
- un radical de formule -CH₂-O-CO-O-, CH₂-CH₂-O-CO-O-, -CH₂-CO-O-, -CH₂-CH₂-CO-O-, -CH₂-O-CO-NH-, -CH₂-CH₂-O-CO-NH-; -CH₂-NH-CO-NH-, -CH₂-CH₂-NH-CO-NH- ;-CH₂-CHOH-, -CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R")-, -CH₂-CH(NR'R")-, -CH₂-CH₂-CH₂-NR'-, -CH₂-CH₂-CH₂-O-; -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux.

5. Polymère selon l'une des revendications précédentes, dans lequel, dans la formule (I), n est compris entre 5 et 200 inclus, et encore mieux entre 7 et 100 inclus, voire entre 9 et 50 inclus.

6. Polymère selon l'une des revendications précédentes, dans lequel, dans la formule (I), R3 est un atome d'hydrogène; un radical succinimido, maléimido, mésityle, tosyle, triéthoxysilane, phtalimide ou -CH₂-CH₂CN; ou encore un radical benzyle ou phényle éventuellement substitué par un radical alkyle en C1-C12 comprenant éventuellement 1 à 8 hétéroatomes choisis parmi O, N, S, F, Si et P; un radical alkyle en C1-C30, notamment C1-C22, voire C2-C16, comprenant éventuellement 1 à 18 hétéroatomes choisis parmi O, N, S, F, Si et P ;
lesdits radicaux benzyle, phényle ou alkyle pouvant comprendre en outre une fonction choisie parmi les fonctions suivantes: Succinimido ; Glutarate-succinimido ; Glutarate ; maléimido ; Mésityle ; Benzoate ; Tosyle ; Triéthoxysilane ; Phtalimide ; Thioester ; Benzotriazole carbonate ; Butyraldéhyde ; Acétaldéhyde diéthylacétal ; Biotine ; Phospholipide ; Succinate ; N-hydroxysuccinimide ; - SO₃H, -COOH, -PO₄, -NR5R6 ou -N⁺ R5R6R7, avec R5, R6 et R7, indépendamment l'un de l'autre, choisis parmi H ou alkyles en C1-C18, linéaire, ramifié ou cyclique, notamment méthyle, comprenant éventuellement 1 ou plusieurs hétéroatomes ou encore des groupements protecteurs tels que le t-butyloxycarbonyle ou le 9-fluorenylmethoxycarbonyle.

7. Polymère selon l'une des revendications précédentes, dans lequel le monomère de formule (I) est choisi parmi, seul ou en mélange :
- le (méth)acrylate de poly(éthylène glycol) ;
- le (méth)acrylate de méthyl-poly(éthylène glycol) ;
- les (méth)acrylate d'alkyl-poly(éthylène glycol) ;
- les (méth)acrylates de phényl-poly(éthylène glycol);
- le monomère suivant : 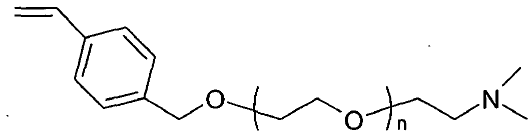 dans lequel n est de préférence compris entre 3 et 100 inclus, notamment 5 à 50 inclus, voire 7 à 30 inclus.

8. Polymère selon l'une des revendications précédentes, dans lequel le monomère de formule (I) a un poids moléculaire compris entre 350 et 13 000 g/mol., notamment entre 500 et 8000 g/mol.

9. Polymère selon l'une des revendications précédentes, dans lequel le monomère de formule (I) est choisi parmi les (méth)acrylates de poly(éthylène glycol) et les (méth)acrylates de méthyl-poly(éthylène glycol), de préférence ceux ayant un poids moléculaire compris entre 350 et 13 000 g/mol., notamment entre 500 et 8000 g/mol.

10. Polymère selon l'une des revendications précédentes, dans lequel le monomère de formule (I) est choisi parmi les (méth)acrylates de poly(éthylène glycol) et en particulier ceux ayant un poids moléculaire entre 350 et 13 000 g/mol., notamment entre 500 et 8000 g/mol.

11. Polymère selon l'une des revendications précédentes, dans lequel le monomère de formule (I), seul ou en mélange, est présent à raison de 20 à 60% en poids, par rapport au poids du polymère final, de préférence de 30 à 50% en poids.

12. Polymère selon l'une des revendications précédentes, dans lequel dans le monomère de formule (IIa), le radical R'2 est choisi parmi :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 25 hétéroatomes choisis parmi N, O, S, F, Si et/ou P; ou bien un radical benzylène -C₆H₄-CH₂-éventuellement substitué, par un radical alkyle en C1-C12 comprenant éventuellement 1 à 25 hétéroatomes choisis parmi O, N, S, F, Si et P;
- un radical de formule -CH₂-O-CO-O-, CH₂-CH₂-O-CO-O-, -CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, - CH₂-CH₂-O-CO-NH-; -CH₂-NH-CO-NH- ou -CH₂-CH₂-NH-CO-NH-, -CH₂-CHOH-, - CH₂-CH₂-CHOH-, -CH₂-CH₂-CH(NH₂)-, -CH₂-CH(NH₂)-, -CH₂-CH₂-CH(NHR')-, - CH₂-CH(NHR')-, -CH₂-CH₂-CH(NR'R")-, -CH₂-CH(NR'R")-, -CH₂-CH₂-CH₂-NR'-, - CH₂-CH₂-CH₂-O-; -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux;

13. Polymère selon l'une des revendications précédentes, dans lequel dans le monomère de formule (IIa), les radicaux R6 et R7 sont choisis parmi l'hydrogène, un groupement méthyle, éthyle, propyle, isopropyle, n-butyle, t-butyle, isobutyle, octyle, lauryle, stéaryle; de préférence hydrogène, méthyle et éthyle.

14. Polymère selon l'une des revendications précédentes, dans lequel dans le monomère de formule (IIa), X est un radical choisi parmi les radicaux de type pyridine, indolyle, isoindolinyle, imidazolyle, imidazolinyle, piperidinyl, pyrazolynyl, pyrazolyl, quinoline, pyrazolinyl, pyridinyl, piperazinyl, pyrrolidinyl, quinidinyl, thiazolinyl, morpholine, guanidino, amidino, et leurs mélanges.

15. Polymère selon l'une des revendications précédentes, dans lequel le monomère de formule (IIa) est choisi parmi, seul ou en mélange :
le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle, et les monomères ci-après :

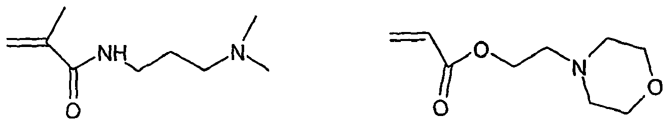

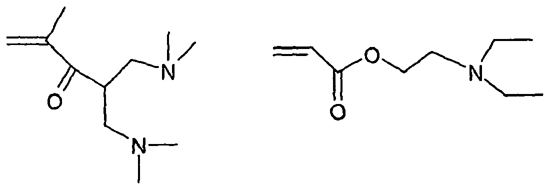

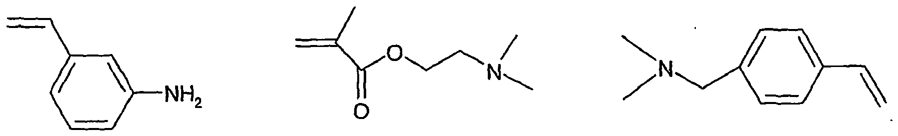

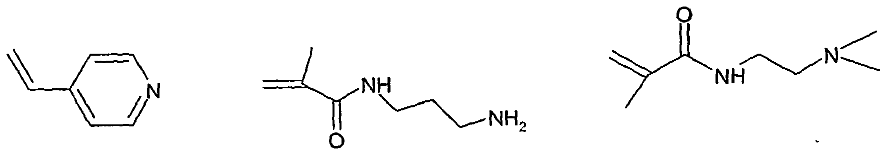

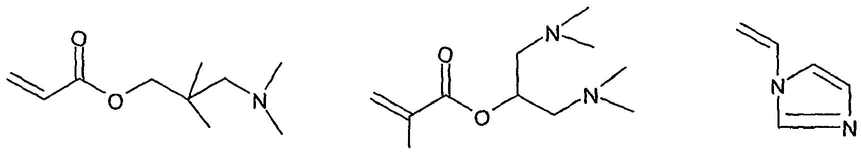

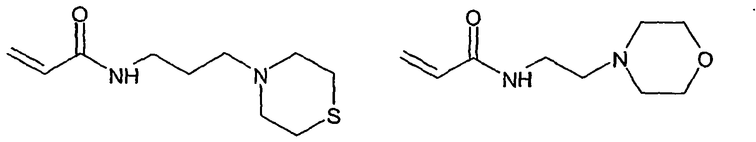

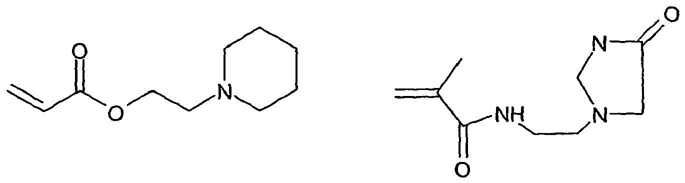

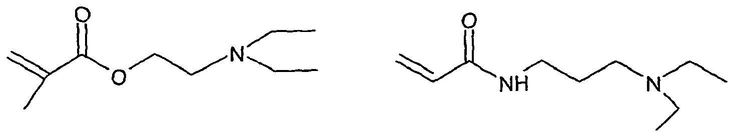

16. Polymère selon l'une des revendications précédentes, dans lequel le monomère cationique de formule (IIa) est présent à raison de 40 à 80% en poids par rapport au poids du polymère final, de préférence de 50 à 70% en poids.

17. Polymère selon l'une des revendications précédentes, comprenant un ou plusieurs monomères ioniques additionnels, choisis parmi les monomères amphotères de formules (IIc) et (IId), et/ou les monomères anioniques choisis parmi l'anhydride maléique et/ou ceux de formule (IIb) : 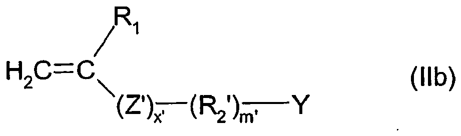 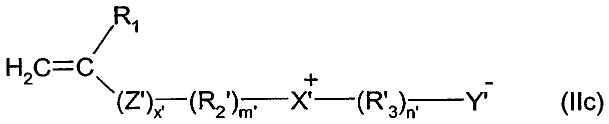 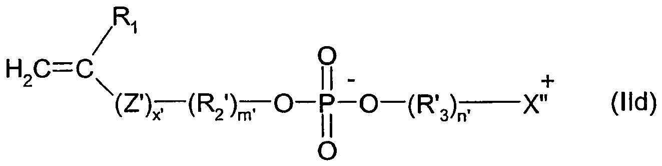 dans lesquelles :
- R1, Z', x', R'2 et m' ont la même signification que celle donnée dans la revendication 1, pour la formule (IIa);
- Y est un groupement choisi parmi -COOH, -SO₃H, -OSO₃H, -PO₃H₂ et -OPO₃H₂.
- X'⁺ est un groupe divalent de formule -N⁺(R₆)(R₇)- avec R6 et R7 représentant, indépendamment l'un de l'autre, soit (i) un atome d'hydrogène, soit (ii) un groupement alkyle linéaire, ramifié ou cyclique, éventuellement aromatique, comprenant de 1 à 25 atomes de carbone, pouvant comprendre 1 à 20 hétéroatomes choisis parmi O, N, S et P; soit (iii) R6 et R7 peuvent former avec l'atome d'azote un premier cycle, saturé ou insaturé, éventuellement aromatique, comprenant au total 5, 6, 7 ou 8 atomes, et notamment 4, 5, 6 ou 7 atomes de carbone et/ou 2 à 3 hétéroatomes choisi parmi O, S et N; ledit premier cycle pouvant être fusionné avec un ou plusieurs autres cycles, saturés ou insaturés, éventuellement aromatiques, comprenant chacun 5, 6, 7 ou 8 atomes, et notamment 4, 5, 6 ou 7 atomes de carbone et/ou 2 à 3 hétéroatomes choisi parmi O, S et N.
- Y'⁻ est un groupement choisi parmi -COO⁻, -SO₃^{-,} -OSO₃⁻, -PO₃²⁻ et -OPO₃²⁻.
- R'3 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P.
- n' est compris entre 1 et 100, de préférence 1 et 5 inclus.
- X"⁺ est un groupement de formule -N⁺R₆R₇R₈ avec R6, R7 et R8 représentant, indépendamment l'un de l'autre, soit (i) un atome d'hydrogène, soit (ii) un groupement alkyle linéaire, ramifié ou cyclique, éventuellement aromatique, comprenant de 1 à 18 atomes de carbone, pouvant comprendre 1 à 5 hétéroatomes choisis parmi O, N, S et P; soit (iii) R6 et R7 peuvent former avec l'atome d'azote un premier cycle, saturé ou insaturé, éventuellement aromatique, comprenant au total 5, 6 ou 7 atomes, et notamment 4, 5 ou 6 atomes de carbone et/ou 2 à 3 hétéroatomes choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 3 hétéroatomes choisi parmi O, S et N; notamment les radicaux triméthylammonium; triéthylammonium; N,N-diméthyl,N-octylammonium; N,N-diméthyl,N-laurylam-monium.

18. Polymère selon la revendication 17, dans lequel dans le monomère de formule (IIc) et/ou (IId), le radical R'3 est choisi parmi :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 5 hétéroatomes choisis parmi O, N, S, F, Si et P; ou bien un radical benzylène -C₆H₄-CH₂- éventuellement substitué, par un radical alkyle en C1-C12 comprenant éventuellement 1 à 5 hétéroatomes choisis parmi O, N, S, F, Si et P;
- un radical de formule -CH₂-O-CO-O-, CH₂-CH₂-O-CO-O-, -CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, - CH₂-CH₂-O-CO-NH-; -CH₂-NH-CO-NH- ou -CH₂-CH₂-NH-CO-NH-, -CH₂-CHOH-, - CH₂-CH₂-CHOH-, -CH₂-CH₂-CH(NH₂)-, -CH₂-CH(NH₂)-, -CH₂-CH₂-CH(NHR')-, - CH₂-CH(NHR')-, -CH₂-CH₂-CH(NR'R")-, -CH₂-CH(NR'R")-, -CH₂-CH₂-CH₂-NR'-, - CH₂-CH₂-CH₂-O-; -[CH₂-CH₂-O]ₙ- et -[CH₂-CH(CH₃)-O]ₙ-, -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux;

19. Polymère selon l'une des revendications 17 à 18, dans lequel les monomères anioniques sont choisis parmi l'anhydride maléique, l'acide acrylique, l'acide méthacrylique, l'acide crotonique, l'acide itaconique, l'acide fumarique, l'acide maléique, l'acrylate de 2-carboxyéthyle (CH2=CH-C(O)-O-(CH₂)₂-COOH); l'acide styrènesulfonique, l'acide 2-acrylamido 2-methylpropanesulfonique, l'acide vinylbenzoïque, l'acide vinylphosphorique, le (méth)acrylate de sulfopropyle, et les sels de ceux-ci.

20. Polymère selon l'une des revendications 17 à 19, dans lequel les monomères de formules (IIc) ou (IId) sont choisis parmi la N,N-dimethyl-N-(2-methacryloyloxyethyl)-N-(3-sulfopropyl) ammonium bétaïne, la N,N-dimethyl-N-(3-methacrylamidopropyl)-N-(3-sulfopropyl) ammonium bétaïne, la 1-(3-sulfopropyl)-2-vinylpyridinium bétaïne et la 2-méthacryloyloxyéthylphosphorylcholine.

21. Polymère selon l'une des revendications 17 à 20, dans lequel lesdits monomères ioniques additionnels sont présents à raison de 5 à 40% en poids, par rapport au poids du mélange "cationiques + ioniques additionnels", notamment de 10 à 30% en poids, de préférence de 15 à 25% en poids.

22. Polymère selon l'une des revendications 17 à 21, dans lequel lesdits monomères ioniques sont présents à raison de 20 à 90% en poids par rapport au poids du polymère final, notamment de 40 à 80% en poids, de préférence de 50 à 70% en poids.

23. Polymère selon l'une des revendications précédentes, comprenant en outre au moins un monomère hydrophile non ionique additionnel choisi parmi ceux de formule (III), seuls ou en mélange, : 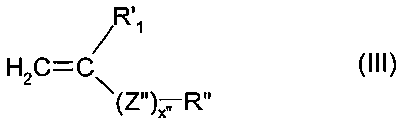 dans laquelle :
- R'₁ est l'hydrogène ou -CH₃;
- Z" est un groupement divalent choisi parmi -COO-, -CONH-, -CONCH₃-, -OCO-, - SO₂, -CO-O-CO-, -CO-CH₂-CO- ou -O- ; de préférence COO et CONH;
- x" est 0 ou 1;
- R" est un radical carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P; notamment méthyle, éthyle, propyle, isopropyle, n-butyle, isobutyle, tertiobutyle, phényle, benzyle, ou un radical de formule -CH₂-CH₂-CH₂OH, -CH₂-CH₂-OH, -CH₂-CH₂-CH₂OH ou furfuryle.

24. Polymère selon la revendication 23, dans lequel le monomère hydrophile non ionique additionnel est choisi parmi : le méthacrylate de méthyle, l'acrylate de méthyle, le méthacrylate d'éthyle, l'acrylate d'éthyle, l'acrylate de propyle, l'acrylate d'isopropyle, le méthacrylate de tetrahydrofurfuryle, l'acrylate de tetrahydrofurfuryle, le méthacrylate de 2-hydroxyéthyle, l'acrylate de 2-hydroxyéthyle, le méthacrylate d'éthoxyéthyle, l'acrylate d'éthoxyéthyle, le N-isopropyl acrylamide, le N-isopropyl méthacrylamide, le N,N-diméthyl acrylamide, le N,N-diméthyl méthacrylamide, l'acétate de vinyle, le méthyl vinyl éther, l'éthyl vinyl éther, la vinylpyrrolidone, la vinylcaprolactame, la N-vinyl acétamide et l'acrylate d'hydroxypropyle ; la N-vinyllactame, l'acrylamide, la N-méthyl acrylamide, la N,N-diméthyl acrylamide, la N-méthyl-N-vinylacétamide, le N-vinylformamide, le N-méthyl-N-vinylformamide, l'alcool vinylique (copolymérisé sous forme d'acétate de vinyle puis hydrolysé).

25. Polymère selon l'une des revendications 23 à 24, dans lequel le monomère hydrophile non ionique additionnel, seul ou en mélange, est présent en une quantité de 0,1 à 35% en poids, de préférence de 1 à 25% en poids, par exemple de 3 à 15% en poids, voire de 5 à 9,5% en poids, par rapport au poids total du polymère.

26. Polymère selon l'une des revendications précédentes, dans lequel l'agent neutralisant est choisi parmi les acides aliphatiques linéaires, ramifiés ou cycliques et/ou les acides insaturés ou aromatiques, et peuvent notamment comprendre 1 à 1000 atomes de carbone, notamment 2 à 500 atomes de carbone; possèdant au moins une fonction acide au sens de Bronsted, et notamment un ou plusieurs groupes acide carboxylique, sulfonique et/ou phosphonique; et pouvant comporter, en outre, un ou plusieurs hétéroatomes choisis parmi O, N, Si, F et P, par exemple sous la forme de groupes hydroxyle.

27. Polymère selon l'une des revendications précédentes, dans lequel l'agent neutralisant est choisi parmi, seul ou en mélange, :
- les acides gras ayant 6 à 32 atomes de carbone, notamment 8 à 28, linéaires, ramifiés ou cycliques, saturés ou insaturés, éventuellement aromatiques, et comportant au moins une fonction COOH ou acide sulfonique (-SO₃H);
- les hydroxyacides, notamment des alphahydroxy-acides, ayant 6 à 32 atomes de carbone, notamment 8 à 28, linéaires, ramifiés ou cycliques, saturés ou insaturés, éventuellement aromatiques, et comportant au moins une fonction COOH ou acide sulfonique (-SO₃H);.
- les acides alkylbenzène-sulfoniques, dans lesquels le groupement alkyle peut comprendre de 4 à 30, notamment 6 à 24, atomes de carbone;
- les agents neutralisants amphotères, notamment du type alkyl-bétaïnes ou alkylamidopropylbétaïnes, dans lesquels le groupement alkyle peut comprendre 4 à 30, notamment 6 à 24, atomes de carbone; en particulier la cocoamidopropylbétaïne.

28. Polymère selon l'une des revendications précédentes, dans lequel l'agent neutralisant est choisi parmi l'acide alpha-hydroxyéthanoïque, l'acide alphahydroxyoctanoïque, l'acide alpha-hydroxycaprylique, l'acide ascorbique, l'acide acétique, l'acide benzoïque, l'acide béhénique, l'acide caprique, l'acide citrique, l'acide caproïque, l'acide caprylique, l'acide dodécylbenzène sulfonique, l'acide 2-éthylcaproïque, l'acide folique, l'acide fumarique, l'acide galactarique, l'acide gluconique, l'acide glycolique, l'acide 2-hexadécyl eicosanoïque, l'acide hydroxycaproique, l'acide 12-hydroxystéarique, l'acide isolaurique (ou 2-butyl octanoïque), l'acide isomyristique (ou 2-hexyl octanoïque), l'acide isoarachidique (ou 2-octyl dodécanoïque), l'acide isolignocérique (ou 2-décyl tétradécanoïque), l'acide lactique, l'acide laurique, l'acide malique, l'acide myristique, l'acide oléïque, l'acide palmitique, l'acide propionique, l'acide sébacique, l'acide stéarique, l'acide tartrique, l'acide téréphtalique, l'acide trimésique, l'acide undécylénique, la propylebétaïne, la cocoamidopropylbétaïne, et le chlorhydrate de bétaïne de formule [(CH₃)₃N+CH₂CO₂H.Cl-], ainsi que leurs mélanges.

29. Polymère selon l'une des revendications précédentes, dans lequel l'agent neutralisant est choisi parmi l'acide caproïque, l'acide 2-éthylcaproïque, l'acide oléïque, l'acide béhénique, l'acide stéarique, l'acide acétique, l'acide citrique, l'acide tartrique, le chlorhydrate de bétaïne et/ou l'acide gluconique, et préférentiellement le chlorhydrate de bétaïne et/ou l'acide béhénique.

30. Polymère selon l'une des revendications précédentes, dans lequel l'agent neutralisant, seul ou en mélange, est ajouté en une quantité de 0,01 à 3 équivalent molaire, notamment 0,05 à 2,5, voire 0,1 à 2 équivalent molaire, par rapport aux fonctions amines totales du polymère ou des monomères.

31. Polymère selon la revendication 30, dans lequel l'agent neutralisant, seul ou en mélange, est ajouté en une quantité de 0,01 à 0,99 équivalent molaire, notamment 0,05 à 0,9, voire 0,1 à 0,8 équivalent molaire, par rapport aux fonctions amines totales du polymère ou des monomères.

32. Polymère selon la revendication 30, dans lequel l'agent neutralisant, seul ou en mélange, est ajouté en une quantité de 1,01 à 3 équivalent molaire, notamment 1,2 à 2,5, voire 1,5 à 2 équivalent molaire, par rapport aux fonctions amines totales du polymère ou des monomères.

33. Polymère selon la revendication 30, dans lequel l'agent neutralisant, seul ou en mélange, est ajouté en une quantité de 1 équivalent molaire, par rapport aux fonctions amines totales du polymère ou des monomères.

34. Polymère selon l'une des revendications précédentes, présentant une masse moléculaire moyenne en poids (Mw) comprise entre 500 et 5 000 000, notamment comprise entre 1 000 et 3 000 000 et plus préférentiellement comprise entre 2 000 et 2 000 000, voire 4 000 et 500 000, entre autre 7 000 et 400 000, encore mieux 20 000 et 350 000, et encore 150 000 et 300 000.

35. Polymère selon l'une des revendications précédentes, **caractérisé par le fait qu'**il est véhiculable en milieu aqueux, de préférence hydrosoluble ou hydrodispersible.

36. Polymère selon l'une des revendications précédentes, **caractérisé par le fait qu'**il présente une viscosité dans l'eau comprise entre 1 et 1000 mPa.s, de préférence entre 1,5 et 750 mPa.s, et encore mieux entre 2 et 500 mPa.s, mesurée à 25°C, à l'aide d'un viscosimètre BROOKFIELD, pour une solution à 15% en poids de polymère dans l'eau ou la méthyléthylcétone, à 25°C, avec un mobile de type aiguille (spindle) no 1 ; pour un temps de mesure de 5 minutes, à une vitesse comprise entre 0,1 et 6 tours/minute.

37. Polymère selon l'une des revendications précédentes, **caractérisé par le fait qu'**il présente une température de transition vitreuse (Tg) comprise entre -150°C et 20°C, notamment -120°C et 10°C, mieux entre -100°C et 0°C.

38. Polymère selon l'une des revendications précédentes, **caractérisé par le fait qu'**il présente une reprise en eau comprise entre 3% et 150% en poids, de préférence entre 4% et 100% en poids, notamment entre 5% et 50% en poids, à 75% d'humidité relative (75%HR) ; et/ou qu'il présente une reprise en eau comprise entre 3% et 200% en poids, de préférence entre 55% et 150% en poids, notamment entre 60% et 120% en poids, voire entre 70 et 100% en poids, à 85% d'humidité relative (85%HR).

39. Polymère selon l'une des revendications précédentes, dans lequel :
- le monomère de formule (I), seul ou en mélange, est présent à raison de 10 à 80% en poids, par rapport au poids du polymère final, notamment de 20 à 60% en poids, de préférence de 30 à 50% en poids, et est choisi parmi, seul ou en mélange, les (méth)acrylates de poly(éthylène glycol) de préférence ceux ayant un poids moléculaire compris entre 350 et 13 000 g/mol., notamment entre 500 et 8000 g/mol.; et
- le monomère 'essentiellement cationique' est présent à raison de 20 à 90% en poids par rapport au poids du polymère final, notamment de 40 à 80% en poids, de préférence de 50 à 70% en poids et est choisi parmi, seul ou en mélange, le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle; et
- le polymère est neutralisé par un agent neutralisant choisi parmi l'acide 2-éthylcaproïque, l'acide oléïque, l'acide béhénique, l'acide stéarique, l'acide acétique, l'acide citrique, l'acide tartrique, le chlorhydrate de bétaïne et/ou l'acide gluconique, et préférentiellement l'acide béhénique et/ou le chlorhydrate de bétaïne.

40. Polymère selon l'une des revendications précédentes, dans lequel :
- le monomère de formule (I), seul ou en mélange, est présent à raison de 10 à 80% en poids, par rapport au poids du polymère final, notamment de 20 à 60% en poids, de préférence de 30 à 50% en poids, et est choisi parmi, seul ou en mélange, les (méth)acrylates de poly(éthylène glycol) de préférence ceux ayant un poids moléculaire compris entre 350 et 13 000 g/mol., notamment entre 500 et 8000 g/mol.; et
- le monomère 'essentiellement cationique' est présent à raison de 20 à 90% en poids par rapport au poids du polymère final, notamment de 40 à 80% en poids, de préférence de 50 à 70% en poids et est choisi parmi, seul ou en mélange, le (méth)acrylamide de diméthylaminopropyle, et
- le polymère est neutralisé par un agent neutralisant choisi parmi l'acide béhénique et/ou le chlorhydrate de bétaïne.

41. Composition cosmétique ou pharmaceutique comprenant dans un milieu physiologiquement acceptable, notamment cosmétiquement ou pharmaceutiquement acceptable, au moins un polymère tel que défini à l'une des revendications 1 à 40.

42. Composition selon la revendication 41, comprenant une solution dans l'eau ou dans un solvant organique, ou bien une dispersion aqueuse ou organique du polymère selon l'une des revendications 1 à 40.

43. Composition selon l'une des revendications 41 à 42, dans laquelle le polymère est présent à raison de 0,01 à 50% en poids de matière sèche, notamment de 0,1 à 30% en poids, voire de 0,3 à 10% en poids, encore mieux de 1 à 3% en poids, par rapport au poids total de la composition.

44. Composition selon l'une des revendications 41 à 43, dans laquelle le milieu physiologiquement acceptable comprend au moins un constituant choisi parmi l'eau ; les solvants organiques hydrophiles comme les alcools, notamment les monoalcools, linéaires ou ramifiés en C1-C6 et les polyols et les éthers de glycols notamment en C₂ et des aldéhydes en C₂-C₄ hydrophiles ; les cires, les corps gras pâteux, les gommes et leurs mélanges, d'origine animale, végétale, minérale ou synthétique ; des solvants organiques lipophiles ; des huiles d'origine animale, végétale, minérale ou synthétique ; les esters et les éthers de synthèse ; les esters du pentaérythritol; des alcools gras ayant de 12 à 26 atomes de carbone ; les huiles fluorées partiellement hydrocarbonées et/ou siliconées; les huiles siliconées volatiles ou non, linéaires ou cycliques, liquides ou pâteux à température ambiante; des pigments, des nacres, des charges ; les colorants hydrosolubles, les colorants liposolubles ; les polymères ; les agents auxiliaires de filmification ; les tensioactifs ; les vitamines, les parfums, les agents nacrants, les épaississants, les gélifiants, les oligo-éléments, les adoucissants, les séquestrants, les parfums, les agents alcalinisants ou acidifiants, les conservateurs, les filtres solaires, les anti-oxydants, les agents anti-chutes des cheveux, les agents anti-pelliculaires, les agents propulseurs, les céramides ; leurs mélanges.

45. Composition selon l'une des revendications 41 à 44, se présentant sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; d'une lotion, d'une crème, d'une pommade, d'une pâte souple, d'un onguent, d'un solide coulé ou moulé et notamment en stick ou en coupelle, ou encore de solide compacté.

46. Composition selon l'une des revendications 41 à 45, se présentant sous la forme d'un produit de soin et/ou de maquillage de la peau du corps ou du visage, des lèvres et des cheveux, d'un produit solaire ou autobronzant, d'un produit capillaire.

47. Composition selon l'une des revendications 41 à 46, se présentant sous la forme d'une composition capillaire, notamment pour le maintien de la coiffure ou la mise en forme des cheveux, par exemple sous forme de shampooings, de gels, de lotions de mise en plis, de lotions pour le brushing, de compositions de fixation et de coiffage telles que les laques ou spray ; d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

48. Procédé cosmétique de traitement des matières kératiniques telles que la peau du corps ou du visage, des ongles, des poils, des cheveux et/ou des cils, **caractérisé en ce qu'**il consiste à appliquer sur les matières kératiniques une composition cosmétique telle que définie dans l'une des revendications 41 à 47, puis à effectuer éventuellement un rinçage à l'eau.

49. Procédé cosmétique de maintien de la coiffure; de traitement, soin et/ou lavage et/ou démaquillage de la peau du corps ou du visage, des ongles, des poils, des cheveux et/ou des cils, **caractérisé en ce qu'**il consiste à appliquer sur les matières kératiniques une composition cosmétique telle que définie dans l'une des revendications 41 à 47, puis à effectuer éventuellement un rinçage à l'eau.

50. Procédé de préparation d'un copolymère éthylénique tel que défini dans l'une des revendications 1 à 40, dans lequel on neutralise les motifs amines des monomères entrant dans la constitution du polymère, en particulier les motifs amines des monomères cationiques de formule (IIa), avant leur polymérisation, puis on copolymérise les monomères neutralisés afin d'obtenir le copolymère recherché.

51. Procédé de préparation d'un copolymère éthylénique tel que défini dans l'une des revendications 1 à 40, dans lequel on copolymérise les monomères non neutralisés entrant dans la constitution du copolymère, puis on neutralise ledit copolymère après sa formation.

## Claims

1. A non-crosslinked ethylenic copolymer comprising, as a weight percentage relative to the total weight of the polymer:
- a) 10-80% by weight of one or more monomers of formula (I): 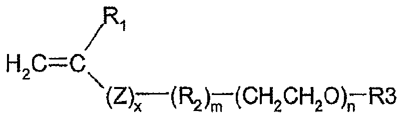 in which:
- R1 is a hydrogen atom or a linear or branched hydrocarbon-based radical, of the type CₚH₂ₚ₊₁, with p being an integer between 1 and 12 inclusive;
- Z is a divalent group chosen from -COO-, -CONH-,
- CONCH₃-, -OCO-, -O-, -SO₂-, -CO-O-CO- and -CO-CH₂-CO- ;
- x is 0 or 1;
- R2 is a saturated or unsaturated, optionally aromatic, linear, branched or cyclic carbon-based divalent radical of 1 to 30 carbon atoms, possibly comprising 1 to 18 heteroatoms chosen from O, N, S, F, Si and P;
- m is 0 or 1;
- n is an integer between 3 and 300 inclusive;
- R3 is a hydrogen atom or a saturated or unsaturated, optionally aromatic, linear, branched or cyclic carbon-based radical of 1 to 30 carbon atoms, possibly comprising 1 to 20 heteroatoms chosen from O, N, S, F, Si and P;
and salts thereof;
- b) 20-90% by weight of at least one cationic monomer, or a salt thereof, chosen from the monomers of formula (IIa) : 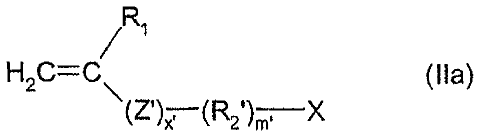 in which:
- R1 is a hydrogen atom or a linear or branched hydrocarbon-based radical of the type CₚH₂ₚ₊₁, with p being an integer between 1 and 12 inclusive; preferably hydrogen or a methyl, ethyl, propyl or butyl radical;
- Z' is a divalent group chosen from -COO-, -CONH-,
- CONCH₃-, -OCO- or -O-, -SO₂- -CO-O-CO- or -CO-CH₂-CO- ; preferably COO and CONH;
- x' is 0 or 1, preferably 1;
- R'2 is a saturated or unsaturated, optionally aromatic, linear, branched or cyclic divalent carbon-based radical of 1 to 30 carbon atoms, possibly comprising 1 to 18 heteroatoms chosen from O, N, S, F, Si and P ;
- m' is 0 or 1 ;
- X is
(a) a guanidino or amidino group, or
(b) a group of formula -N(R₆)(R₇) or -P(R₆)(R₇) or -P⁺R₆R₇R₈, with R6, R7 and R8 representing, independently of each other, either (i) a hydrogen atom, or (ii) a linear, branched or cyclic, saturated or unsaturated, optionally aromatic alkyl group containing from 1 to 18 carbon atoms, possibly comprising 1 to 10 heteroatoms chosen from O, N, S, F, Si and P; or (iii) R6 and R7 may form with the nitrogen or phosphorus atom a first saturated or unsaturated, optionally aromatic ring comprising in total 5, 6, 7 or 8 atoms, and especially 4, 5 or 6 carbon atoms and/or 2 to 4 heteroatoms chosen from O, S and N; said first ring possibly being fused with one or more other saturated or unsaturated, optionally aromatic rings each comprising 5, 6 or 7 atoms, and especially 4, 5, 6 or 7 carbon atoms and/or 2 to 4 heteroatoms chosen from O, S and N; or
(c) a group -R'6-N-R'7- in which R'6 and R'7 form with the nitrogen atom a saturated or unsaturated, optionally aromatic ring comprising in total 5, 6, 7 or 8 atoms, and especially 4, 5 or 6 carbon atoms and/or 2 to 4 heteroatoms chosen from O, S and N; said ring possibly being fused with one or more other saturated or unsaturated, optionally aromatic rings, each comprising 5, 6 or 7 atoms, and especially 4, 5, 6, 7 or 8 carbon atoms and/or 2 to 4 heteroatoms chosen from O, S and N;
said copolymer being at least partially neutralized with at least one neutralizer chosen from organic acids in the Brönsted sense.

2. The polymer as claimed in claim 1, in which, in formula (I), R1 represents hydrogen or a methyl, ethyl, propyl or butyl radical.

3. The polymer as claimed in either of the preceding claims, in which, in formula (I), Z represents COO or CONH.

4. The polymer as claimed in one of the preceding claims, in which, in formula (I), the radical R2 is chosen from:
- an alkylene radical such as methylene, ethylene, propylene, n-butylene, isobutylene, tert-butylene, n-hexylene, n-octylene, n-dodecylene, n-octadecylene, n-tetradecylene or n-docosanylene;
- a phenylene radical -C₆H₄- (ortho, meta or para), optionally substituted with a C1-C12 alkyl radical optionally comprising 1 to 25 heteroatoms chosen from O, N, S, F, Si and P; or alternatively a benzylene radical -C₆H₄-CH₂-, optionally substituted with a C1-C12 alkyl radical optionally comprising 1 to 8 heteroatoms chosen from O, N, S, F, Si and P;
- a pyridinium radical of formula: 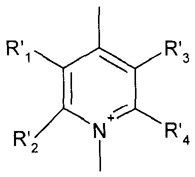 with R'1 to R'4, which may be identical or different, chosen from H and a C1-C12 alkyl radical optionally comprising 1 to 8 heteroatoms chosen from O, N, S, F, Si and P; R'1 to R'4 may especially be methyl and/or ethyl;
- a radical of formula -CH₂-O-CO-O-, CH₂-CH₂-O-CO-O-,
- CH₂-CO-O-, -CH₂-CH₂-CO-O-, -CH₂-O-CO-NH-, -CH₂-CH₂-O-CO-NH- ; -CH₂-NH-CO-NH-, -CH₂-CH₂-NH-CO-NH-; -CH₂-CHOH-, - CH₂-CH₂-CHOH-, CH₂-CH₂-CH(NH₂)-, -CH₂-CH(NH₂) -, -CH₂-CH₂-CH(NHR')-, -CH₂-CH(NHR')-, -CH₂-CH₂-CH(NR'R")-, -CH₂-CH (NR'R") -, -CH₂-CH₂-CH₂-NR'-, -CH₂-CH₂-CH₂-O-; -CH₂-CH₂-CHR'-O- with R' and R" representing a linear or branched C1-C22 alkyl optionally comprising 1 to 12 heteroatoms chosen from O, N, S, F, Si and P;
- or a mixture of these radicals.

5. The polymer as claimed in one of the preceding claims, in which, in formula (I), n is between 5 and 200 inclusive and better still between 7 and 100 inclusive, or even between 9 and 50 inclusive.

6. The polymer as claimed in one of the preceding claims, in which, in formula (I), R3 is a hydrogen atom; a succinimido, maleimido, mesityl, tosyl, triethoxysilane, phthalimide or -CH₂-CH₂CN radical; or alternatively a benzyl or phenyl radical optionally substituted with a C1-C12 alkyl radical optionally comprising 1 to 8 heteroatoms chosen from O, N, S, F, Si and P; a C1-C30 and especially C1-C22 or even C2-C16 alkyl radical, optionally comprising 1 to 18 heteroatoms chosen from O, N, S, F, Si and P;
said benzyl, phenyl or alkyl radicals also possibly comprising a function chosen from the following functions: succinimido; glutarate-succinimido; glutarate; maleimido; mesityl, benzoate; tosyl; triethoxysilane; phthalimide; thioester; benzotriazole carbonate; butyraldehyde; acetaldehyde diethyl acetal; biotin; phospholipid; succinate; N-hydroxysuccinimide; -SO₃H, -COOH, -PO₄, -NR5R6 or -N⁺R5R6R7, with R5, R6 and R7, independently of each other, chosen from H and linear, branched or cyclic C1-C18 alkyls, especially methyl, optionally comprising one or more heteroatoms or alternatively protecting groups such as t-butyloxycarbonyl or 9-fluorenylmethoxycarbonyl.

7. The polymer as claimed in one of the preceding claims, in which the monomer of formula (I) is chosen, alone or as a mixture, from:
- poly(ethylene glycol) (meth)acrylate;
- methylpoly(ethylene glycol) (meth)acrylate;
- alkylpoly(ethylene glycol) (meth)acrylates;
- phenylpoly(ethylene glycol) (meth)acrylates;
- the following monomer: 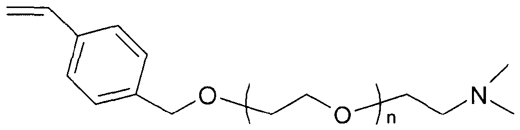 in which n is preferably between 3 and 100 inclusive and especially 5 to 50 inclusive, or even 7 to 30 inclusive.

8. The polymer as claimed in one of the preceding claims, in which the monomer of formula (I) has a molecular weight of between 350 and 13 000 g/mol and especially between 500 and 8000 g/mol.

9. The polymer as claimed in one of the preceding claims, in which the monomer of formula (I) is chosen from poly(ethylene glycol) (meth)acrylates and methylpoly(ethylene glycol) (meth)acrylates, preferably those with a molecular weight of between 350 and 13 000 g/mol and especially between 500 and 8000 g/mol.

10. The polymer as claimed in one of the preceding claims, in which the monomer of formula (I) is chosen from poly(ethylene glycol) (meth)acrylates and in particular those with a molecular weight of between 350 and 13 000 g/mol and especially between 500 and 8000 g/mol.

11. The polymer as claimed in one of the preceding claims, in which the monomer of formula (I), alone or as a mixture, is present in a proportion of from 20% to 60% by weight and preferably from 30% to 50% by weight relative to the weight of the final polymer.

12. The polymer as claimed in one of the preceding claims, in which, in the monomer of formula (IIa), the radical R'2 is chosen from:
- an alkylene radical such as methylene, ethylene, propylene, n-butylene, isobutylene, tert-butylene, n-hexylene, n-octylene, n-dodecylene, n-octadecylene, n-tetradecylene or n-docosanylene;
- a phenylene radical -C₆H₄- (ortho, meta or para), optionally substituted with a C1-C12 alkyl radical optionally comprising 1 to 25 heteroatoms chosen from N, O, S, F, Si and/or P; or alternatively a benzylene radical -C₆H₄-CH₂-, optionally substituted with a C1-C12 alkyl radical optionally comprising 1 to 25 heteroatoms chosen from O, N, S, F, Si and P;
- a radical of formula -CH₂-O-CO-O-, CH₂-CH₂-O-CO-O-, -CH₂-CO-O-, -CH₂-CH₂-CO-O-, -[(CH₂)₅-CO-O]ₙ-, -CH₂-CH(CH₃)-O-, (CH₂)₂-O-, -CH₂-O-CO-NH-, -CH₂-CH₂-O-CO-NH-; -CH₂-NH-CO-NH- or -CH₂-CH₂-NH-CO-NH-, -CH₂-CHOH-, -CH₂-CH₂-CHOH-, -CH₂-CH₂-CH (NH₂) -, -CH₂-CH (NH₂) -, -CH₂-CH₂-CH(NHR')-, -CH₂-CH(NHR')-, -CH₂-CH₂-CH(NR'R")-, -CH₂-CH(NR'R")-, -CH₂-CH₂-CH₂-NR'-, -CH₂-CH₂-CH₂-O- ; -CH₂-CH₂-CHR'-O- with R' and R" representing a linear or branched C1-C22 alkyl optionally comprising 1 to 12 heteroatoms chosen from O, N, S, F, Si and P;
- or a mixture of these radicals.

13. The polymer as claimed in one of the preceding claims, in which, in the monomer of formula (IIa), the radicals R6 and R7 are chosen from hydrogen and a methyl, ethyl, propyl, isopropyl, n-butyl, t-butyl, isobutyl, octyl, lauryl or stearyl group; preferably hydrogen, methyl or ethyl.

14. The polymer as claimed in one of the preceding claims, in which, in the monomer of formula (IIa), X is a radical chosen from radicals of pyridine, indolyl, isoindolinyl, imidazolyl, imidazolinyl, piperidyl, pyrazolinyl, pyrazolyl, quinoline, pyrazolinyl, pyridyl, piperazinyl, pyrrolidinyl, quinidinyl, thiazolinyl, morpholine, guanidino or amidino type, and mixtures thereof.

15. The polymer as claimed in one of the preceding claims, in which the monomer of formula (IIa) is chosen, alone or as a mixture, from:
dimethylaminopropyl(meth)acrylamide, dimethylaminoethyl(meth)acrylamide, diethylaminoethyl (meth)acrylate, dimethylaminoethyl (meth)acrylate, vinylimidazole, vinylpyridine and morpholinoethyl (meth) acrylate, and the monomers below:

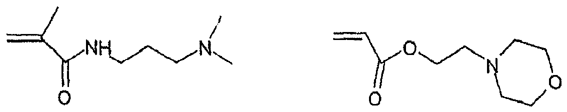

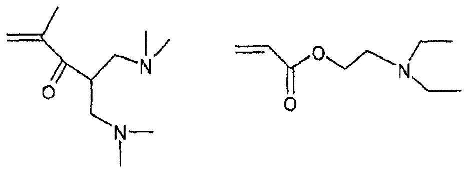

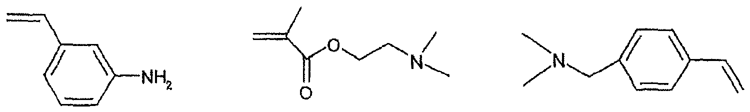

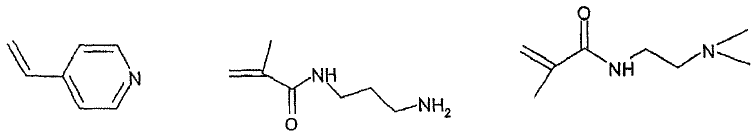

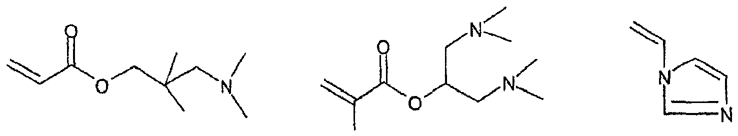

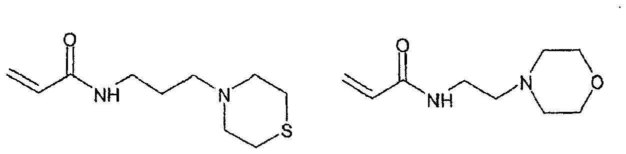

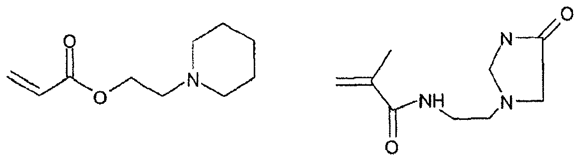

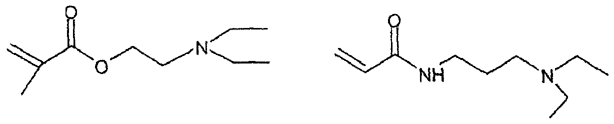

16. The polymer as claimed in one of the preceding claims, in which the cationic monomer of formula (IIa) is present in a proportion of from 40% to 80% by weight and preferably from 50% to 70% by weight relative to the weight of the final polymer.

17. The polymer as claimed in one of the preceding claims, comprising one or more additional ionic monomers chosen from the amphoteric monomers of formulae (IIc) and (IId) and/or anionic monomers chosen from maleic anhydride and/or those of formula (IIb) : 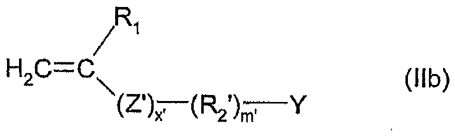 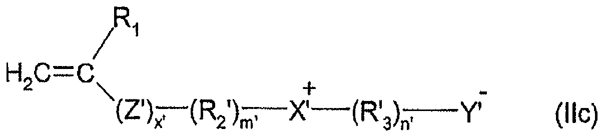 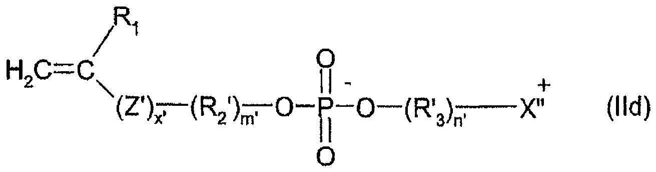 in which:
- R1, Z', x', R'2 and m' have the same meaning as that given in claim L, for formula (IIa);
- Y is a group chosen from -COOH, -SO₃H, -OSO₃H, -PO₃H₂ and -OPO₃H₂;
- X'⁺ is a divalent group of formula -N⁺(R₆) (R₇)- with R6 and R7 representing, independently of each other, either (i) a hydrogen atom, or (ii) a linear, branched or cyclic, optionally aromatic alkyl group containing from 1 to 25 carbon atoms, possibly comprising 1 to 20 heteroatoms chosen from O, N, S and P; or (iii) R6 and R7 may form with the nitrogen atom a first saturated or unsaturated, optionally aromatic ring comprising in total 5, 6, 7 or 8 atoms, and especially 4, 5, 6 or 7 carbon atoms and/or 2 to 3 heteroatoms chosen from O, S and N; said first ring possibly being fused with one or more other saturated or unsaturated, optionally aromatic rings, each comprising 5, 6, 7 or 8 atoms, and especially 4, 5, 6 or 7 carbon atoms and/or 2 to 3 heteroatoms chosen from O, S and N;
- Y'⁻ is a group chosen from -COO⁻, -SO₃⁻, -OSO₃⁻, -PO₃²⁻ and -OPO₃²⁻;
- R'3 is a saturated or unsaturated, optionally aromatic, linear, branched or cyclic divalent carbon-based radical of 1 to 30 carbon atoms, possibly comprising 1 to 18 heteroatoms chosen from O, N, S, F, Si and P;
- n' is between 1 and 100 and preferably between 1 and 5 inclusive.
- X"⁺ is a group of formula -N⁺R₆R₇R₈ with R6, R7 and R8 representing, independently of each other, either (i) a hydrogen atom, or (ii) a linear, branched or cyclic, optionally aromatic alkyl group containing from 1 to 18 carbon atoms, possibly comprising 1 to 5 heteroatoms chosen from O, N, S and P; or (iii) R6 and R7 may form with the nitrogen atom a first saturated or unsaturated, optionally aromatic ring comprising in total 5, 6 or 7 atoms, and especially 4, 5 or 6 carbon atoms and/or 2 to 3 heteroatoms chosen from O, S and N; said first ring possibly being fused with one or more other saturated or unsaturated, optionally aromatic rings, each comprising 5, 6 or 7 atoms, and especially 4, 5, 6 or 7 carbon atoms and/or 2 to 3 heteroatoms chosen from O, S and N; especially trimethylammonium; triethylammonium; N,N-dimethyl-N-octylammonium; N,N-dimethyl-N-laurylammonium radicals.

18. The polymer as claimed in claim 17, in which, in the monomer of formula (IIc) and/or (IId), the radical R'3 is chosen from:
- an alkylene radical such as methylene, ethylene, propylene, n-butylene, isobutylene, tert-butylene, n-hexylene, n-octylene, n-dodecylene, n-octadecylene, n-tetradecylene or n-docosanylene;
- a phenylene radical -C₆H₄- (ortho, meta or para), optionally substituted with a C1-C12 alkyl radical optionally comprising 1 to 5 heteroatoms chosen from O, N, S, F, Si and P; or alternatively a benzylene radical
- C₆H₄-CH₂-, optionally substituted with a C1-C12 alkyl radical optionally comprising 1 to 5 heteroatoms chosen from O, N, S, F, Si and P;
- a radical of formula -CH₂-O-CO-O-, CH₂-CH₂-O-CO-O-,
- CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, -CH₂-CH₂-O-CO-NH- ;
- CH₂-NH-CO-NH- or -CH₂-CH₂-NH-CO-NH-, -CH₂-CHOH-, -CH₂-CH₂-CHOH-, -CH₂-CH₂-CH (NH₂) -, -CH₂-CH (NH₂) -, -CH₂-CH₂-CH(NHR')-, -CH₂-CH (NHR') -, -CH₂-CH₂-CH (NR'R") -, -CH₂-CH(NR'R")-, -CH₂-CH₂-CH₂-NR'-, -CH₂-CH₂-CH₂-O- ; -[CH₂-CH₂-O]ₙ- and - [CH₂-CH(CH₃)-O]ₙ-, -CH₂-CH₂-CHR'-O- with R' and R" representing a linear or branched C1-C22 alkyl optionally comprising 1 to 12 heteroatoms chosen from O, N, S, F, Si and P ;
- or a mixture of these radicals.

19. The polymer as claimed in either of claims 17 and 18, in which the anionic monomers are chosen from maleic anhydride, acrylic acid, methacrylic acid, crotonic acid, itaconic acid, fumaric acid, maleic acid, 2-carboxyethyl acrylate (CH₂=CH-C(O)-O-(CH₂)₂-COOH) ; styrenesulfonic acid, 2-acrylamido-2-methylpropanesulfonic acid, vinylbenzoic acid, vinylphosphoric acid and sulfopropyl (meth)acrylate, and salts thereof.

20. The polymer as claimed in one of claims 17 to 19, in which the monomers of formula (IIc) or (IId) are chosen from N,N-dimethyl-N-(2-methacryloyloxyethyl)-N-(3-sulfopropyl)ammonium betaine, N,N-dimethyl-N-(3-methacrylamidopropyl)-N-(3-sulfopropyl)ammonium betaine, 1-(3-sulfopropyl)-2-vinylpyridinium betaine and 2-methacryloyloxyethylphosphorylcholine.

21. The polymer as claimed in one of claims 17 to 20, in which said additional ionic monomers are present in a proportion of from 5% to 40% by weight, especially from 10% to 30% by weight and preferably from 15% to 25% by weight relative to the weight of the "additional cationic + ionic monomers" mixture.

22. The polymer as claimed in one of claims 17 to 21, in which said ionic monomers are present in a proportion of from 20% to 90% by weight, especially from 40% to 80% by weight and preferably from 50% to 70% by weight relative to the weight of the final polymer.

23. The polymer as claimed in one of the preceding claims, also comprising at least one additional nonionic hydrcphilic monomer chosen from those of formula (III), alone or as a mixture: 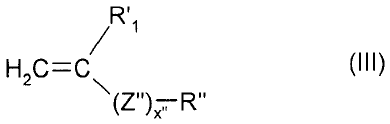 in which:
- R'₁ is hydrogen or -CH₃ ;
- Z" is a divalent group chosen from -COO-, -CONH-, -CONCH₃-, -OCO-, -SO₂ , -CO-O-CO-, -CO-CH₂-CO- and -O- ; preferably COO and CONH;
- x" is 0 or 1;
- R" is a saturated or unsaturated, optionally aromatic, linear, branched or cyclic carbon-based radical of 1 to 30 carbon atoms, possibly comprising 1 to 18 heteroatoms chosen from O, N, S, F, Si and P; especially methyl, ethyl, propyl, isopropyl, n-butyl, isobutyl, tert-butyl, phenyl, benzyl or a radical of formula -CH₂-CH₂-CH₂OH, -CH₂-CH₂-OH, -CH₂-CH₂-CH₂OH or furfuryl.

24. The polymer as claimed in claim 23, in which the additional nonionic hydrophilic monomer is chosen from:
methyl methacrylate, methyl acrylate, ethyl methacrylate, ethyl acrylate, propyl acrylate, isopropyl acrylate, tetrahydrofurfuryl methacrylate, tetrahydrofurfuryl acrylate, 2-hydroxyethyl methacrylate, 2-hydroxyethyl acrylate, ethoxyethyl methacrylate, ethoxyethyl acrylate, N-isopropylacrylamide, N-isopropylmethacrylamide, N,N-dimethylacrylamide, N,N-dimethylmethacrylamide, vinyl acetate, methyl vinyl ether, ethyl vinyl ether, vinylpyrrolidone, vinylcaprolactam, N-vinylacetamide and hydroxypropyl acrylate; N-vinyllactam, acrylamide, N-methylacrylamide, N,N-dimethylacrylamide, N-methyl-N-vinylacetamide, N-vinylformamide, N-methyl-N-vinylformamide, vinyl alcohol (copolymerized in the form of vinyl acetate and then hydrolyzed).

25. The polymer as claimed in either of claims 23 and 24, in which the additional nonionic hydrophilic monomer, alone or as a mixture, is present in an amount of from 0.1% to 35% by weight, preferably from 1% to 25% by weight, for example from 3% to 15% by weight, or even from 5% to 9.5% by weight, relative to the total weight of the polymer.

26. The polymer as claimed in one of the preceding claims, in which the neutralizer is chosen from linear, branched or cyclic aliphatic acids and/or unsaturated or aromatic acids, and may especially contain 1 to 1000 carbon atoms and especially 2 to 500 carbon atoms; containing at least one acid function in the Brönsted sense, and especially one or more carboxylic, sulfonic and/or phosphonic acid groups; and also possibly comprising one or more heteroatoms chosen from O, N, Si, F and P, for example in the form of hydroxyl groups.

27. The polymer as claimed in one of the preceding claims, in which the neutralizer is chosen, alone or as a mixture, from:
- linear, branched or cyclic, saturated or unsaturated, optionally aromatic fatty acids containing 6 to 32 and especially 8 to 28 carbon atoms, and comprising at least one COOH or sulfonic acid (-SO₃H) function;
- linear, branched or cyclic, saturated or unsaturated, optionally aromatic hydroxy acids, especially α-hydroxy acids, containing 6 to 32 and especially 8 to 28 carbon atoms, and comprising at least one COOH or sulfonic acid (-SO₃H) function;
- alkylbenzenesulfonic acids, in which the alkyl group may contain from 4 to 30 and especially from 6 to 24 carbon atoms;
- amphoteric neutralizers, especially of the alkylbetaine or alkylamidopropylbetaine type, in which the alkyl group may contain 4 to 30 and especially 6 to 24 carbon atoms; in particular cocoamidopropylbetaine.

28. The polymer as claimed in one of the preceding claims, in which the neutralizer is chosen from α-hydroxyethanoic acid, α-hydroxyoctanoic acid, α-hydroxycaprylic acid, ascorbic acid, acetic acid, benzoic acid, behenic acid, capric acid, citric acid, caproic acid, caprylic acid, dodecylbenzenesulfonic acid, 2-ethylcaproic acid, folic acid, fumaric acid, galactaric acid, gluconic acid, glycolic acid, 2-hexadecyleicosanoic acid, hydroxycaproic acid, 12-hydroxystearic acid, isolauric acid (or 2-butyloctanoic acid), isomyristic acid (or 2-hexyloctanoic acid), isoarachidic acid (or 2-octyldodecanoic acid), isolignoceric acid (or 2-decyltetradecanoic acid), lactic acid, lauric acid, malic acid, myristic acid, oleic acid, palmitic acid, propionic acid, sebacic acid, stearic acid, tartaric acid, terephthalic acid, trimesic acid, undecylenic acid, propylbetaine, cocoamidopropylbetaine, and betaine hydrochloride of formula [(CH₃)₃N+CH₂CO₂H.Cl-], and mixtures thereof.

29. The polymer as claimed in one of the preceding claims, in which the neutralizer is chosen from caproic acid, 2-ethylcaproic acid, oleic acid, behenic acid, stearic acid, acetic acid, citric acid, tartaric acid, betaine hydrochloride and/or gluconic acid, and preferentially betaine hydrochloride and/or behenic acid.

30. The polymer as claimed in one of the preceding claims, in which the neutralizer, alone or as a mixture, is added in an amount of from 0.01% to 3 molar equivalents, especially 0.05 to 2.5 or even 0.1 to 2 molar equivalents relative to the total amine functions of the polymer or of the monomers.

31. The polymer as claimed in claim 30, in which the neutralizer, alone or as a mixture, is added in an amount of from 0.01 to 0.99 molar equivalents, especially 0.05 to 0.9 or even 0.1 to 0.8 molar equivalents relative to the total amine functions of the polymer or of the monomers.

32. The polymer as claimed in claim 30, in which the neutralizer, alone or as a mixture, is added in an amount of from 1.01 to 3 molar equivalents, especially 1.2 to 2.5 or even 1.5 to 2 molar equivalents relative to the total amine functions of the polymer or of the monomers.

33. The polymer as claimed in claim 30, in which the neutralizer, alone or as a mixture, is added in an amount of 1 molar equivalent relative to the total amine functions of the polymer or of the monomers.

34. The polymer as claimed in one of the preceding claims, with a weight-average molecular mass (Mw) of between 500 and 5 000 000, especially between 1000 and 3 000 000 and even more preferentially between 2000 and 2 000 000, or even 4000 and 500 000, inter alia between 7000 and 400 000, better still between 20 000 and 350 000 and even better between 150 000 and 300 000.

35. The polymer as claimed in one of the preceding claims, **characterized in that** it is conveyable in aqueous medium, preferably water-soluble or waterdispersible.

36. The polymer as claimed in one of the preceding claims, **characterized in that** it has a viscosity in water of between 1 and 1000 mPa.s, preferably between 1.5 and 750 nPa.s and better still between 2 and 500 mPa.s, measured at 25°C, using a Brookfield viscometer, for a solution containing 15% by weight of the polymer in water or methyl ethyl ketone, at 25°C, with a No. 1 spindle of needle type ; for a measuring time of 5 minutes, at a speed of between 0.1 and 6 rpm.

37. The polymer as claimed in one of the preceding claims, **characterized in that** it has a glass transition temperature (Tg) of between -150°C and 20°C, especially -120°C and 10°C and better still between -100°C and 0°C.

38. The polymer as claimed in one of the preceding claims, **characterized in that** it has a water uptake of between 3% and 150% by weight, preferably between 4% and 100% by weight and especially between 5% and 50% by weight, at 75% relative humidity (75% RH) ; and/or **in that** it has a water uptake of between 3% and 200% by weight, preferably between 55% and 150% by weight, especially between 60% and 120% by weight or even between 70% and 100% by weight, at 85% relative humidity (85% RH).

39. The polymer as claimed in one of the preceding claims, in which:
- the monomer of formula (I), alone or as a mixture, is present in a proportion of from 10% to 80% by weight, especially from 20% to 60% by weight and preferably from 30% to 50% by weight relative to the weight of the final polymer, and is chosen, alone or as a mixture, from poly(ethylene glycol) (meth)acrylates, preferably those with a molecular weight of between 350 and 13 000 g/mol and especially between 500 and 8000 g/mol; and
- the "essentially cationic" monomer is present in a proportion of from 20% to 90% by weight, especially from 40% to 80% by weight and preferably from 50% to 70% by weight relative to the weight of the final polymer and is chosen, alone or as a mixture, from dimethylaminopropyl(meth)acrylamide, dimethylaminoethyl(meth)acrylamide, diethylaminoethyl (meth)acrylate, dimethylaminoethyl (meth)acrylate, vinylimidazole, vinylpyridine and morpholinoethyl (meth)acrylate; and
- the polymer is neutralized with a neutralizer chosen from 2-ethylcaproic acid, oleic acid, behenic acid, stearic acid, acetic acid, citric acid, tartaric acid, betaine hydrochloride and/or gluconic acid, and preferentially behenic acid and/or betaine hydrochloride.

40. The polymer as claimed in one of the preceding claims, in which:
- the monomer of formula (I), alone or as a mixture, is present in a proportion of from 10% to 80% by weight, especially from 20% to 60% by weight and preferably from 30% to 50% by weight and is chosen, alone or as a mixture, from poly(ethylene glycol) (meth)acrylates, preferably those with a molecular weight of between 350 and 13 000 g/mol and especially between 500 and 8000 g/mol; and
- the "essentially cationic" monomer is present in a proportion of from 20% to 90% by weight, especially from 40% to 80% by weight and preferably from 50% to 70% by weight relative to the weight of the final polymer and is chosen, alone or as a mixture, from dimethylaminopropyl(meth)acrylamide, and
- the polymer is neutralized with a neutralizer chosen from behenic acid and/or betaine hydrochloride.

41. A cosmetic or pharmaceutical composition comprising, in a physiologically acceptable medium, especially a cosmetically or pharmaceutically acceptable medium, at least one polymer as defined in one of claims 1 to 40.

42. The composition as claimed in claim 41, comprising a solution in water or in an organic solvent, or alternatively an aqueous or organic dispersion of the polymer as claimed in one of claims 1 to 40.

43. The composition as claimed in either of claims 41 and 42, in which the polymer is present in a proportion of from 0.01% to 50% by weight, especially from 0.1% to 30% by weight or even from 0.3% to 10% by weight and better still from 1% to 3% by weight of solids relative to the total weight of the composition.

44. The composition as claimed in one of claims 41 to 43, in which the physiologically acceptable medium comprises at least one constituent chosen from water ; hydrophilic organic solvents, for instance alcohols, especially linear or branched C1-C6 monoalcohols and polyols and glycol ethers, especially C₂ glycol ethers and hydrophilic C₂-C₄ aldehydes; waxes, pasty fatty substances, gums and mixtures thereof, of animal, plant, mineral or synthetic origin; lipophilic organic solvents ; oils of animal, plant, mineral or synthetic origin; synthetic esters and ethers; pentaerythritol esters; fatty alcohols containing from 12 to 26 carbon atoms; partially hydrocarbon-based and/or silicone-based fluoro oils; volatile or nonvolatile, linear or cyclic silicone oils, which are liquid or pasty at room temperature; pigments, nacres, fillers, water-soluble dyes and liposoluble dyes ; polymers ; auxiliary film-forming agents; surfactants ; vitamins, fragrances, nacreous agents, thickeners, gelling agents, trace elements, softeners, sequestrants, fragrances, acidifying or basifying agents, preserving agents, sunscreens, antioxidants, hair-loss counteractants, antidandruff agents, propellants and ceramides; mixtures thereof.

45. The composition as claimed in one of claims 41 to 44, which is is the form of a suspension, a dispersion especially of oil in water by means of vesicles ; an optionally thickened or even gelled oily solution; an oil-in-water, water-in-oil or multiple emulsion; a gel or a mousse; an oily or emulsified gel; a dispersion of vesicles, especially lipid vesicles; a two-phase or multiphase lotion; a spray; a lotion, a cream, a pomade, a soft paste, an ointment, a cast or molded solid especially in stick or dish form, or alternatively a compacted solid.

46. The composition as claimed in one of claims 41 to 45, which is in the form of a care and/or makeup product for bodily or facial skin, the lips and the hair, an antisun or self-tanning product or a haircare product.

47. The composition as claimed in one of claims 41 to 46, which is in the form of a hair composition, especially for holding the hairstyle or shaping the hair, for example in the form of shampoos, hairsetting gels or lotions, blow-drying lotions, fixing and styling compositions such as lacquers or sprays; rinse-out or leave-in hair conditioners, compositions for permanent-waving, relaxing, dyeing or bleaching the hair, or alternatively in the form of rinse-out compositions, to be applied before or after dyeing, bleaching, permanent-waving or relaxing the hair or between the two steps of a permanent-waving or hair-relaxing operation.

48. A cosmetic process for treating keratin materials such as bodily or facial skin, the nails, bodily hair, head hair and/or the eyelashes, **characterized in that** it consists in applying to the keratin materials a cosmetic composition as defined in one of claims 41 to 47 and then optionally rinsing with water.

49. A cosmetic process for holding the hairstyle; for treating, caring for and/or washing and/or removing makeup from bodily or facial skin, the nails, bodily hair, head hair and/or the eyelashes, **characterized in that** it consists in applying to the keratin materials a cosmetic composition as defined in one of claims 41 to 47 and then optionally rinsing with water.

50. A process for preparing an ethylenic copolymer as defined in one of claims 1 to 40, in which the amine units of the monomers included in the constitution of the polymer, in particular the amine units of the cationic monomers of formula (IIa), are neutralized before the polymerization, and the neutralized monomers are then copolymerized in order to obtain the desired copolymer.

51. A process for preparing an ethylenic copolymer as defined in one of claims 1 to 40, in which the non-neutralized monomers included in the constitution of the polymer are polymerized, and said copolymer is then neutralized after its formation.

## Patentansprüche

1. Unvernetztes ethylenisches Copolymer, umfassend in Gew.-%, bezogen auf das Gesamtgewicht des Polymers:
- a) 10-80 Gew.-% eines oder mehrerer Monomere der Formel (I): 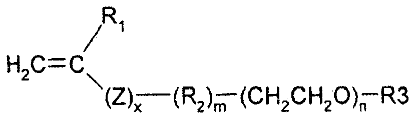 worin:
- R1 für ein Wasserstoffatom oder einen linearen oder verzweigten auf Kohlenwasserstoff basierenden Rest vom Typ CₚH₂ₚ₊₁, wobei p eine ganze Zahl zwischen 1 und 12 einschließlich bedeutet, steht;
- Z für eine unter -COO-, -CONH-, -CONCH₃-, -OCO-,
- O-, -SO₂-, -CO-O-CO- oder -CO-CH₂-CO- ausgewählte zweiwertige Gruppe steht;
- x für 0 oder 1 steht;
- R2 für einen gesättigten oder ungesättigten, gegebenenfalls aromatischen, linearen, verzweigten oder cyclischen auf Kohlenstoff basierenden zweiwertigen Rest mit 1 bis 30 Kohlenstoffatomen, der 1 bis 18 unter O, N, S, F, Si und P ausgewählte Heteroatome umfassen kann, steht;
- m für 0 oder 1 steht;
- n für eine ganze Zahl zwischen 3 und 300 einschließlich steht;
- R3 für ein Wasserstoffatom oder einen gesättigten oder ungesättigten, gegebenenfalls aromatischen, linearen, verzweigten oder cyclischen auf Kohlenstoff basierenden Rest mit 1 bis 30 Kohlenstoffatomen, der 1 bis 20 unter O, N, S, F, Si und P ausgewählte Heteroatome umfassen kann, steht;
und Salze davon;
- b) 20-90 Gew.-% mindestens eines kationischen Monomers oder eines Salzes davon, ausgewählt unter den Monomeren der Formel (IIa): 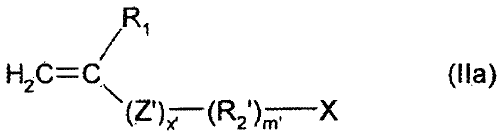 worin:
- R1 für ein Wasserstoffatom oder einen linearen oder verzweigten auf Kohlenwasserstoff basierenden Rest vom Typ CₚH₂ₚ₊₁, wobei p eine ganze Zahl zwischen 1 und 12 einschließlich bedeutet; vorzugsweise Wasserstoff oder einen Methyl-, Ethyl-, Propyl- oder Butylrest, steht;
- Z' für eine unter -COO-, -CONH-, -CONCH₃-, -OCO-oder -O-, -SO₂- -CO-O-CO- oder -CO-CH₂-CO-, vorzugsweise COO und CONH, ausgewählte zweiwertige Gruppe steht;
- x' für 0 oder 1, vorzugsweise 1, steht;
- R'2 für einen gesättigten oder ungesättigten, gegebenenfalls aromatischen, linearen, verzweigten oder cyclischen auf Kohlenstoff basierenden zweiwertigen Rest mit 1 bis 30 Kohlenstoffatomen, der 1 bis 18 unter O, N, S, F, Si und P ausgewählte Heteroatome umfassen kann, steht;
- m' für 0 oder 1 steht;
- X für
(a) eine Guanidino- oder Amidinogruppe oder
(b) eine Gruppe der Formel -N(R₆)(R₇) oder -P(R₆)(R₇) oder -P⁺R₆R₇R₈, wobei R6, R7 und R8 unabhängig voneinander (i) ein Wasserstoffatom oder (ii) eine lineare, verzweigte oder cyclische, gesättigte oder ungesättigte, gegebenenfalls aromatische Alkylgruppe mit 1 bis 18 Kohlenstoffatomen, die 1 bis 10 unter 0, N, S, F, Si und P ausgewählte Heteroatome umfassen kann, bedeuten; oder (iii) R6 und R7 mit dem Stickstoff- bzw. Phosphoratom einen ersten gesättigten oder ungesättigten, gegebenenfalls aromatischen Ring mit insgesamt 5, 6, 7 oder 8 Atomen und insbesondere 4, 5 oder 6 Kohlenstoffatomen und/oder 2 bis 4 unter O, S und N ausgewählten Heteroatomen bilden können; wobei der erste Ring mit einem oder mehreren weiteren gesättigten oder ungesättigten, gegebenenfalls aromatischen Ringen mit jeweils 5, 6 oder 7 Atomen und insbesondere 4, 5, 6 oder 7 Kohlenstoffatomen und/oder 2 bis 4 unter O, S und N ausgewählten Heteroatomen anelliert sein kann;
oder
(c) eine Gruppe -R'6-N-R'7-, worin R'6 und R'7 mit dem Stickstoffatom einen gesättigten oder ungesättigten, gegebenenfalls aromatischen Ring mit insgesamt 5, 6, 7 oder 8 Atomen und insbesondere 4, 5 oder 6 Kohlenstoffatomen und/oder 2 bis 4 unter O, S und N ausgewählten Heteroatomen bilden können; wobei der Ring mit einem oder mehreren weiteren gesättigten oder ungesättigten, gegebenenfalls aromatischen Ringen mit jeweils 5, 6 oder 7 Atomen und insbesondere 4, 5, 6, 7 oder 8 Kohlenstoffatomen und/oder 2 bis 4 unter O, S und N ausgewählten Heteroatomen anelliert sein kann;
steht;
wobei das Copolymer zumindest teilweise durch mindestens ein unter organischen Säuren im Sinne von Brönsted ausgewählten Neutralisationsmittel neutralisiert ist.

2. Polymer nach Anspruch 1, wobei in der Formel (I) R1 für Wasserstoff oder einen Methyl-, Ethyl-, Propyl- oder Butylrest steht.

3. Polymer nach einem der vorhergehenden Ansprüche, wobei in der Formel (I) Z für COO oder CONH steht.

4. Polymer nach einem der vorhergehenden Ansprüche, wobei in der Formel (I) der Rest R2 unter:
- einem Alkylenrest wie Methylen, Ethylen, Propylen, n-Butylen, Isobutylen, tert.-Butylen, n-Hexylen, n-Octylen, n-Dodecylen, n-Octadecylen, n-Tetradecylen oder n-Docosanylen;
- einem Phenylenrest -C₆H₄- (ortho, meta oder para), der gegebenenfalls durch einen C1-C12-Alkylrest, der gegebenenfalls 1 bis 25 unter O, N, S, F, Si und P ausgewählte Heteroatome umfasst, substituiert ist, oder einem Benzylenrest -C₆H₄-CH₂-, der gegebenenfalls durch einen C1-C12-Alkylrest, der gegebenenfalls 1 bis 8 unter O, N, S, F, Si und P ausgewählte Heteroatome umfasst, substituiert ist;
- einem Pyridiniumrest der Formel: 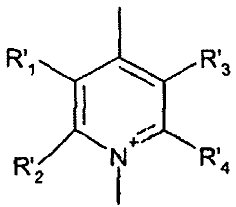 wobei R'1 bis R'4 gleich oder verschieden sind und unter H und einem C1-C12-Alkylrest, der gegebenenfalls 1 bis 8 unter O, N, S, F, Si und P ausgewählte Heteroatome umfasst, ausgewählt sind und R'1 bis R'4 insbesondere für Methyl und/oder Ethyl stehen können;
- einem Rest der Formel -CH₂-O-CO-O-, CH₂-CH₂-O-CO-O-, -CH₂-CO-O-, -CH₂-CH₂-CO-O-, -CH₂-O-CO-NH-, -CH₂-CH₂-O-CO-NH-; -CH₂-NH-CO-NH-, -CH₂-CH₂-NH-CO-NH-; -CH₂-CHOH-, -CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R'')-, -CH₂-CH(NR'R") -, -CH₂-CH₂-CH₂-NR'-, -CH₂-CH₂-CH₂-O- ; -CH₂-CH₂-CHR'-O-, wobei R' und R" für einen linearen oder verzweigten C1-C22-Alkylrest, der gegebenenfalls 1 bis 12 unter O, N, S, F, Si und P ausgewählte Heteroatome umfasst, stehen;
- oder einer Mischung dieser Reste
ausgewählt ist.

5. Polymer nach einem der vorhergehenden Ansprüche, wobei in der Formel (I) n zwischen 5 und 200 einschließlich und noch besser zwischen 7 und 100 einschließlich oder sogar zwischen 9 und 50 einschließlich liegt.

6. Polymer nach einem der vorhergehenden Ansprüche, wobei in der Formel (I) R3 für ein Wasserstoffatom; einen Succinimido-, Maleinimido-, Mesityl-, Tosyl-, Triethoxysilan-, Phthalimid- oder -CH₂-CH₂CN-Rest oder einen Benzyl- oder Phenylrest, der gegebenenfalls durch einen C1-C12-Alkylrest, der gegebenenfalls 1 bis 8 unter O, N, S, F, Si und P ausgewählte Heteroatome umfasst, substituiert ist; einen C1-C30-, insbesondere C1-C22- oder sogar C2-C16-Alkylrest, der gegebenenfalls 1 bis 18 unter O, N, S, F, Si und P ausgewählte Heteroatome umfasst, steht;
wobei die Benzyl-, Phenyl- oder Alkylreste außerdem gegebenenfalls eine unter den folgenden Funktionen ausgewählte Funktion umfassen können:
Succinimido; Glutarat-succinimido; Glutarat; Maleinimido; Mesityl; Benzoat; Tosyl; Triethoxysilan; Phthalimid; Thioester; Benzotriazolcarbonat; Butyraldehyd; Acetaldehyddiethylacetal ; Biotin; Phospholipid; Succinat; N-Hydroxysuccinimid; -SO₃H, -COOH, -PO₄, -NR5R6 oder -N⁺R5R6R7, wobei R5, R6 und R7 unabhängig voneinander unter H oder linearen, verzweigten oder cyclischen C1-C18-Alkylresten, insbesondere Methyl, die gegebenenfalls 1 oder mehrere Heteroatome oder auch Schutzgruppen wie t-Butyloxycarbonyl oder 9-Fluorenylmethoxycarbonyl umfassen, ausgewählt sind.

7. Polymer nach einem der vorhergehenden Ansprüche, wobei das Monomer der Formel (I) unter:
- Poly(ethylenglykol)(meth)acrylat;
- Methylpoly(ethylenglykol)(meth)acrylat ;
- Alkylpoly(ethylenglykol)(meth)acrylaten ;
- Phenylpoly(ethylenglykol)(meth)acrylaten;
- dem folgenden Monomer: 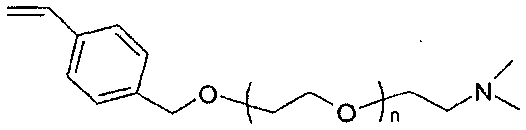 worin n vorzugsweise zwischen 3 und 100 einschließlich, insbesondere 5 bis 50 einschließlich oder sogar 7 bis 30 einschließlich liegt;
allein oder als Mischung, ausgewählt ist.

8. Polymer nach einem der vorhergehenden Ansprüche, wobei das Monomer der Formel (I) ein Molekulargewicht zwischen 350 und 13.000 g/mol und insbesondere zwischen 500 und 8000 g/mol aufweist.

9. Polymer nach einem der vorhergehenden Ansprüche, wobei das Monomer der Formel (I) unter Poly-(ethylenglykol)(meth)acrylaten und Methylpoly-(ethylenglykol)(meth)acrylaten, vorzugsweise denjenigen mit einem Molekulargewicht zwischen 350 und 13.000 g/mol und insbesondere zwischen 500 und 8000 g/mol ausgewählt ist.

10. Polymer nach einem der vorhergehenden Ansprüche, wobei das Monomer der Formel (I) unter Poly-(ethylenglykol)(meth)acrylaten und insbesondere denjenigen mit einem Molekulargewicht zwischen 350 und 13.000 g/mol und insbesondere zwischen 500 und 8000 g/mol ausgewählt ist.

11. Polymer nach einem der vorhergehenden Ansprüche, wobei das Monomer der Formel (I) allein oder als Mischung in einem Anteil von 20 bis 60 Gew.-%, bezogen auf das Gewicht des letztendlichen Polymers, vorzugsweise 30 bis 50 Gew.-%, vorliegt.

12. Polymer nach einem der vorhergehenden Ansprüche, wobei in dem Monomer der Formel (IIa) der Rest R'2 unter:
- einem Alkylenrest wie Methylen, Ethylen, Propylen, n-Butylen, Isobutylen, tert.-Butylen, n-Hexylen, n-Octylen, n-Dodecylen, n-Octadecylen, n-Tetradecylen oder n-Docosanylen;
- einem Phenylenrest -C₆H₄- (ortho, meta oder para), der gegebenenfalls durch einen C1-C12-Alkylrest, der gegebenenfalls 1 bis 25 unter O, N, S, F, Si und/oder P ausgewählte Heteroatome umfasst, substituiert ist, oder einem Benzylenrest -C₆H₄-CH₂-, der gegebenenfalls durch einen C1-C12-Alkylrest, der gegebenenfalls 1 bis 25 unter O, N, S, F, Si und P ausgewählte Heteroatome umfasst, substituiert ist;
- einem Rest der Formel -CH₂-O-CO-O-, CH₂-CH₂-O-CO-O-, -CH₂-CO-O-, -CH₂-CH₂-CO-O-, - [(CH₂)₅-CO-O]ₙ-, -CH₂-CH(CH₃)-O-, -(CH₂)₂-O-, -CH₂-O-CO-NH-, -CH₂-CH₂-O-CO-NH- ; -CH₂-NH-CO-NH- oder -CH₂-CH₂-NH-CO-NH-, -CH₂-CHOH-, -CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R'')-, -CH₂-CH(NR'R")-, -CH₂-CH₂-CH₂-NR'-, -CH₂-CH₂-CH₂-O- ; -CH₂-CH₂-CHR'-O-, wobei R' und R" für einen linearen oder verzweigten C1-C22-Alkylrest, der gegebenenfalls 1 bis 12 unter O, N, S, F, Si und P ausgewählte Heteroatome umfasst, stehen;
- oder einer Mischung dieser Reste
ausgewählt ist.

13. Polymer nach einem der vorhergehenden Ansprüche, wobei in dem Monomer der Formel (IIa) die Reste R6 und R7 unter Wasserstoff und einer Methyl-, Ethyl, Propyl-, Isopropyl-, n-Butyl-, t-Butyl-, Isobutyl-, Octyl-, Lauryl- oder Stearylgruppe; vorzugsweise Wasserstoff, Methyl und Ethyl, ausgewählt sind.

14. Polymer nach einem der vorhergehenden Ansprüche, wobei in dem Monomer der Formel (IIa) X für einen unter Resten vom Pyridin-, Indolyl-, Isoindolinyl, Imidazolyl-, Imidazolinyl-, Piperidinyl-, Pyrazolinyl-, Pyrazolyl-, Chinolin-, Pyrazolinyl-, Pyridinyl-, Piperazinyl-, Pyrrolidinyl-, Chinidinyl-, Thiazolinyl-, Morpholin-, Guanidinooder Amidino-Typ und Mischungen davon ausgewählten Rest steht.

15. Polymer nach einem der vorhergehenden Ansprüche, wobei das Monomer der Formel (IIa)unter Dimethylaminopropyl(meth)acrylamid, Dimethylaminoethyl(meth)acrylamid, Diethylaminoethyl(meth)-acrylat, Dimethylaminoethyl(meth)acrylat, Vinylimidazol, Vinylpyridin und Morpholinoethyl(meth)-acrylat und den nachstehenden Monomeren: 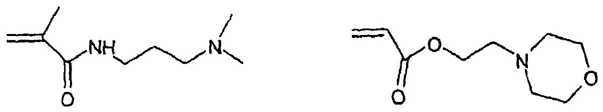 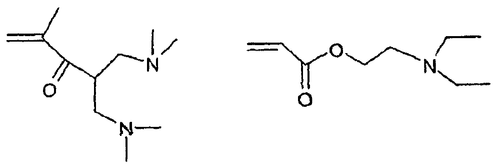 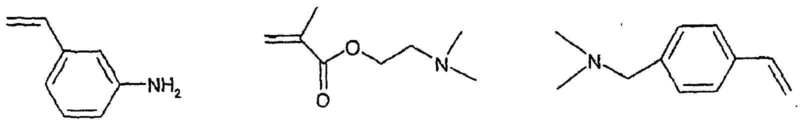 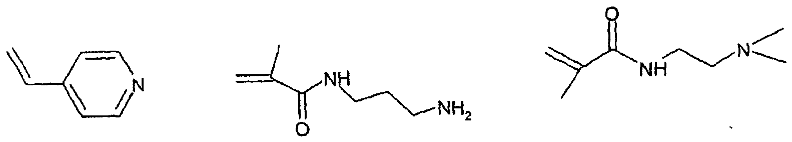 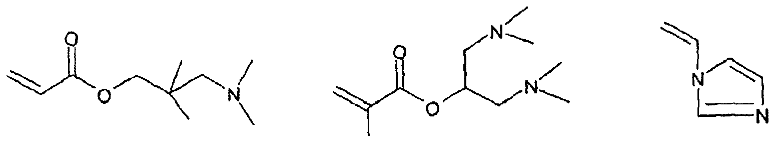 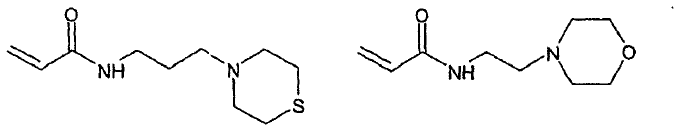 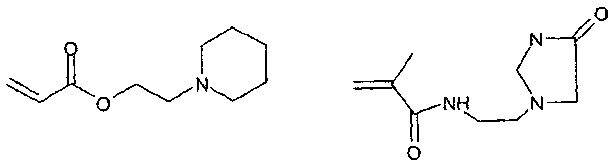 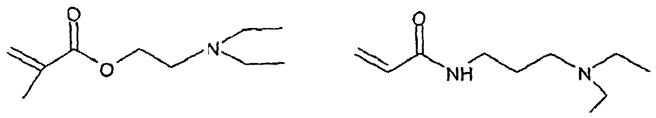 allein oder als Mischung ausgewählt ist.

16. Polymer nach einem der vorhergehenden Ansprüche, wobei das kationische Monomer der Formel (IIa) in einem Anteil von 40 bis 80 Gew.-%, bezogen auf das Gewicht des letztendlichen Polymers, vorzugsweise 50 bis 70 Gew.-%, vorliegt.

17. Polymer nach einem der vorhergehenden Ansprüche, umfassend ein oder mehrere zusätzliche ionische Monomere, ausgewählt unter amphoteren Monomeren der Formeln (IIc) und (IId) und/oder anionischen Monomeren, ausgewählt unter Maleinsäureanhydrid und/oder denjenigen der Formel (IIb): 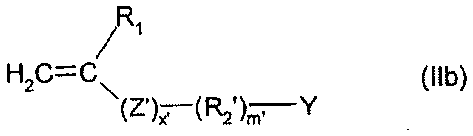 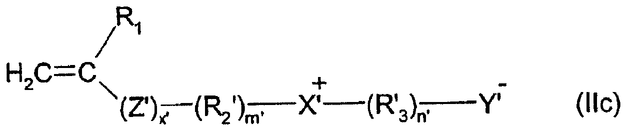 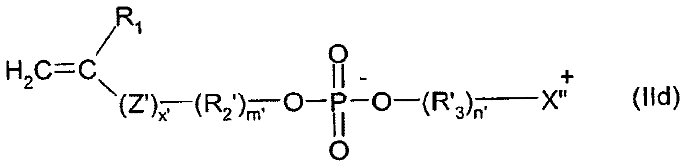 worin:
- R1, Z', x', R'2 und m' die gleiche Bedeutung wie in Anspruch 1 für Formel (IIa) angegeben besitzen;
- Y für eine unter -COOH, -SO₃H, -OSO₃H, -PO₃H₂ und
- OPO₃H₂ ausgewählte Gruppe steht;
- X'⁺ für eine zweiwertige Gruppe der Formel -N⁺(R₆) (R₇)- steht, wobei R6 und R7 unabhängig voneinander (i) ein Wasserstoffatom oder (ii) eine lineare, verzweigte oder cyclische, gegebenenfalls aromatische Alkylgruppe mit 1 bis 25 Kohlenstoffatomen, die 1 bis 20 unter O, N, S und P ausgewählte Heteroatome umfassen kann, bedeuten; oder (iii) R6 und R7 mit dem Stickstoffatom einen ersten gesättigten oder ungesättigten, gegebenenfalls aromatischen Ring mit insgesamt 5, 6, 7 oder 8 Atomen und insbesondere 4, 5, 6 oder 7 Kohlenstoffatomen und/oder 2 bis 3 unter O, S und N ausgewählten Heteroatomen bilden können; wobei der erste Ring mit einem oder mehreren weiteren gesättigten oder ungesättigten, gegebenenfalls aromatischen Ringen mit jeweils 5, 6, 7 oder 8 Atomen und insbesondere 4, 5, 6 oder 7 Kohlenstoffatomen und/oder 2 bis 3 unter O, S und N ausgewählten Heteroatomen anelliert sein kann;
- Y'⁻ für eine unter -COO⁻, -SO₃⁻, -OSO₃⁻, -PO₃²⁻ und -OPO₃²⁻ ausgewählte Gruppe steht;
- R'3 für einen gesättigten oder ungesättigten, gegebenenfalls aromatischen, linearen, verzweigten oder cyclischen zweiwertigen auf Kohlenstoff basierenden Rest mit 1 bis 30 Kohlenstoffatomen, der 1 bis 18 unter O, N, S, F, Si und P ausgewählte Heteroatome umfassen kann, steht;
n' zwischen 1 und 100 und vorzugsweise zwischen 1 und 5 einschließlich liegt;
- X"⁺ für eine Gruppe der Formel -N⁺R₆R₇R₈, wobei R6, R7 und R8 unabhängig voneinander (i) ein Wasserstoffatom oder (ii) eine lineare, verzweigte oder cyclische, gegebenenfalls aromatische Alkylgruppe mit 1 bis 18 Kohlenstoffatomen, die 1 bis 5 unter O, N, S und P ausgewählte Heteroatome umfassen kann, bedeuten; oder (iii) R6 und R7 mit dem Stickstoffatom einen ersten gesättigten oder ungesättigten, gegebenenfalls aromatischen Ring mit insgesamt 5, 6 oder 7 Atomen und insbesondere 4, 5 oder 6 Kohlenstoffatomen und/oder 2 bis 3 unter 0, S und N ausgewählten Heteroatomen bilden können; wobei der erste Ring mit einem oder mehreren weiteren gesättigten oder ungesättigten, gegebenenfalls aromatischen Ringen mit jeweils 5, 6 oder 7 Atomen und insbesondere 4, 5, 6 oder 7 Kohlenstoffatomen und/oder 2 bis 3 unter O, S und N ausgewählten Heteroatomen anelliert sein kann; insbesondere die Reste Trimethylammonium;
Triethylammonium; N,N-Dimethyl-N-octylammonium ; N,N-Dimethyl-N-laurylammonium; steht.

18. Polymer nach Anspruch 17, wobei in dem Monomer der Formel (IIc) und/oder (IId) der Rest R'3 unter:
- einem Alkylenrest wie Methylen, Ethylen, Propylen, n-Butylen, Isobutylen, tert.-Butylen, n-Hexylen, n-Octylen, n-Dodecylen, n-Octadecylen, n-Tetradecylen oder n-Docosanylen;
- einem Phenylenrest -C₆H₄- (ortho, meta oder para), der gegebenenfalls durch einen C1-C12-Alkylrest, der gegebenenfalls 1 bis 5 unter O, N, S, F, Si und P ausgewählte Heteroatome umfasst, substituiert ist, oder einem Benzylenrest -C₆H₄-CH₂-, der gegebenenfalls durch einen C1-C12-Alkylrest, der gegebenenfalls 1 bis 5 unter O, N, S, F, Si und P ausgewählte Heteroatome umfasst, substituiert ist;
- einem Rest der Formel -CH₂-O-CO-O-, CH₂-CH₂-O-CO-O-, -CH₂-CO-O-, -CH₂-CH₂-CO-O-, [(CH₂)₅-CO-O]ₙ-, -CH₂-CH(CH₃)-O-, - (CH₂)₂-O-, -CH₂-O-CO-NH-, -CH₂-CH₂-O-CO-NH-; -CH₂-NH-CO-NH- oder -CH₂-CH₂-NH-CO-NH-, -CH₂-CHOH-, -CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R") -, -CH₂-CH (NR'R") -, -CH₂-CH₂-CH₂-NR'-, -CH₂-CH₂-CH₂-O-; -[CH₂-CH₂-O]ₙ- und - [CH₂-CH (CH₃) -O]ₙ-, -CH₂-CH₂-CHR'-O-, wobei R' und R" für einen linearen oder verzweigten C1-C22-Alkylrest, der gegebenenfalls 1 bis 12 unter O, N, S, F, Si und P ausgewählte Heteroatome umfasst, stehen;
- oder einer Mischung dieser Reste ausgewählt ist.

19. Polymer nach einem der Ansprüche 17 bis 18, wobei die anionischen Monomere unter Maleinsäureanhydrid, Acrylsäure, Methacrylsäure, Crotonsäure, Itaconsäure, Fumarsäure, Maleinsäure, 2-Carboxyethylacrylat (CH₂=CH-C (O) -O- (CH₂)₂-COOH) ; Styrolsulfonsäure, 2-Acrylamido-2-methylpropansulfonsäure, Vinylbenzoesäure, Vinylphosphorsäure, Sulfopropyl(meth)acrylat und Salzen davon ausgewählt sind.

20. Polymer nach einem der Ansprüche 17 bis 19, wobei die Monomere der Formeln (IIc) bzw. (IId) unter N,N-Dimethyl-N-(2-methacryloyloxyethyl)-N-(3-sulfopropyl)ammoniumbetain, N,N-Dimethyl-N-(3-methacrylamidopropyl)-N-(3-sulfopropyl)ammonium-betain, 1-(3-Sulfopropyl)-2-vinylpyridiniumbetain und 2-Methacryloyloxyethylphosphorylcholin ausgewählt sind.

21. Polymer nach einem der Ansprüche 17 bis 20, wobei die zusätzlichen ionischen Monomere in einem Anteil von 5 bis 40 Gew.-%, bezogen auf das Gewicht der Mischung von "kationischen + zusätzlichen ionischen Monomeren", insbesondere 10 bis 30 Gew.-%, vorzugsweise 15 bis 25 Gew.-%, vorliegen.

22. Polymer nach einem der Ansprüche 17 bis 21, wobei die ionischen Monomere in einem Anteil von 20 bis 90 Gew.-%, bezogen auf das Gewicht des letztendlichen Polymers, insbesondere 40 bis 80 Gew.-%, vorzugsweise 50 bis 70 Gew.-%, vorliegen.

23. Polymer nach einem der vorhergehenden Ansprüche, das außerdem mindestens ein zusätzliches nichtionisches hydrophiles Monomer umfasst, das unter denjenigen der Formel (III): 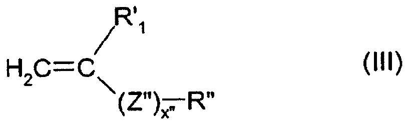 worin:
- R'₁ für Wasserstoff oder -CH₃ steht;
- Z" für eine unter -COO-, -CONH-, -CONCH₃-, -OCO-, -SO₂ -CO-O-CO-, -CO-CH₂-CO- oder -O-, vorzugsweise COO und CONH, ausgewählte zweiwertige Gruppe steht;
- x" für 0 oder 1 steht;
- R" für einen gesättigten oder ungesättigten, gegebenenfalls aromatischen, linearen, verzweigten oder cyclischen auf Kohlenstoff basierenden Rest mit 1 bis 30 Kohlenstoffatomen, der 1 bis 18 unter O, N, S, F, Si und P ausgewählte Heteroatome umfassen kann; insbesondere Methyl, Ethyl, Propyl, Isopropyl, n-Butyl, Isobutyl, tert.-Butyl, Phenyl, Benzyl oder einen Rest der Formel -CH₂-CH₂-CH₂OH, - CH₂-CH₂-OH, -CH₂-CH₂-CH₂OH oder Furfuryl, steht; allein oder als Mischung ausgewählt ist.

24. Polymer nach Anspruch 23, wobei das zusätzliche nichtionische hydrophile Monomer unter Methylmethacrylat, Methylacrylat, Ethylmethacrylat, Ethylacrylat, Propylacrylat, Isopropylacrylat, Tetrahydrofurfurylmethacrylat, Tetrahydrofurfurylacrylat, 2-Hydroxyethylmethacrylat, 2-Hydroxyethylacrylat, Ethoxyethylmethacrylat, Ethoxyethylacrylat, N-Isopropylacrylamid, N-Isopropylmethacrylamid, N,N-Dimethylacrylamid, N,N-Dimethylmethacrylamid, Vinylacetat, Methylvinylether, Ethylvinylether, Vinylpyrrolidon, Vinylcaprolactam, N-Vinylacetamid und Hydroxypropylacrylat; N-Vinyllactam, Acrylamid, N-Methylacrylamid, N,N-Dimethylacrylamid, N-Methyl-N-vinylacetamid, N-Vinylformamid, N-Methyl-N-vinylformamid, Vinylalkohol (copolymerisiert in Form von Vinylacetat und dann hydrolysiert) ausgewählt ist.

25. Polymer nach einem der Ansprüche 23 bis 24, wobei das zusätzliche nichtionische hydrophile Monomer allein oder als Mischung in einer Menge von 0,1 bis 35 Gew.-%, vorzugsweise 1 bis 25 Gew.-%, beispielsweise 3 bis 15 Gew.-% oder sogar 5 bis 9,5 Gew.-%, bezogen auf das Gesamtgewicht des Polymers, vorliegt.

26. Polymer nach einem der vorhergehenden Ansprüche, wobei das Neutralisationsmittel unter linearen, verzweigten oder cyclischen aliphatischen Säuren und/oder ungesättigten oder aromatischen Säuren, die insbesondere 1 bis 1000 Kohlenstoffatome, insbesondere 2 bis 500 Kohlenstoffatome, umfassen können, mindestens eine Säurefunktion im Sinne von Brönsted und insbesondere eine oder mehrere Carbon-, Sulfon- und/oder Phosphonsäuregruppen aufweisen und außerdem ein oder mehrere unter O, N, Si, F und P ausgewählte Heteroatome umfassen können, beispielsweise in Form von Hydroxylgruppen, ausgewählt ist.

27. Polymer nach einem der vorhergehenden Ansprüche, wobei das Neutralisationsmittel unter:
- linearen, verzweigten oder cyclischen, gesättigten oder ungesättigten, gegebenenfalls aromatischen Fettsäuren mit 6 bis 32 und insbesondere 8 bis 28 Kohlenstoffatomen und mindestens einer COOH-Funktion oder Sulfonsäurefunktion (-SO₃H);
- lineare, verzweigte oder cyclische, gesättigte oder ungesättigte, gegebenenfalls aromatische Hydroxysäuren, insbesondere alpha-Hydroxysäuren mit 6 bis 32 und insbesondere 8 bis 28 Kohlenstoffatomen und mindestens einer COOH-Funktion oder Sulfonsäurefunktion (-SO₃H);
- Alkylbenzolsulfonsäuren, in denen die Alkylgruppe 4 bis 30 und insbesondere 6 bis 24 Kohlenstoffatome umfassen kann;
- amphoteren Neutralisationsmitteln, insbesondere vom Alkylbetain- oder Alkylamidopropylbetain-Typ, in denen die Alkylgruppe 4 bis 30 und insbesondere 6 bis 24 Kohlenstoffatome umfassen kann; insbesondere Cocoamidopropylbetain;
allein oder als Mischung ausgewählt ist.

28. Polymer nach einem der vorhergehenden Ansprüche, wobei das Neutralisationsmittel unter α-Hydroxyethansäure, α-Hydroxyoctansäure, α-Hydroxycaprylsäure, Ascorbinsäure, Essigsäure, Benzoesäure, Behensäure, Caprinsäure, Citronensäure, Capronsäure, Caprylsäure, Dodecylbenzolsulfonsäure, 2-Ethylcapronsäure, Folsäure, Fumarsäure, Galaktarsäure, Gluconsäure, Glykolsäure, 2-Hexadecyleicosansäure, Hydroxycapronsäure, 12-Hydroxystearinsäure, Isolaurinsäure (oder 2-Butyloctansäure), Isomyristinsäure (oder 2-Hexyloctansäure), Isoarachinsäure (oder 2-Octyldodecansäure), Isolignocerinsäure (oder 2-Decyltetradecansäure), Milchsäure, Laurinsäure, Äpfelsäure, Myristinsäure, ölsäure, Palmitinsäure, Propionsäure, Sebacinsäure, Stearinsäure, Weinsäure, Terephthalsäure, Trimesinsäure, Undecylensäure, Propylbetain, Cocoamidopropylbetain und Betain-Hydrochlorid der Formel [(CH₃)₃N+CH₂CO₂H.Cl-] und Mischungen davon ausgewählt ist.

29. Polymer nach einem der vorhergehenden Ansprüche, wobei das Neutralisationsmittel unter Capronsäure, 2-Ethylcapronsäure, Ölsäure, Behensäure, Stearinsäure, Essigsäure, Citronensäure, Weinsäure, Betain-Hydrochlorid und/oder Gluconsäure und vorzugsweise Betain-Hydrochlorid und/oder Behensäure ausgewählt ist.

30. Polymer nach einem der vorhergehenden Ansprüche, wobei das Neutralisationsmittel allein oder als Mischung in einer Menge von 0,01 bis 3 Moläquivalenten, insbesondere 0,05 bis 2,5 oder sogar 0,1 bis 2 Moläquivalenten, bezogen auf die gesamten Aminfunktionen des Polymers oder der Monomere, zugesetzt ist.

31. Polymer nach Anspruch 30, wobei das Neutralisationsmittel allein oder als Mischung in einer Menge von 0,01 bis 0,99 Moläquivalenten, insbesondere 0,05 bis 0,9 oder sogar 0,1 bis 0,8 Moläquivalenten, bezogen auf die gesamten Aminfunktionen des Polymers oder der Monomere, zugesetzt ist.

32. Polymer nach Anspruch 30, wobei das Neutralisationsmittel allein oder als Mischung in einer Menge von 1,01 bis 3 Moläquivalenten, insbesondere 1,2 bis 2,5 oder sogar 1,5 bis 2 Moläquivalenten, bezogen auf die gesamten Aminfunktionen des Polymers oder der Monomere, zugesetzt ist.

33. Polymer nach Anspruch 30, wobei das Neutralisationsmittel allein oder als Mischung in einer Menge von 1 Moläquivalent, bezogen auf die gesamten Aminfunktionen des Polymers oder der Monomere, zugesetzt ist.

34. Polymer nach einem der vorhergehenden Ansprüche, das eine gewichtsmittlere Molmasse (Mw) zwischen 500 und 5.000.000, insbesondere zwischen 1000 und 3.000.000 und weiter bevorzugt zwischen 2000 und 2.000.000 oder sogar zwischen 4000 und 500.000, u.a. zwischen 7000 und 400.000, noch besser 20.000 und 350.000 und noch besser 150.000 bis 300.000 aufweist.

35. Polymer nach einem der vorhergehenden Ansprüche, **dadurch gekennzeichnet, dass** es in wässriges Medium überführbar und vorzugsweise wasserlöslich oder wasserdispergierbar ist.

36. Polymer nach einem der vorhergehenden Ansprüche, **dadurch gekennzeichnet, dass** es eine Viskosität in Wasser zwischen 1 und 1000 mPa.s, vorzugsweise zwischen 1,5 und 750 mPa.s und noch besser zwischen 2 und 500 mPa.s, gemessen bei 25°C mit Hilfe eines Brookfield-Viskosimeters für eine 15 gew.-%ige Lösung des Polymers in Wasser oder Methylethylketon bei 25°C mit Spindel Nr. 1 über einen Messzeitraum von 5 Minuten bei einer Geschwindigkeit zwischen 0,1 und 6 Umdrehungen/Minute aufweist.

37. Polymer nach einem der vorhergehenden Ansprüche, **dadurch gekennzeichnet, dass** es eine Glasübergangstemperatur (Tg) zwischen -150 und 20°C, insbesondere -120°C und 10°C und noch besser zwischen -100°C und 0°C aufweist.

38. Polymer nach einem der vorhergehenden Ansprüche, **dadurch gekennzeichnet, dass** es eine Wasseraufnahme zwischen 3 und 150 Gew.-%, vorzugsweise zwischen 4 und 100 Gew.-%, insbesondere zwischen 5 und 50 Gew.-%, bei 75% relativer Feuchte (75% RF) und/oder eine Wasseraufnahme zwischen 3 und 200 Gew.-%, vorzugsweise zwischen 55 und 150 Gew.-%, insbesondere zwischen 60 und 120 Gew.-% oder sogar zwischen 70 und 100 Gew.-% bei 85% relativer Feuchte (85% RF) aufweist.

39. Polymer nach einem der vorhergehenden Ansprüche, wobei:
- das Monomer der Formel (I) allein oder als Mischung in einem Anteil von 10 bis 80 Gew.-%, bezogen auf das Gewicht des letztendlichen Polymers, insbesondere 20 bis 60 Gew.-%, vorzugsweise 30 bis 50 Gew.-%, vorliegt und unter Poly-(ethylenglykol)(meth)acrylaten und vorzugsweise denjenigen mit einem Molekulargewicht zwischen 350 und 13.000 g/mol und insbesondere zwischen 500 und 8000 g/mol allein oder als Mischung ausgewählt ist; und
- das 'im Wesentlichen kationische' Monomer in einem Anteil von 20 bis 90 Gew.-%, bezogen auf das Gewicht des letztendlichen Polymers, insbesondere 40 bis 80 Gew.-%, vorzugsweise 50 bis 70 Gew.-%, vorliegt und unter Dimethylaminopropyl(meth)acrylamid, Dimethylaminoethyl(meth)acrylamid, Diethylaminoethyl(meth)acrylat, Dimethylaminoethyl(meth)-acrylat, Vinylimidazol, Vinylpyridin und Morpholinoethyl(meth)acrylat allein oder als Mischung ausgewählt ist; und
- das Polymer durch ein unter 2-Ethylcapronsäure, Ölsäure, Behensäure, Stearinsäure, Essigsäure, Citronensäure, Weinsäure, Betain-Hydrochlorid und/oder Gluconsäure und vorzugsweise Behensäure und/oder Betain-Hydrochlorid ausgewähltes Neutralisationsmittel neutralisiert ist.

40. Polymer nach einem der vorhergehenden Ansprüche, wobei:
- das Monomer der Formel (I) allein oder als Mischung in einem Anteil von 10 bis 80 Gew.-%, bezogen auf das Gewicht des letztendlichen Polymers, insbesondere 20 bis 60 Gew.-%, vorzugsweise 30 bis 50 Gew.-%, vorliegt und unter Poly-(ethylenglykol)(meth)acrylaten und vorzugsweise denjenigen mit einem Molekulargewicht zwischen 350 und 13.000 g/mol und insbesondere zwischen 500 und 8000 g/mol allein oder als Mischung ausgewählt ist; und
- das 'im Wesentlichen kationische' Monomer in einem Anteil von 20 bis 90 Gew.-%, bezogen auf das Gewicht des letztendlichen Polymers, insbesondere 40 bis 80 Gew.-%, vorzugsweise 50 bis 70 Gew.-%, vorliegt und unter Dimethylaminopropyl(meth)acrylamid ausgewählt ist; und
- das Polymer durch ein unter Behensäure und/oder Betain-Hydrochlorid ausgewähltes Neutralisationsmittel neutralisiert ist.

41. Kosmetische oder pharmazeutische Zusammensetzung, die in einem physiologisch unbedenklichen und insbesondere kosmetisch oder pharmazeutisch unbedenklichen Medium mindestens ein Polymer gemäß einem der Ansprüche 1 bis 40 umfasst.

42. Zusammensetzung nach Anspruch 41, die eine Lösung des Polymers gemäß einem der Ansprüche 1 bis 40 in Wasser oder in einem organischen Lösungsmittel oder eine wässrige oder organische Dispersion des Polymers gemäß einem der Ansprüche 1 bis 40 umfasst.

43. Zusammensetzung nach Anspruch 41 oder 42, in der das Polymer in einem Anteil von 0,01 bis 50 Gew.-% Trockensubstanz, insbesondere 0,1 bis 30 Gew.-% oder sogar 0,3 bis 10 Gew.-% oder noch besser 1 bis 3 Gew.-%, bezogen auf das Gesamtgewicht der Zusammenset.zung, vorliegt.

44. Zusammensetzung nach einem der Ansprüche 41 bis 43, in der das physiologisch unbedenkliche Medium mindestens einen unter Wasser; hydrophilen organischen Lösungsmitteln wie Alkoholen, insbesondere linearen oder verzweigten C1-C6-Monoalkohole und Polyolen und Glykolethern, insbesondere C₂-Glykolethern, und hydrophilen C₂-C₄-Aldehyden; Wachsen, pastösen Fettkörpern, Gummen und Mischungen davon tierischen, pflanzlichen, mineralischen oder synthetischen Ursprungs; organischen lipophilen Lösungsmitteln; Ölen tierischen, pflanzlichen, mineralischen oder synthetischen Ursprungs; synthetischen Estern und Ethern; Pentaerythritestern; Fettalkoholen mit 12 bis 26 Kohlenstoffatomen; teilfluorierten Kohlenwasserstoff- und/oder Silikonölen; flüchtigen oder nichtflüchtigen, linearen oder cyclischen, bei Raumtemperatur flüssigen oder pastösen Silikonölen; Pigmenten, Perlmutt, Füllstoffen; wasserlöslichen Farbmitteln; fettlöslichen Farbmitteln; Polymeren; Filmbildungshilfsmitteln; Tensiden; Vitaminen,
Parfümen, Perlglanzmitteln, Verdickungsmitteln, Gelierungsmitteln, Spurenelementen, Glättungsmitteln, Sequestriermitteln, Parfümen, Alkalinisierungs- oder Ansäuerungsmitteln, Konservierungsmitteln, Sonnenschutzmitteln, Antioxidantien, Mitteln gegen Haarausfall, Antischuppenmitteln, Treibmitteln, Ceramiden und Mischungen davon ausgewählten Bestandteil umfasst.

45. Zusammensetzung nach einem der Ansprüche 41 bis 44, die in Form einer Suspension, einer Dispersion, insbesondere einer Öl-in-Wasser-Dispersion durch Vehikel; einer gegebenenfalls verdickten oder sogar gelierten öligen Lösung; einer Öl-in-Wasser-Emulsion, Wasser-in-Öl-Emulsion oder multiplen Emulsion; eines Gels oder eines Schaums; eines öligen oder emulgierten Gels; einer Dispersion von Vesikeln, insbesondere Lipidvesikeln; einer zweiphasigen oder multiphasigen Lotion; eines Sprays; einer Lotion, einer Creme, einer Pomade; einer weichen Paste, einer Salbe, eines gegossenen oder geformten Feststoffs, insbesondere in Stift- oder Schalenform, oder eines kompaktieren Feststoffs vorliegt.

46. Zusammensetzung nach einem der Ansprüche 41 bis 45, die in Form eines Produkts zur Pflege und/oder zum Make-up der Körper- oder Gesichtshaut, der Lippen und der Haare, eines Sonnenschutz- oder Selbstbräunungsprodukts oder eines Haarprodukts vorliegt.

47. Zusammensetzung nach einem der Ansprüche 41 bis 46, die in Form eines Haarprodukts, insbesondere zur Festigung der Frisur oder zur Haargestaltung, beispielsweise in Form von Shampoos, Gelen, Lotionen für die Wasserwelle, Lotionen für die Fönwelle, Zusammensetzungen zur Fixierung der Frisur wie Lacken oder Sprays; Wash-out- oder Leave-in-Haarpflegespülungen, Dauerwellen-, Entkrausungs-, Färbe- oder Bleichzusammensetzungen oder auch in Form von Wash-out-Zusammensetzungen zur Anwendung vor oder nach einer Färbung, Bleichung, Dauerwelle oder Entkrausung oder auch zwischen zwei Schritten einer Dauerwelle oder Entkrausung.

48. Verfahren zur kosmetischen Behandlung von Keratinmaterialien wie der Körper- oder Gesichtshaut, der Nägel, der Haare, der Wimpern und/oder der Augenbrauen, **dadurch gekennzeichnet, dass** es daraus besteht, dass man auf die Keratinmaterialien eine kosmetische Zusammensetzung gemäß einem der Ansprüche 41 bis 47 aufbringt und danach gegebenenfalls mit Wasser ausspült.

49. Verfahren zur Festigung der Frisur, kosmetischen Behandlung, Pflege, Wäsche und/oder Abschminkung der Körper- oder Gesichtshaut, der Nägel, der Haare, der Wimpern und/oder der Augenbrauen, **dadurch gekennzeichnet, dass** es daraus besteht, dass man auf die Keratinmaterialien eine kosmetische Zusammensetzung gemäß einem der Ansprüche 41 bis 47 aufbringt und dann gegebenenfalls mit Wasser auswäscht.

50. Verfahren zur Herstellung eines ethylenischen Copolymers gemäß einem der Ansprüche 1 bis 40, bei dem man die Amineinheiten der Monomere, die in den Aufbau des Polymers eingehen, insbesondere die Amineinheiten der kationischen Monomere der Formel (IIa) vor der Polymerisation neutralisiert und dann die neutralisierten Monomere zu dem gewünschten Copolymer copolymerisiert.

51. Verfahren zur Herstellung eines ethylenischen Copolymers gemäß einem der Ansprüche 1 bis 40, bei dem man die nicht neutralisierten Monomere, die in den Aufbau des Copolymers eingehen, copolymerisiert und dann das Copolymer nach seiner Bildung neutralisiert.
